(19) Europäisches Patentamt
European Patent Office
Office européen des brevets

(11) EP 2 224 001 A1

(12) EUROPEAN PATENT APPLICATION

(43) Date of publication:
**01.09.2010 Bulletin 2010/35**

(21) Application number: **10004494.0**

(22) Date of filing: **28.02.1992**

(51) Int Cl.:
***C12N 15/10*** *(2006.01)* ***C12N 15/62*** *(2006.01)*
***G01N 33/50*** *(2006.01)* ***G01N 33/68*** *(2006.01)*

(84) Designated Contracting States:
**AT BE CH DE DK ES FR GB GR IT LI LU MC NL PT SE**

(30) Priority: **01.03.1991 US 664989**

(62) Document number(s) of the earlier application(s) in accordance with Art. 76 EPC:
**04006079.0 / 1 452 599**
**92908799.7 / 0 573 611**

(71) Applicant: **Dyax Corporation**
**Cambridge, MA 02139 (US)**

(72) Inventors:
- **Roberts, Bruce Lindsay**
  **Southborough, MA 01772 (US)**
- **Markland, William**
  **Southborough, MA 01772 (US)**
- **Ladner, Robert Charles**
  **Ijamsville, MD 21754 (US)**
- **Ley, Arthur Charles**
  **Newton, MA 02165 (US)**

(74) Representative: **Wichmann, Hendrik**
**Isenbruck Bösl Hörschler Wichmann LLP**
**Prinzregentenstrasse 68**
**81675 München (DE)**

Remarks:
This application was filed on 28-04-2010 as a divisional application to the application mentioned under INID code 62.

# (54) Improved epitope displaying phage

(57) The present invention relates to libraries of display phage which display various mutant epitopic peptides or protein domains with potential to bind to a target material of interest. Site-specific protease cleavable linkers may be incorporated into these phages to overcome the problem of irreversible binding to target materials. When the displayed insert is a mini-protein, a reagent which cleaves the crosslink may be used to facilitate release of a tightly bound display phage. The invention also relates to providing a more balanced library of epitopic peptide display phage.

EP 2 224 001 A1

## Description

## BACKGROUND OF THE INVENTION

### Field of the Invention

This invention relates to the expression and display of libraries of mutated epitopic peptides or potential binding protein domains on the surface of phage, and the screening of those libraries to identify high affinity species.

### Information Disclosure Statement

The amino acid sequence of a protein determines its three-dimensional (3D) structure, which in turn determines protein function. Some residues on the polypeptide chain are more important than others in determining the 3D structure of a protein. Substitutions of amino acids that are exposed to solvent are less likely to affect the 3D structure than are substitutions at internal loci.

"Protein engineering" is the art of manipulating the sequence of a protein in order to alter its binding characteristics. The factors affecting protein binding are known, but designing new complementary surfaces has proven difficult.

With the development of recombinant DNA techniques, it became possible to obtain a mutant protein by mutating the gene encoding the native protein and then expressing the mutated gene. Several mutagenesis strategies are known. One, "protein surgery", involves the introduction of one or more predetermined mutations within the gene of choice. A single polypeptide of completely predetermined sequence is expressed, and its binding characteristics are evaluated.

At the other extreme is random mutagenesis by.means of relatively nonspecific mutagens such as radiation and various chemical agents.

It is possible to randomly vary predetermined nucleotides using a mixture of bases in the appropriate cycles of a nucleic acid synthesis procedure. The proportion of bases in the mixture, for each position of a codon, will determine the frequency at which each amino acid will occur in the polypeptides expressed from the degenerate DNA population. Oliphant et al. (OLIP86) and Oliphant and Struhl (OLIP87) have demonstrated ligation and cloning of highly degenerate oligonucleotides, which were used in the mutation of promoters. They suggested that similar methods could be used in the variation of protein coding regions. They do not say how one should: a) choose protein residues to vary, or b) select or screen mutants with desirable properties. Reidhaar-Olson and Sauer (REID88a) have used synthetic degenerate oligo-nts to vary simultaneously two or three residues through all twenty amino acids. See also Vershon et al. (VERS86a; VERS86b). Reidhaar-Olson and Sauer do not discuss the limits on how many residues could be varied at once nor do they mention the problem of unequal abundance of DNA encoding different amino acids.

A number of researchers have directed unmutated foreign antigenic epitopes to the surface of phage, fused to a native phage surface protein, and demonstrated that the epitopes were recognized by antibodies.

Dulbecco (DULB86) suggests a procedure for incorporating a foreign antigenic epitope into a viral surface protein so that the expressed chimeric protein is displayed on the surface of the virus in a manner such that the foreign epitope is accessible to antibody. In 1985 Smith (SMIT85) reported inserting a nonfunctional segment of the EcoRI endonuclease gene into gene III of bacteriophage f1, "in phase". The gene III protein is a minor coat protein necessary for infectivity. Smith demonstrated that the recombinant phage were adsorbed by immobilized antibody raised against the EcoRI endonuclease, and could be eluted with acid. De la Cruz et al. (DELA88) have expressed a fragment of the repeat region of the circumsporozoite protein from Plasmodium falciparum on the surface of M13 as an insert in the gene III protein. They showed that the recombinant phage were both antigenic and immunogenic in rabbits, and that such recombinant phage could be used for B epitope mapping. The researchers suggest that similar recombinant phage could be used for T epitope mapping and for vaccine development.

McCafferty et al. (MCCA90) expressed a fusion of an Fv fragment of an antibody to the N-terminal of the pIII protein. The Fv fragment was not mutated.

Ladner, Glick, and Bird, WO88/06630 (publ. 7 Sept. 1988 and having priority from US application 07/021,046, assigned to Genex Corp.) (LGB) speculate that diverse single chain antibody domains (SCAD) may be screened for binding to a particular antigen by varying the DNA encoding the combining determining regions of a single chain antibody, subcloning the SCAD gene into the gpV gene of phage lambda so that a SCAD/gpV chimera is displayed on the outer surface of phage lambda, and selecting phage which bind to the antigen through affinity chromatography.

Parmley and Smith (PARM88) suggested that an epitope library that exhibits all possible hexapeptides could be constructed and used to isolate epitopes that bind to antibodies. In discussing the epitope library, the authors did not suggest that it was desirable to balance the representation of different amino acids. Nor did they teach that the insert should encode a complete domain of the exogenous protein. Epitopes are considered to be unstructured peptides as opposed to structured proteins. Scott and Smith (SCOT90) and Cwirla et al. (CWIR90) prepared "epitope libraries" in which potential hexapeptide epitopes for a target antibody were randomly mutated by fusing degenerate oligonucleotides,

encoding the epitopes, with gene III of fd phage, and expressing the fused gene in phage-infected cells. The cells manufactured fusion phage which displayed the epitopes on their surface; the phage which bound to immobilized antibody were eluted with acid and studied. Devlin et al. (DEVL90) similarly screened, using M13 phage, for random 15 residue epitopes recognized by streptavidin.

The Scott and Smith, Cwirla et al., and Devlin et al., libraries provided a highly biased sampling of the possible amino acids at each position. Their primary concern in designing the degenerate oligonucleotide encoding their variable region was to ensure that all twenty amino acids were encodible at each position; a secondary consideration was minimizing the frequency of occurrence of stop signals. Consequently, Scott and Smith and Cwirla et al. employed NNK (N=equal mixture of G, A, T, C; K-equal mixture of G and T) while Devlin et al. used NNS (S=equal mixture of G and C). There was no attempt to minimize the frequency ratio of most favored-to-least favored amino acid, or to equalize the rate of occurrence of acidic and basic amino acids.

Devlin et al. characterized several affinity-selected streptavidin-binding peptides, but did not measure the affinity constants for these peptides. Cwirla et al. did determine the affinity constant for his peptides, but were disappointed to find that his best hexapeptides had affinities (350-300nM), "orders of magnitude" weaker than that of the native Met-enkephalin epitope (7nM) recognized by the target antibody. Cwirla et al. speculated that phage bearing peptides with higher affinities remained bound under acidic elution, possibly because of multivalent interactions between phage (carrying about 4 copies of pIII) and the divalent target IgG. Scott and Smith were able to find peptides whose affinity for the target antibody (A2) was comparable to that of the reference myohemerythrin epitope (50nM). However, Scott and Smith likewise expressed concern that some high-affinity peptides were lost, possibly through irreversible binding of fusion phage to target.

Ladner, et al, WO90/02809, incorporated by reference herein, describe a process for the generation and identification of novel binding proteins having affinity for a predetermined target. In this process, a gene encoding a potential binding domain (as distinct from a mere epitopic peptide), said gene being obtained by random mutagenesis of a limited number of predetermined codons, is fused to a genetic element which causes the resulting chimeric expression product to be displayed on the outer surface of a virus (especially a filamentous phage) or a cell. Chromatographic selection is then used to identify viruses or cells whose genome includes such a fused gene which coded for the protein which bound to the chromatographic target. Ladner, et al. discusss several methods of recovering the gene of interest when the viruses or cells is so tightly bound to the target that it cannot be washed off in viable form. These are growing them in situ on the chromatographic matrix, fragmenting the matrix and using it as an inoculant into a culture vessel, degrading the linkage between the matrix and the target material, and degrading the viruses or cells but then recovering their DNA. However, these methods will also recover viruses or cells which are nonspecifically bound to the target material.

WO90/02809 also addressed strategies for mutagenesis, including one which provides all twenty amino acids in substantially equal proportions, bust only in the context of mutagenesis of protein domains, not epitopic peptides.

## SUMMARY OF THE INVENTION

The present invention is intended to overcome the deficiencies discussed above. In one embodiment of the invention, a library of "display phage" is used to identify binding domains with a high affinity for a predetermined target. Potential binding domains are displayed on the surface of the phage. This is achieved by expressing a fused gene which encodes a chimeric outer surface protein comprising the potential binding domain and at least a functional portion of a coat protein native to the phage. The preferred embodiment uses a pattern of semirandom mutagenesis, called "variegation", that focuses mutations into those residues of a parental binding domain that are most likely to affect its binding properties and are least likely to destroy its underlying structure. As a result, while any one phage displays only a single foreign binding domain (though possibly in multiple copies), the phage library collectively displays thousands, even millions, of different binding domains. The phage library is screened by affinity separation techniques to identify those phage bearing successful (high affinity) binding domains, and these phage are recovered and characterized to determine the sequence of the successful binding domains. These successful binding domains may then serve as the parental binding domains for another round of variegation and affinity separation.

In another embodiment of the invention, the display phage display on their surface a chimeric outer surface protein comprising a functional portion of a native outer surface protein and a potential epitope. In an epitope library made of these display phage, the region corresponding to the foreign epitope is hypervariable. The library is screened with an antibody or other binding protein of interest and high affinity epitopes are identified. References to display, mutagenesis and screening of potential binding domains should be taken to apply, mutatis mutandis, to display, mutagenesis and screening of potential epitopes, unless stated otherwise.

As previously mentioned, when several copies of the chimeric coat protein are displayed on a single phage, there is a risk that irreversible binding will occur, especially if the target is multivalent (as with an antibody). In this case, the phage last eluted by an elution gradient will not be the ones bearing the highest affinity epitopes or binding domains, but rather will be those having an affinity high enough to hold on to the target under the initial elution conditions but not

so high as to bind irreversibly. As a result, the methodology known in the art may fail to recover every high affinity epitopes or binding domains, which for many purposes are the most desirable species.

We propose to cope with the problem of irreversible binding by incorporating into the chimeric coat protein a linker sequence, between the foreign epitope or binding domain, and the sequence native to the wild-type phage coat protein, which is cleavable by a site-specific protease. In this case, the phage library is incubated with the immobilized target. Lower affinity phage are eluted off the target and only the solid phase (bearing the high affinity phage) is retained. The aforementioned linker sequence is cleaved, and the phage particles are released, leaving the bound epitope or binding domain behind. One may then recover the particles (and sequence their DNA to determine the sequence of the corresponding epitope or binding domain) or the bound peptide (and sequence its amino acids directly). The former recovery method is preferred, as the encoding DNA may be amplified in vitro using PCR or in vivo by transfecting suitable host cells with the high affinity display phage. While the production of fusion proteins with cleavable linkers is known in the art, the use of such linkers to facilitate controlled cleavage of a chimeric coat protein of a phage has not previously been reported.

Another method of addressing irreversible binding is appropriate when the binding domains are "mini-proteins", i.e., relatively small peptides whose stability of structure primarily attributable to the presence of one or more covalent crosslinks, e.g., disulfide bonds. As in the example above, low affinity phage are removed first. The remaining, high affinity, bound phage are then treated with a reagent which breaks the crosslink, such as dithiothreitol in the case of a domain with disulfide bonds, but does not cleave peptidyl bonds or modify the side chains of amino acids which are not crosslinked. This will usually result in sufficient denaturation to either release the phage outright or to permit their elution by other means.

These two methods, of course, are not mutually exclusive.

In the previously known epitope display phage libraries, the phage genome was altered by replacing the gene encoding the wild-type gene III protein of M13 with one encoding a chimeric coat protein. As a result, the five normal copies of the wild type gene III protein were all replaced by the chimeric coat protein, whereby each phage had five potential binding sites for the target, and hence a very high potential avidity. With high affinity epitopes (or binding domains), this might well contribute to irreversible binding.

One method of the present invention of reducing the avidity of display phage, especially epitope display phage, for their target, and hence of alleviating the problem of irreversible binding, is to engineer the phage to contain two genes that each express a coat protein, one encoding the wild type coat protein, and the other the cognate chimeric coat protein. Thus, phage bearing identical epitopes or binding domains may yet bear different ratios of wild type to chimeric coat protein molecules, and hence have different avidities. The average ratio for the library will be dependent on the relative levels of expression of the two cognate genes.

It may be advantageous to be able to modulate the ratio of the chimeric coat protein to its cognate wild-type coat protein. For example, early in the evolutionary process, the affinity of the binding domains for their target may be rather low, especially if they are based on a parental binding domain which has no affinity for the target.

Modulation may be achieved by placing the chimeric gene under the control of a regulatable promoter.

While it may be possible to place the cognate wild-type gene under the control of a second, differently regulated promoter, this may be impracticible if, as with the M13 geneIII, the gene is part of a polycistronic operon. In this case, expression of the wild-type gene may be reduced by replacing its methionine initiation codon with a leucine initiation codon.

M13 gene III, as previously noted, encodes one of the minor coat proteins of this filamentous phage (five copies per phage). In view of the difficulties with irreversible binding reported by those modifying this gene so that a foreign epitope is displayed on the phage coat, use of the M13 major coat protein was clearly discouraged. However, we have found that chimeric major coat proteins are in fact useful for displaying potential binding domains for screening purposes even though (indeed, sometimes because) there are over a thousand copies of this protein per phage. It is believed that the major (VIII) coat protein would likewise be useful in constructing an epitope phage library.

We have also developed a linker suitable for attaching potential binding domains (or epitopic peptides) to this major coat protein, and perhaps to other proteins as well.

Finally, to the extent that some of the problems experienced with epitope libraries have been attributable to the use of patterns of mutagenesis which lead to highly biased allocations of amino acids, the present invention is also directed to a variety of improved patterns that lead to less biased and hence more efficient epitope phage libraries.

## BRIEF DESCRIPTION OF THE DRAWINGS

Figure 1 shows how a phage may be used as a genetic phage. At (a) we have a wild-type precoat protein lodged in the lipid bilayer. The signal peptide is in the periplasmic space. At (b), a chimeric precoat protein, with a potential binding domain interposed between the signal peptide and the mature coat protein sequence, is similarly trapped.

At (c) and (d), the signal peptide has been cleaved off the wild-type and chimeric proteins, respectively, but certain residues of the coat protein sequence interact with the lipid bilayer to prevent the mature protein from passing entirely into the periplasm. At (e) and (f), mature wild-type and chimeric protein are assembled into the coat of a single stranded DNA phage as it emerges into the periplasmic space. The phage will pass through the outer membrane into the medium where it can be recovered and chromatographically evaluated.
Figure 2 shows the $C_\alpha$s of the coat protein of phage f1.

## DETAILED DESCRIPTION OF THE PREFERRED EMBODIMENTS

### I. DISPLAY STRATEGY

A. General Considerations

The present invention contemplates that a potential binding domain (pbd) or a potential epitope will be displayed on the surface of a phage in the form of a fusion with a coat (outer surface) protein (OSP) of the phage. This chimeric outer surface protein is the processed product of the polypeptide expressed by an display gene inserted into the phage genome; therefore: 1) the genome of the phage must allow introduction of the display gene either by tolerating additional genetic material or by having replaceable genetic material; 2) the virion must be capable of packaging the genome after accepting the insertion or substitution of genetic material, and 3) the display of the OSP-IPBD protein on the phage surface must not disrupt virion structure sufficiently to interfere with phage propagation.
When the viral particle is assembled, its coat proteins may attach themselves to the phage: a) from the cytoplasm, b) from the periplasm, or c) from within the lipid bilayer. The immediate expression product of the display gene must feature, at its amino terminal, a functional secretion signal peptide, such as the phoA signal (MRQSTIALALLPLLFTPVTKA), if the coat protein attaches to the phage from the periplasm or from within the lipid bilayer. If a secretion signal is necessary for the display of the potential binding domain, in an especially preferred embodiment the bacterial cell in which the hybrid gene is expressed is of a "secretion-permissive" strain.
The DNA sequence encoding the foreign epitope or binding domain should precede the sequence encoding the coat protein proper if the amino terminal of the processed coat protein is normally its free end, and should follow it if the carboxy terminal is the normal free end.
The morphogenetic pathway of the phage determines the environment in which the IPBD will have opportunity to fold. Periplasmically assembled phage are preferred when IPBDs contain essential disulfides, as such IPBDs may not fold within a cell (these proteins may fold after the phage is released from the cell). Intracellularly assembled phage are preferred when the IPBD needs large or insoluble prosthetic groups (such as $Fe_4S_4$ clusters), since the IPBD may not fold if secreted because the prosthetic group is lacking.
When variegation is introduced, multiple infections could generate hybrid GPs that carry the gene for one PBD but have at least some copies of a different PBD on their surfaces; it is preferable to minimize this possibility by infecting cells with phage under conditions resulting in a low multiple-of-infection (MOI).
For a given bacteriophage, the preferred OSP is usually one that is present on the phage surface in the largest number of copies, as this allows the greatest flexibility in varying the ratio of OSP-IPBD to wild type OSP and also gives the highest likelihood of obtaining satisfactory affinity separation. Moreover, a protein present in only one or a few copies usually performs an essential function in morphogenesis or infection; mutating such a protein by addition or insertion is likely to result in reduction in viability of the GP. Nevertheless, an OSP such as M13 gIII protein may be an excellent choice as OSP to cause display of the PBD.
It is preferred that the wild-type osp gene be preserved. The ipbd gene fragment may be inserted either into a second copy of the recipient osp gene or into a novel engineered osp gene. It is preferred that the osp-ipbd gene be placed under control of a regulated promoter. Our process forces the evolution of the PBDs derived from IPBD so that some of them develop a novel function, viz. binding to a chosen target. Placing the gene that is subject to evolution on a duplicate gene is an imitation of the widely-accepted scenario for the evolution of protein families. It is now generally accepted that gene duplication is the first step in the evolution of a protein family from an ancestral protein. By having two copies of a gene, the affected physiological process can tolerate mutations in one of the genes. This process is well understood and documented for the globin family (cf. DICK83, p65ff, and CRBI84, p117-125).
The user must choose a site in the candidate OSP gene for inserting a ipbd gene fragment. The coats of most bacteriophage are highly ordered. Filamentous phage can be described by a helical lattice; isometric phage, by an icosahedral lattice. Each monomer of each major coat protein sits on a lattice point and makes defined interactions with each of its neighbors. Proteins that fit into the lattice by making some, but not all, of the normal lattice contacts are likely to destabilize the virion by: a) aborting formation of the virion, b) making the virion unstable, or c) leaving gaps in the virion so that the nucleic acid is not protected. Thus in bacteriophage, it is important to retain in engineered OSP-IPBD fusion proteins those residues of the parental OSP that interact with other proteins in the virion. For M13 gVIII, we prefer

to retain the entire mature protein, while for M13 gIII, it might suffice to retain the last 100 residues (or even fewer). Such a truncated gIII protein would be expressed in parallel with the complete gIII protein, as gIII protein is required for phage infectivity.

The display gene is placed downstream of a known promoter, preferably a regulated promoter such as lacUV5, tac, or trp.

## B. Filamentous Phages

The filamentous phages, which include M13, f1, fd, If1, Ike, Xf, Pf1, and Pf3, are of particular interest. The entire life cycle of the filamentous phage M13, a common cloning and sequencing vector, is well understood. The genetic structure (SCHA78) of M13 is well known as is the physical structure of the virion (BANN81, BOEK80, CHAN79, ITOK79, KAPL78, KUHN85b, KUHN87, MAR080, MARV78, MESS78, OHKA81, RASC86, RUSS81, SCHA78, SMIT85, WEBS78, and ZIMM82); see RASC86 for a recent review of the structure and function of the coat proteins.

Marvin and collaborators (MARV78, MAKO80, BANN81) have determined an approximate 3D virion structure of the closely related phage f1 by a combination of genetics, biochemistry, and X-ray diffraction from fibers of the virus. Figure 2 is drawn after the model of Banner et al. (BANN81) and shows only the $C_\alpha$s of the protein. The apparent holes in the cylindrical sheath are actually filled by protein side groups so that the DNA within is protected. The amino terminus of each protein monomer is to the outside of the cylinder, while the carboxy terminus is at smaller radius, near the DNA. Although other filamentous phages (e.g. Pf1 or Ike) have different helical symmetry, all have coats composed of many short $\alpha$-helical monomers with the amino terminus of each monomer on the virion surface.

### 1. M13 Major Coat Protein (gVIII)

The major coat protein of M13 is encoded by gene VIII. The 50 amino acid mature gene VIII coat protein is synthesized as a 73 amino acid precoat (SCHA78; ITOK79). The first 23 amino acids constitute a typical signal-sequence which causes the nascent polypeptide to be inserted into the inner cell membrane. Whether the precoat inserts into the membrane by itself or through the action of host secretion components, such as SecA and SecY, remains controversial, but has no effect on the operation of the present invention.

An E. coli signal peptidase (SP-I) recognizes amino acids 18, 21, and 23, and, to a lesser extent, residue 22, and cuts between residues 23 and 24 of the precoat (KUHN85a, KUHN85b, OLIV87). After removal of the signal sequence, the amino terminus of the mature coat is located on the periplasmic side of the inner membrane; the carboxy terminus is on the cytoplasmic side. About 3000 copies of the mature 50 amino acid coat protein associate side-by-side in the inner membrane.

We have constructed a tripartite gene comprising:

1) DNA encoding a signal sequence directing secretion of parts (2) and (3) through the inner membrane,
2) DNA encoding the mature BPTI sequence, and
3) DNA encoding the mature M13 gVIII protein.

This gene causes BPTI to appear in active form on the surface of M13 phage.

### 2. M13 Minor Coat Proteins, Generally

An introduced binding domain or epitope may also be displayed on a filamentous phage as a portion of a chimeric minor coat protein. These are encoded by genes III, VI, VIII, and IX, and each is present in about 5 copies per virion and is related to morphogenesis or infection. In contrast, the major coat protein is present in more than 2500 copies per virion. The gene III. VI. VIII, and IX proteins are present at the ends of the virion.

### 3. The M13 gIII Minor Coat Protein

The single-stranded circular phage DNA associates with about five copies of the gene III protein and is then extruded through the patch of membrane-associated coat protein in such a way that the DNA is encased in a helical sheath of protein (WEBS78). The DNA does not base pair (that would impose severe restrictions on the virus genome); rather the bases intercalate with each other independent of sequence.

Smith (SMIT85) and de la Cruz et al. (DELA88) have shown that insertions into gene III cause novel protein domains to appear on the virion outer surface. The mini-protein's gene may be fused to gene III at the site used by Smith and by de la Cruz et al., at a codon corresponding to another domain boundary or to a surface loop of the protein, or to the amino terminus of the mature protein.

All published works use a vector containing a single modified gene III of fd. Thus, all five copies of gIII are identically modified. Gene III is quite large (1272 b.p. or about 20% of the phage genome) and it is uncertain whether a duplicate of the whole gene can be stably inserted into the phage. Furthermore, all five copies of gIII protein are at one end of the virion. When bivalent target molecules (such as antibodies) bind a pentavalent phage, the resulting complex may be irreversible. Irreversible binding of the phage to the target greatly interferes with affinity enrichment of the GPs that carry the genetic sequences encoding the novel polypeptide having the highest affinity for the target.

To reduce the likelihood of formation of irreversible complexes, we may use a second, synthetic gene that encodes only the carboxy-terminal portion of III. We might, for example, engineer a gene that comprises (from 5' to 3'):

1) a promoter (preferably regulated),
2) a ribosome-binding site,
3) an initiation codon,
4) a sequence encoding a functional signal peptide directing secretion of parts (5) and (6) through the inner membrane,
5) DNA encoding a potential binding domain,
6) DNA encoding residues 275 through 424 of M13 gIII protein,
7) a translation stop codon, and
8) (optionally) a transcription stop signal.

Note that in the gIII protein, the amino terminal moiety is responsible for pilus binding (i.e., for infectivity) and the carboxy terminal moiety for packaging, so that the chimeric gIII protein described above is able to assemble into the viral coat, but does not contribute to infectivity.

We leave the wild-type gene III so that some unaltered gene III protein will be present.

Thus, the hybrid gene may comprise DNA encoding a potential binding domain operably linked to a signal sequence (e.g., the signal sequences of the bacterial phoA or bla genes or the signal sequence of M13 phage geneIII) and to DNA encoding at least a functional portion of a coat protein (e.g., the M13 gene III or gene VIII proteins) of a filamentous phage (e.g., M13). The expression product is transported to the inner membrane (lipid bilayer) of the host cell, whereupon the signal peptide is cleaved off to leave a processed hybrid protein. The C-terminus of the coat protein-like component of this hybrid protein is trapped in the lipid bilayer, so that the hybrid protein does not escape into the periplasmic space. (This is typical of the wild-type coat protein.) As the single-stranded DNA of the nascent phage particle passes into the periplasmic space, it collects both wild-type coat protein and the hybrid protein from the lipid bilayer. The hybrid protein is thus phaged into the surface sheath of the filamentous phage, leaving the potential binding domain exposed on its outer surface.

4. Coat Proteins of Pf3

Similar constructions could be made with other filamentous phage. Pf3 is a well known filamentous phage that infects Poeudomonas aerugenosa cells that harbor an IncP-1 plasmid. The entire genome has been sequenced (LUIT85) and the genetic signals involved in replication and assembly are known (LUIT87). The major coat protein of PF3 is unusual in having no signal peptide to direct its secretion. The sequence has charged residues $ASP_7$, $ARG_{37}$, $LYS_{40}$, and $PHE_{44}$-$COO^-$which is consistent with the amino terminus being exposed. Thus, to cause an IPBD to appear on the surface of Pf3, we construct a tripartite gene comprising:

1) a signal sequence known to cause secretion in P. aerugenosa (preferably known to cause secretion of IPBD) fused in-frame to,
2) a gene fragment encoding the IPBD sequence, fused in-frame to,
3) DNA encoding the mature Pf3 coat protein.

Optionally, DNA encoding a flexible linker of one to 10 amino acids is introduced between the ipbd gene fragment and the Pf3 coat-protein gene. Optionally, DNA encoding the recognition site for a specific protease, such as tissue plasminogen activator or blood clotting Factor Xa, is introduced between the ipbd gene fragment and the Pf3 coat-protein gene. Amino acids that form the recognition site for a specific protease may also serve the function of a flexible linker. This tripartite gene is introduced into Pf3 so that it does not interfere with expression of any Pf3 genes. To reduce the possibility of genetic recombination, part (3) is designed to have numerous silent mutations relative to the wild-type gene. Once the signal sequence is cleaved off, the IPBD is in the periplasm and the mature coat protein acts as an anchor and phage-assembly signal. It does not matter that this fusion protein comes to rest in the lipid bilayer by a route different from the route followed by the wild-type coat protein.

Gene Construction

The structural coding sequence of the display gene encodes a chimeric coat protein and any required secretion signal. A "chimeric coat protein" is a fusion of a first amino acid sequence (essentially corresponding to at least a functional portion of a phage coat protein) with a second amino acid sequence, e.g., a domain foreign to and not substantially homologous with any domain of the first protein. A chimeric protein may present a foreign domain which is found (albeit in a different protein) in an organism which also expresses the first protein, or it may be an "interspecies", "intergeneric", etc. fusion of protein structures expressed by different kinds of organisms. The foreign domain may appear at the amino or carboxy terminal of the first amino acid sequence (with or without an intervening spacer), or it may interrupt the first amino acid sequence. The first amino acid sequence may correspond exactly to a surface protein of the phage, or it may be modified, e.g., to facilitate the display of the binding domain.

A preferred site for insertion of the ipbd gene into the phage osp gene is one in which: a) the IPBD folds into its original shape, b) the OSP domains fold into their original shapes, and c) there is no interference between the two domains.

If there is a model of the phage that indicates that either the amino or carboxy terminus of an OSP is exposed to solvent, then the exposed terminus of that mature OSP becomes the prime candidate for insertion of the ipbd gene. A low resolution 3D model suffice.

In the absence of a 3D structure, the amino and carboxy termini of the mature OSP are the best candidates for insertion of the ipbd gene. A functional fusion may require additional residues between the IPBD and OSP domains to avoid unwanted interactions between the domains. Random-sequence DNA or DNA coding for a specific sequence of a protein homologous to the IPBD or OSP, can be inserted between the osp fragment and the ipbd fragment if needed.

Fusion at a domain boundary within the OSP is also a good approach for obtaining a functional fusion. Smith exploited such a boundary when subcloning heterologous DNA into gene III of f1 (SMIT85).

The criteria for identifying OSP domains suitable for causing display of an IPBD are somewhat different from those used to identify and IPBD. When identifying an OSP, minimal size is not so important because the OSP domain will not appear in the final binding molecule nor will we need to synthesize the gene repeatedly in each variegation round. The major design concerns are that: a) the OSP::IPBD fusion causes display of IPBD, b) the initial genetic construction be reasonably convenient, and c) the osp::ipbd gene be genetically stable and easily manipulated. There are several methods of identifying domains. Methods that rely on atomic coordinates have been reviewed by Janin and Chothia (JANI85). These methods use matrices of distances between $\alpha$ carbons ($C_\alpha$), dividing planes (cf. ROSE85), or buried surface (RASH84). Chothia and collaborators have correlated the behavior of many natural proteins with domain structure (according to their definition) . Rashin correctly predicted the stability of a domain comprising residues 206-316 of thermolysin (VITA84, RASH84).

Many researchers have used partial proteolysis and protein sequence analysis to isolate and identify stable domains. (See, for example, VITA84, POTE83, SCOT87a, and PAB079.) Pabo et al. used calorimetry as an indicator that the cI repressor from the coliphage)\ contains two domains; they then used partial proteolysis to determine the location of the domain boundary.

If the only structural information available is the amino acid sequence of the candidate OSP, we can use the sequence to predict turns and loops. There is a high probability that some of the loops and turns will be correctly predicted (cf. Chou and Fasman, (CHOU74)); these locations are also candidates for insertion of the ipbd gene fragment.

Fusing one or more new domains to a protein may make the ability of the new protein to be exported from the cell different from the ability of the parental protein. The signal peptide of the wild-type coat protein may function for authentic polypeptide but be unable to direct export of a fusion. To utilize the Sec-dependent pathway, one may need a different signal peptide. Thus, to express and display a chimeric BPTI/M13 gene VIII protein, we found it necessary to utilize a heterologous signal peptide (that of phoA).

Phage that display peptides having high affinity for the target may be quite difficult to elute from the target, particularly a multivalent target. One can introduce a cleavage site for a specific protease, such as blood-clotting Factor Xa, into the fusion protein so that the binding domain can be cleaved from the genetic package. Such cleavage has the advantage that all resulting phage have identical coat proteins and therefore are equally infective, even if polypeptide-displaying phage can be eluted from the affinity matrix without cleavage. This step allows recovery of valuable genes which might otherwise be lost. To our knowledge, no one has disclosed or suggested using a specific protease as a means to recover an information-containing genetic package or of converting a population of phage that vary in infectivity into phage having identical infectivity.

There exist a number of highly specific proteases. While the invention does not reside in the choice of any particular protease, the protease is preferably sufficiently specific so that under the cleavage conditions, it will cleave the linker but not any polypeptide essential to the viability of the phage, or (save in rare cases) the potential epitope/ binding domain. It is possible that choice of particular cleavage conditions, e.g., low temperature, may make it feasible to use a protease that would otherwise be unsuitable.

The blood-clotting and complementation systems contains a number of very specific proteases. Usually, the enzymes at the early stages of a cascade are more specific than are the later ones. For example, Factor X. ($F.X_2$) is more specific than is thrombin (cp. Table 10-2 of COLM87). Bovine $F.X_2$ cleaves after the sequence Ile-Glu-Gly-Arg while human $F.X_2$ cleaves after Ile-Asp-Gly-Arg. Either protease-linker pair may be used, as desired. If thrombin is used, the most preferred thrombin-sensitive linkers are those found in fibrinogen, Factor XIII, and prothrombin. Preferably, one would take the linker sequence from the species from which the thrombin is obtained; for example, if bovine thrombin is to be used, then one uses a linker taken from bovine fibrinogen or bovine F.XIII.

Human Factor $XI_2$ cleaves human Factor IX at two places (COLM87, p.42):

Q T S K L T $R_{145}$/ A E A V F and
S F N D F T $R_{120}$/ V V G G E.

Thus one could incorporate either of these sequences (especially the underscored portions) as a linker between the PBD and the GP-surface-anchor domain (GPSAD) and use human $F.XI_2$ to cleave them.

Human kallikrein cuts human F.XII at $R_{353}$ (COLM87, p.258):

L F S S M T $R_{353}$/ V V G G L V.

This sequence has significant similarity to the $hF.XI_2$ sites above. One could incorporate the sequence SSMTRVVG as a linker between PBD and GPSAD and cleave PBD from the GP with human kallikrein.

Human $F.XII_2$ cuts human F.XI at $R_{369}$ (COLM87, p.256):

K I K P $R_{369}$/ I V G G T.

One could incorporate KIKPRIVG as a linker between PBD and GPSAD. PBD could then be cleaved from GP with $hF.XII_2$.

Other proteases that have been used to cleave fusion proteins include enterokinase, collagenase, chymosin, urokinase, renin, and certain signal peptidases. See Rutter, US 4,769,326.

When a protease inhibitor is sought, the target protease and other proteases having similar substrate specificity are not preferred for cleaving the PBD from the GP. It is preferred that a linker resembling the substrate of the target protease not be incorporated anywhere on the display phage because this could make separation of excellent binders from the rest of the population needlessly more difficult.

If there is steric hindrance of the site-specific cleavage of the linker, the linker may be modified so that the cleavage site is more exposed, e.g., by interposing glycines (for additional flexibility) or prolines (for maximum elongation) between the cleavage site and the bulk of the protein. GUAN91 improved thrombin cleavage of a GST fusion protein by introducing a glycine-rich linker (PGISGGGGG) immediately after the thrombin cleavage site (LVPRGS). A suitable linker may also be identified by variegation-and-selection.

The sequences of regulatory parts of the gene are taken from the sequences of natural regulatory elements: a) promoters, b) Shine-Dalgarno sequences, and c) transcriptional terminators. Regulatory elements could also be designed from knowledge of consensus sequences of natural regulatory regions. The sequences of these regulatory elements are connected to the coding regions; restriction sites are also inserted in or adjacent to the regulatory regions to allow convenient manipulation.

The essential function of the affinity separation is to separate GPs that bear PBDs (derived from IPBD) having high affinity for the target from GPs bearing PBDs having low affinity for the target. If the elution volume of a phage depends on the number of PBDs on the phage surface, then a phage bearing many PBDs with low affinity, $GP(PBD_w)$, might co-elute with a phage bearing fewer PBDs with high affinity, $GP(PBD_2)$. Regulation of the display gene preferably is such that most phage display sufficient PBD to effect a good separation according to affinity. Use of a regulatable promoter to control the level of expression of the display gene allows fine adjustment of the chromatographic behavior of the variegated population.

Induction of synthesis of engineered genes in vegetative bacterial cells has been exercised through the use of regulated promoters such as lacUV5, trpP, or tac (MANI82). The factors that regulate the quantity of protein synthesized are sufficiently well understood that a wide variety of heterologous proteins can now be produced in E. coli, B. subtilis and other host cells in at least moderate quantities (SKER88, BETT88). Preferably, the promoter for the display gene is subject to regulation by a small chemical inducer. For example, the lac promoter and the hybrid trp-lac (tac) promoter are regulatable with isopropyl thiogalactoside (IPTG). The promoter for the constructed gene need not come from a natural osp gene; any regulatable promoter functional in bacteria can be used. A non-leaky promoter is preferred.

The coding portions of genes to be synthesized are designed at the protein level and then encoded in DNA. While the primary consideration in devising the DNA sequence is obtaining the desired diverse population of potential binding domains (or epitopes), consideration is also given to providing restriction sites to facilitate further gene manipulation, minimizing the potential for recombination and spontaneous mutation, and achieving efficient translation in the chosen host cells.

The present invention is not limited to any particular method of DNA synthesis or construction. Conventional DNA synthesizers may be used, with appropriate reagent modifications for production of variegated DNA (similar to that now used for production of mixed probes).

The phage are genetically engineered and then transfected into host cells, e.g., E. coli. B. subtilis, or P. aeruginosa, suitable for amplification. The present invention is not limited to any one method of transforming cells with DNA or to any particular host cells.

## THE INITIAL POTENTIAL BINDING DOMAIN (IPBD) :

By virtue of the present invention, proteins may be obtained which can bind specifically to targets other than the antigen-combining sites of antibodies. For the purposes of the appended claims, a protein P is a biriding protein if for some target other than the variable domain of an antibody, the dissociation constant $K_D$ (P,A) < $10^{-6}$ moles/liter (preferably, < $10^{-7}$ moles/liter). The exclusion of "variable domain of an antibody" is intended to make clear that for the purposes herein a protein is not to be considered a "binding protein" merely because it is antigenic. However, an antigen may nonetheless qualify as a binding protein because it specifically binds to a substance other than an antibody, e.g., an enzyme for its substrate, or a hormone for its cellular receptor. Additionally, it should be pointed out that "binding protein" may include a protein which binds specifically to the Fc of an antibody, e.g., staphylococcal protein A.

While the present invention may be used to develop novel antibodies through variegation of codons corresponding to the hypervariable region of an antibody's variable domain, its primary utility resides in the development of binding proteins which are not antibodies or even variable domains of antibodies. Novel antibodies can be obtained by immunological techniques; novel enzymes, hormones, etc. cannot.

Most larger proteins fold into distinguishable globules called domains. The display strategy is first perfected by modifying a genetic phage to display a stable, structured domain (the "initial potential binding domain", IPBD) for which an affinity molecule (which may be an antibody) is obtainable. The success of the modifications is readily measured by, e.g., determining whether the modified genetic phage binds to the affinity molecule. For the purpose of identifying IPBDs, definitions of "domain" which emphasize stability -- retention of the overall structure in the face of perturbing forces such as elevated temperatures or chaotropic agents -- are favored, though atomic coordinates and protein sequence homology are not completely ignored. When a domain of a protein is primarily responsible for the protein's ability to specifically bind a chosen target, it is referred to herein as a "binding domain" (BD).

The IPBD is chosen with a view to its tolerance for extensive mutagenesis. Once it is known that the IPBD can be displayed on a surface of a phage and subjected to affinity selection, the gene encoding the IPBD is subjected to a special pattern of multiple mutagenesis, here termed "variegation", which after appropriate cloning and amplification steps leads to the production of a population of phage each of which displays a single potential binding domain (a mutant of the IPBD), but which collectively display a multitude of different though structurally related potential binding domains (PEDs). Each genetic phage carries the version of the pbd gene that encodes the PBD displayed on the surface of that particular phage. Affinity selection is then used to identify the display phage bearing the PBDs with the desired binding characteristics, and these display phage may then be amplified. After one or more cycles of enrichment by affinity selection and amplification, the DNA encoding the successful binding domains (SBDs) may then be recovered from selected phage.

If need be, the DNA from the SBD-bearing phage may then be further "variegated", using an SBD of the last round of variegation as the "parental potential binding domain" (PPBD) to the next generation of PBDs, and the process continued until the worker in the art is satisfied with the result. At that point, the SBD may be produced by any conventional means, including chemical synthesis.

The initial potential binding domain may be: 1) a domain of a naturally occurring protein, 2) a non-naturally occurring domain which substantially corresponds in sequence to a naturally occurring domain, but which differs from it in sequence by one or more substitutions, insertions or deletions, 3) a domain substantially corresponding in sequence to a hybrid of subsequences of two or more naturally occurring proteins, or 4) an artificial domain designed entirely on theoretical grounds based on knowledge of amino acid geometries and statistical evidence of secondary structure preferences of amino acids. (However, the limitations of a priori protein design prompted the present invention.) Usually, the domain will be a known binding domain, or at least a homologue thereof, but it may be derived from a protein which, while not possessing a known binding activity, possesses a secondary or higher structure that lends itself to binding activity (clefts, grooves, etc.). The protein to which the IPBD is related need not have any specific affinity for the target material.

In determining whether sequences should be deemed to "substantially correspond", one should consider the following issues: the degree of sequence similarity when the sequences are aligned for best fit according to standard algorithms, the similarity in the connectivity patterns of any crosslinks (e.g., disulfide bonds), the degree to which the

proteins have similar three-dimensional structures, as indicated by, e.g., X-ray diffraction analysis or NMR, and the degree to which the sequenced proteins have similar biological activity. In this context, it should be noted that among the serine protease inhibitors, there are families of proteins recognized to be homologous in which there are pairs of members with as little as 30% sequence homology.

A candidate IPBD should meet the following criteria:

1) a domain exists that will remain stable under the conditions of its intended use (the domain may comprise the entire protein that will be inserted, e.g. BPTI, α-conotoxin GI, or CMTI-III),
2) knowledge of the amino acid sequence is obtainable, and
3) a molecule is obtainable having specific and high affinity for the IPBD, AfM(IPBD).

Preferably, in order to guide the variegation strategy, knowledge of the identity of the residues on the domain's outer surface, and their spatial relationships, is obtainable; however, this consideration is less important if the binding domain is small, e.g., under 40 residues.

Preferably, the IPBD is no larger than necessary because small SBDs (for example, less than 40 amino acids) can be chemically synthesized and because it is easier to arrange restriction sites in smaller amino-acid sequences. For PBDs smaller than about 40 resides, an added advantage is that the entire variegated pbd gene can be synthesized in one piece. In that case, we need arrange only suitable restriction sites in the osp gene. A smaller protein minimizes the metabolic strain on the phage or the host of the GP. The IPBD is preferably smaller than about 200 residues. The IPBD must also be large enough to have acceptable binding affinity and specificity. For an IPBD lacking covalent crosslinks, such as disulfide bonds, the IPBD is preferably at least 40 residues; it may be as small as six residues if it contains a crosslink.

There are many candidate IPBDs, for example, bovine pancreatic trypsin inhibitor (BPTI, 58 residues), CMTI-III (29 residues), crambin (46 residues), third domain of ovomucoid (56 residues), heat-stable enterotoxin (ST-Ia of E. coli) (18 residues), α-Conotoxin GI (13 residues), μ-Conotoxin GIII (22 residues), Conus King Kong mini-protein (27 residues), T4 lysozyme (164 residues), and azurin (128 residues). Table 50 lists several preferred IPBDs.

In some cases, a protein having some affinity for the target may be a preferred IPBD even though some other criteria are not optimally met. For example, the V1 domain of CD4 is a good choice as IPBD for a protein that binds to gp120 of HIV. It is known that mutations in the region 42 to 55 of V1 greatly affect gp120 binding and that other mutations either have much less effect or completely disrupt the structure of V1. Similarly, tumor necrosis factor (TNF) would be a good initial choice if one wants a TNF-like molecule having higher affinity for the TNF receptor.

As even surface mutations may reduce the stability of the PBD, the chosen IPBD should have a high melting temperature (50°C acceptable, the higher the better; BPTI melts at 95°C.) and be stable over a wide pH range (8.0 to 3.0 acceptable; 11.0 to 2.0 preferred), so that the SBDs derived from the chosen IPBD by mutation and selection-through-binding will retain sufficient stability. Preferably, the substitutions in the IPBD yielding the various PBDs do not reduce the melting point of the domain below ~40°C. Mutations may arise that increase the stability of SBDs relative to the IPBD, but the process of the present invention does not depend upon this occurring. Proteins containing covalent crosslinks, such as multiple disulfides, are usually sufficiently stable. A protein having at least two disulfides and having at least 1 disulfide for every twenty residues may be presumed to be sufficiently stable.

If the target is a protein or other macromolecule a preferred embodiment of the IPBD is a small protein such as the Cucurbita maxima trypsin inhibitor III (29 residues), BPTI from Bos Taurus (58 residues), crambin from rape seed (46 residues), or the third domain of ovomucoid from Coturnix soturnix Japonica (Japanese quail) (56 residues), because targets from this class have clefts and grooves that can accommodate small proteins in highly specific ways. If the target is a macromolecule lacking a compact structure, such as starch, it should be treated as if it were a small molecule. Extended macromolecules with defined 3D structure, such as collagen, should be treated as large molecules.

If the target is a small molecule, such as a steroid, a preferred embodiment of the IPBD is a protein of about 80-200 residues, such as ribonuclease from Bos taurus (124 residues), ribonuclease from Aspergillus oruzae (104 residues), hen egg white lysozyme from Gallus grallus (129 residues), azurin from Pseudomonas aerugenosa (128 residues), or T4 lysozyme (164 residues), because such proteins have clefts and grooves into which the small target molecules can fit. The Brookhaven Protein Data Bank contains 3D structures for all of the proteins listed. Genes encoding proteins as large as T4 lysozyme can be manipulated by standard techniques for the purposes of this invention.

If the target is a mineral, insoluble in water, one considers the nature of the molecular surface of the mineral. Minerals that have smooth surfaces, such as crystalline silicon, are best addressed with medium to large proteins, such as ribonuclease, as IPBD in order to have sufficient contact area and specificity. Minerals with rough, grooved surfaces, such as zeolites, could be bound either by small proteins, such as BPTI, or larger proteins, such as T4 lysozyme.

BPTI is an especially preferred IPBD because it meets or exceeds all the criteria: it is a small, very stable protein with a well known 3D structure.

Small polypeptides have potential advantages over larger polypeptides when used as therapeutic or diagnostic

agents, including (but not limited to):

a) better penetration into tissues,
b) faster elimination from the circulation (important for imaging agents),
c) lower antigenicity, and
d) higher activity per mass.

Thus, it would be desirable to be able to employ the combination of variegation and affinity selection to identify small polypeptides which bind a target of choice.

Polypeptides of this size, however, have disadvantages as binding molecules. According to olivera et al. (OLIV90a): "Peptides in this size range normally equilibrate among many conformations (in order to have a fixed conformation, proteins generally have to be much larger)." Specific binding of a peptide to a target molecule requires the peptide to take up one conformation that is complementary to the binding site.

In one embodiment, the present invention overcomes these problems, while retaining the advantages of smaller polypeptides, by fostering the biosynthesis of novel mini-proteins having the desired binding characteristics. Mini-Proteins are small polypeptides (usually less than about 60 residues, more preferably less than 40 residues ("micro-proteins")) which, while too small to have a stable conformation as a result of noncovalent forces alone, are covalently crosslinked (e.g., by disulfide bonds) into a stable conformation and hence have biological activities more typical of larger protein molecules than of unconstrained polypeptides of comparable size.

When mini-proteins are variegated, the residues which are covalently crosslinked in the parental molecule are left unchanged, thereby stabilizing the conformation. For example, in the variegation of a disulfide bonded mini-protein, certain cysteines are invariant so that under the conditions of expression and display, covalent crosslinks (e.g., disulfide bonds between one or more pairs of cysteines) form, and substantially constrain the conformation which may be adopted by the hypervariable linearly intermediate amino acids. In other words, a constraining scaffolding is engineered into polypeptides which are otherwise extensively randomized.

Once a mini-protein of desired binding characteristics is characterized, it may be produced, not only by recombinant DNA techniques, but also by nonbiological synthetic methods.

For the purpose of the appended claims, a mini-protein has between about eight and about 60 residues. An intrachain disulfide bridge connecting amino acids 3 and 8 of a 16 residue polypeptide will be said herein to have a span of 4. If amino acids 4 and 12 are also disulfide bonded, then their bridge has a span of 7. Together, the four cysteines divide the polypeptide into four intercysteine segments (1-2, 5-7, 9-11, and 13-16). (Note that there is no segment between Cys3 and Cys4.)

The connectivity pattern of a crosslinked mini-protein is a simple description of the relative location of the termini of the crosslinks. For example, for a mini-protein with two disulfide bonds, the connectivity pattern "1-3, 2-4" means that the first crosslinked cysteine is disulfide bonded to the third crosslinked cysteine (in the primary sequence), and the second to the fourth.

The variegated disulfide-bonded mini-proteins of the present invention fall into several classes.

Class I mini-proteins are those featuring a single pair of cysteines capable of interacting to form a disulfide bond, said bond having a span of no more than nine residues. This disulfide bridge preferably has a span of at least two residues; this is a function of the geometry of the disulfide bond. When the spacing is two or three residues, one residue is preferably glycine in order to reduce the strain on the bridged residues. The upper limit on spacing is less precise, however, in general, the greater the spacing, the less the constraint on conformation imposed on the linearly intermediate amino acid residues by the disulfide bond.

A disulfide bridge with a span of 4 or 5 is especially preferred. If the span is increased to 6, the constraining influence is reduced. In this case, we prefer that at least one of the enclosed residues be an amino acid that imposes restrictions on the main-chain geometry. Proline imposes the most restriction. Valine and isoleucine restrict the main chain to a lesser extent. The preferred position for this constraining non-cysteine residue is adjacent to one of the invariant cysteines, however, it may be one of the other bridged residues. If the span is seven, we prefer to include two amino acids that limit main-chain conformation. These amino acids could be at any of the seven positions, but are preferably the two bridged residues that are immediately adjacent to the cysteine. If the span is eight or nine, additional constraining amino acids may be provided.

Additional amino acids may appear on the amino side of the first cysteine or the carboxy side of the second cysteine. Only the immediately proximate "unspanned" amino acids are likely to have a significant effect on the conformation of the span.

Class II mini-proteins are those featuring a single disulfide bond having a span of greater than nine amino acids. The bridged amino acids form secondary structures which help to stabilize their conformation. Preferably, these intermediate amino acids form hairpin supersecondary structures such as those schematized below:

S—S

-Cys-αhelix-turn-ßstrand-Cys-

S—S

-Cys-αhelix-turn-αhelix-Cys-

S—S

-Cys-ßstrand-turn-ßstrand-Cys-

In designing a suitable hairpin structure, one may copy an actual structure from a protein whose three-dimensional conformation is known, design the structure using secondary structure tendency data for the individual amino acids, etc., or combine the two approachs. Preferably, one or more actual structures are used as a model, and the frequency data is used to determine which mutations can be made without disrupting the structure.

Preferably, no more than three amino acids lie between the cysteine and the beginning or end of the α helix or β strand.

More complex structures (such as a double hairpin) are also possible.

Class III mini-proteins are those featuring a plurality of disulfide bonds. They optionally may also feature secondary structures such as those discussed above with regard to Class II mini-proteins. Since the number of possible disulfide bond topologies increases rapidly with the number of bonds (two bonds, three topologies; three bonds, 15 topologies; four bonds, 105 topologies) the number of disulfide bonds preferably does not exceed four. Two disulfide bond are preferable to three, and three to four. With two or more disulfide bonds, the disulfide bridge spans preferably do not exceed 30, and the largest intercysteine chain segment preferably does not exceed 20.

Naturally occurring class III mini-proteins, such as heat-stable enterotoxin ST-Ia, frequently have pairs of cysteines that are clustered (-C-C- or -C-X-C-) in the amino-acid sequence. Clustering reduces the number of realizable topologies, and may be advantageous.

Metal Finger Mini-Proteins. The mini-proteins of the present invention are not limited to those crosslinked by disulfide bonds. Another important class of mini-proteins are analogues of finger proteins. Finger proteins are characterized by finger structures in which a metal ion is coordinated by two Cys and two His residues, forming a tetrahedral arrangement around it. The metal ion is most often zinc(II), but may be iron, copper, cobalt, etc. The "finger" has the consensus sequence (Phe or Tyr)-(1 AA)-Cys-(2-4 AAs)-Cys-(3 AAs)-Phe- (5 AAs)-Leu-(2 AAs)-His-(3 AAs)-His-(5 AAs) (BERG88; GIBS88). The present invention encompasses mini-proteins with either one or two fingers.
Further diversity may be introduced into a display phage library of potential binding domains by treating the phage with (preferably nontoxic) enzymes and/or chemical reagents that can selectively modify certain side groups of proteins, and thereby affect the binding properties of the displayed PBDs. Using affinity separation methods, we enrich for the modified GPs that bind the predetermined target. Since the active binding domain is not entirely genetically specified, we must repeat the post-morphogenesis modification at each enrichment round. This approach is particularly appropriate with mini-protein IPBDs because we envision chemical synthesis of these SBDs.

## EPITOPIC PEPTIDBS

The present invention also relates to the identification of epitopic peptides which bind to a target which is the epitopic binding site of an antibody, lectin, enzyme, or other binding protein. In the case of an antibody, the epitopic peptide will be at least four amino acids and more preferably at least six or eight amino acids. Usually, it will be less than 20 amino acids, but there is no fixed upper limit. In general, however, the epitopic peptide will be a "linear" or "sequential" epitope. Typically, in constructing a library for displaying epitopic peptides, all or most of the amino acid positions of the potential epitope will be varied. However, it is desirable that among those amino acids allowed at a particular position, that there be a relatively equal representation, as further discussed below in the context of mutagenesis of protein domains.

## VARIEGATION STRATEGY -- MUTAGENESIS TO OBTAIN POTENTIAL BINDING DOMAINS (OR EPITOPES) WITH DESIRED DIVMSITY

When the number of different amino acid sequences obtainable by mutation of the domain is large when compared to the number of different domains which are displayable in detectable amounts, the efficiency of the forced evolution is greatly enhanced by careful choice of which residues are to be varied. First, residues of a known protein

which are likely to affect its binding activity (e.g., surface residues) and not likely to unduly degrade its stability are identified. Then all or some of the codons encoding these residues are varied simultaneously to produce a variegated population of DNA. The variegated population of DNA is used to express a variety of potential binding domains, whose ability to bind the target of interest may then be evaluated.

The method of the present invention is thus further distinguished from other methods in the nature of the highly variegated population that is produced and from which novel binding proteins are selected. We force the displayed potential binding domain to sample the nearby "sequence space" of related amino-acid sequences in an efficient, organized manner. Four goals guide the various variegation plans used herein, preferably: 1) a very large number (e.g. $10^7$) of variants is available, 2) a very high percentage of the possible variants actually appears in detectable amounts, 3) the frequency of appearance of the desired variants is relatively uniform, and 4) variation occurs only art a limited number of amino-acid residues, most preferably at residues having side groups directed toward a common region on the surface of the potential binding domain.

This is to be distinguished from the simple use of indiscriminate mutagenic agents such as radiation and hydroxylamine to modify a gene, where there is no (or very oblique) control over the site of mutation. Many of the mutations will affect residues that are not a part of the binding domain. Moreover, since at a reasonable level of mutagenesis, any modified codon is likely to be characterized by a single base change, only a limited and biased range of possibilities will be explored. Equally remote is the use of site-specific mutagenesis techniques employing mutagenic oligonucleotides of nonrandomized sequence, since these techniques do not lend themselves to the production and testing of a large number of variants. While focused random mutagenesis techniques are known, the importance of controlling the distribution of variation has been largely overlooked.

The term "variegated DNA" (vgDNA) refers to a mixture of DNA molecules of the same or similar length which, when aligned, vary at some codons so as to encode at each such codon a plurality of different amino acids, but which encode only a single amino acid at other codon positions. It is further understood that in variegated DNA, the codons which are variable, and the range and frequency of occurrence of the different amino acids which a given variable codon encodes, are determined in advance by the synthesizer of the DNA, even though the synthetic method does not allow one to know, a priori, the sequence of any individual DNA molecule in the mixture. The number of designated variable codons in the variegated DNA is preferably no more than 20 codons, and more preferably no more than 5-10 codons. The mix of amino acids encoded at each variable codon may differ from codon to codon. A population of display phage into which variegated DNA has been introduced is likewise said to be "variegated".

When DNA encoding a portion of a known domain of a protein is variegated, the original domain is called the parent of the potential binding domains (PPBD), and the multitude of mutant domains encoded as a result of the variegation are collectively called the "potential binding domains" (PBD), as their ability to bind to the predetermined target is not then known.

We now consider the manner in which we generate a diverse population of potential binding domains in order to facilitate selection of a PBD-bearing phage which binds with the requisite affinity to the target of choice. The potential binding domains are first designed at the amino acid level. Once we have identified which residues are to be mutagenized, and which mutations to allow at those positions, we may then design the variegated DNA which is to encode the various PBDs so as to assure that there is a reasonable probability that if a PBD has an affinity for the target, it will be detected. Of course, the number of independent transformant obtained and the sensitivity of the affinity separation technology will impose limits on the extent of variegation possible within any single round of variegation.

There are many ways to generate diversity in a protein. At one extreme, we vary a few residues of the protein as much as possible ("Focused Mutagenesis"), e.g., we pick a set of five to seven residues and vary each through 13-20 possibilities. An alternative plan of mutagenesis ("Diffuse Mutagenesis") is to vary many more residues through a more limited set of choices (See VERS86a and PAKU86). The variegation pattern adopted may fall between these extremes.

There is no fixed limit on the number of codons which can be mutated simultaneously. However, it is desirable to adopt a mutagenesis strategy which results in a reasonable probability that a possible PBD sequence is in fact displayed by at least one phage. Preferably, the probability that a mutein encoded by the vgDNA and composed of the least favored amino acids at each variegated position will be displayed by at least one independent transformant in the library is at least 0.50, and more preferably at least 0.90. (Muteins composed of more favored amino acids would of course be more likely to occur in the same library.)

Preferably, the variegation is such as will cause a typical transformant population to display $10^6$-$10^7$ different amino acid sequences by means of preferably not more than 10-fold more (more preferably not more than 3-fold) different DNA sequences.

For a mini-protein that lacks $\alpha$ helices and $\beta$ strands, one will, in any given round of mutation, preferably variegate each of 4-6 non-cysteine codons so that they each encode at least eight of the 20 possible amino acids. The variegation at each codon could be customized to that position. Preferably, cysteine is not one of the potential substitutions, though it is not excluded.

When the mini-protein is a metal finger protein, in a typical variegation strategy, the two Cys and two His

residues, and optionally also the aforementioned Phe/Tyr, Phe and Leu residues, are held invariant and a plurality (usually 5-10) of the other residues are varied.

When the mini-protein is of the type featuring one or more $\alpha$ helices and $\beta$ strands, the set of potential amino acid modifications at any given position is picked to favor those which are less likely to disrupt the secondary structure at that position. Since the number of possibilities at each variable amino acid is more limited, the total number of variable amino acids may be greater without altering the sampling efficiency of the selection process.

For the last-mentioned class of mini-proteins, as well as domains other than mini-proteins, preferably not more than 20 and more preferably 5-10 codons will be variegated. However, if diffuse mutagenesis is employed, the number of codons which are variegated can be higher.

The decision as to which residues to modify is eased by knowledge of which residues lie on the surface of the domain and which are buried in the interior.

We choose residues in the PPBD to vary through consideration of several factors, including: a) the 3D structure of the PPBD, b) sequences homologous to PPBD, and c) modeling of the PPBD and mutants of the PPBD. When the number of residues that could strongly influence binding without preventing the normal folding of the PPBD is greater than the number that should be varied simultaneously, the user should pick a subset of those residues to vary at one time. The user picks trial levels of variegation and calculate the abundances of various sequences. The list of varied residues and the level of variegation at each varied residue are adjusted until the composite variegation is commensurate with the sensitivity of the affinity separation and the number of independent transformants that can be made.

Having picked which residues to vary, we now decide the range of amino acids to allow at each variable residue. The total level of variegation is the product of the number of variants at each varied residue. Each varied residue can have a different scheme of variegation, producing 2 to 20 different possibilities. The set of amino acids which are potentially encoded by a given variegated codon are called its "substitution set".

The computer that controls a DNA synthesizer, such as the Milligen 7500, can be programmed to synthesize any base of an oligo-nt with any distribution of nts by taking some nt substrates (e.g. nt phosphoramidites) from each of two or more reservoirs. Alternatively, nt substrates can be mixed in any ratios and placed in one of the extra reservoir for so called "dirty bottle" synthesis. Each codon could be programmed differently. The "mix" of bases at each nucleotide position of the codon determines the relative frequency of occurrence of the different amino acids encoded by that codon.

Simply variegated codons are those in which those nucleotide positions which are degenerate are obtained from a mixture of two or more bases mixed in equimolar proportions. These mixtures are described in this specification by means of the standardized "ambiguous nucleotide" code. In this code, for example, in the degenerate codon "SNT", "S" denotes an equimolar mixture of bases G and C, "N", an equimolar mixture of all four bases, and "T", the single invariant base thymidine.

Complexly variegated codons are those in which at least one of the three positions is filled by a base from an other than equimolar mixture of two of more bases.

Either simply or complexly variegated codons may be used to achieve the desired substitution set.

If we have no information indicating that a particular amino acid or class of amino acid is appropriate, we strive to substitute all amino acids with equal probability because representation of one mini-protein above the detectable level is wasteful. Equal amounts of all four nts at each position in a codon (NNN) yields the amino acid distribution in which each amino acid is present in proportion to the number of codons that code for it. This distribution has the disadvantage of giving two basic residues for every acidic residue. In addition, six times as much R, S, and L as W or M occur. If five codons are synthesized with this distribution, each of the 243 sequences encoding some combination of L, R, and S are 7776-times more abundant than each of the 32 sequences encoding some combination of W and M. To have five Ws present at detectable levels, we must have each of the (L,R,S) sequences present in 7776-fold excess.

It is generally accepted that the sequence of amino acids in a protein or polypeptide determine the three-dimensional structure of the molecule, including the possibility of no definite structure. Among polypeptides of definite length and sequence, some have a defined tertiary structure and most do not.

Particular amino acid residues can influence the tertiary structure of a defined polypeptide in several ways, including by:

a) affecting the flexibility of the polypeptide main chain,
b) adding hydrophobic groups,
c) adding charged groups,
d) allowing hydrogen bonds, and
e) forming cross-links, such as disulfides, chelation to metal ions, or bonding to prosthetic groups.

Flexibility:

GLY is the smallest amino acid, having two hydrogens attached to the $C_\alpha$. Because GLY has no $C_\beta$, it confers

the most flexibility on the main chain. Thus GLY occurs very frequently in reverse turns, particularly in conjunction with PRO, ASP, ASN, SER, and THR.

The amino acids ALA, SER, CYS, ASP, ASN, LEU, MET, PHE, TYR, TRP, ARG, HIS, GLU, GLN, and LYS have unbranched β carbons. Of these, the side groups of SER, ASP, and ASN frequently make hydrogen bonds to the main chain and so can take on main-chain conformations that are energetically unfavorable for the others. VAL, ILE, and THR have branched S carbons which makes the extended main-chain conformation more favorable. Thus VAL and ILE are most often seen in β sheets. Because the side group of THR can easily form hydrogen bonds to the main chain, it has less tendency to exist in a β sheet.

The main chain of proline is particularly constrained by the cyclic side group. The ϕ angle is always close to -60˚. Most prolines are found near the surface of the protein.

Charge:

LYS and ARG carry a single positive charge at any pH below 10.4 or 12.0, respectively. Nevertheless, the methylene groups, four and three respectively, of these amino acids are capable of hydrophobic interactions. The guanidinium group of ARG is capable of donating five hydrogens simultaneously, while the amino group of LYS can donate only three. Furthermore, the geometries of these groups is quite different, so that these groups are often not interchangeable.

ASP and GLU carry a single negative charge at any pH above ~4.5 and 4.6, respectively. Because ASP has but one methylene group, few hydrophobic interactions are possible. The geometry of ASP lends itself to forming hydrogen bonds to main-chain nitrogens which is consistent with ASP being found very often in reverse turns and at the beginning of helices. GLU is more often found in α helices and particularly in the amino-terminal portion of these helices because the negative charge of the side group has a stabilizing interaction with the helix dipole (NICH88, SALI88).

HIS has an ionization pK in the physiological range, viz. 6.2. This pK can be altered by the proximity of charged groups or of hydrogen donators or acceptors. HIS is capable of forming bonds to metal ions such as zinc, copper, and iron.

Hydrogen bonds:

Aside from the charged amino acids, SER, THR, ASN, GLN, TYR, and TRP can participate in hydrogen bonds.

Cross linksg:

The most important form of cross link is the disulfide bond formed between two thiols, especially the thiols of CYS residues. In a suitably oxidizing environment, these bonds form spontaneously. These bonds can greatly stabilize a particular conformation of a protein or mini-protein. When a mixture of oxidized and reduced thiol reagents are present, exchange reactions take place that allow the most stable conformation to predominate. Concerning disulfides in proteins and peptides; see also KATZ90, MATS89, PBRR84, PERR86, SAUE86, WELL86, JANA89, HORV89, KISH85, and SCHN86.

Other cross links that form without need of specific enzymes include:

1) $(CYS)_4$:Fe Rubredoxin (in CREI84, P.376)
2) $(CYS)_4$:Zn Aspartate Transcarbamylase (in CRBI84, P.376) and Zn-fingers (HARD90)
3) $(HIS)_2(MET)(CYS)$: Cu Azurin (in CREI84, P.376) and Basic "Blue" Cu Cucumber protein (GUSS88)
4) $(HIS)_4$:Cu CuZn superoxide dismutase
5) $(CYS)_4$: $(Fe_4S_4)$ Ferredoxin (in CREI84, P.376)
6) $(CYS)_2(HIS)_2$: Zn Zinc-fingers (GIBS88)
7) $(CYS)_3(HIS)$ :Zn Zinc-fingers (GAUS87, GIBS88)

Cross links having $(HIS)_2(MET)(CYS)$:Cu has the potential advantage that HIS and MET can not form other cross links without Cu.

**Simply Variegated Codons**

The following simply variegated codons are useful because they encode a relatively balanced set of amino acids:

1) SNT which encodes the set [L,P,H,R,V,A,D,G]: a) one acidic (D) and one basic (R), b) both aliphatic (L,V) and aromatic hydrophobics (H), c) large (L,R,H) and small (G,A) side groups, d) ridged (P) and flexible (G) amino acids, e) each amino acid encoded once.

2) RNG which encodes the set [M,T,K,R,V,A,E,G]: a) one acidic and two basic (not optimal, but acceptable), b) hydrophilics and hydrophobics, c) each amino acid encoded once.

3) RMG which encodes the set [T,K,A,E]: a) one acidic, one basic, one neutral hydrophilic, b) three favor α helices, c) each amino acid encoded once.

4) VNT which encodes the set [L,P,E,R,I,T,N,S,A,D,G]: a) one acidic, one basic, b) all classes: charged, neutral hydrophilic, hydrophobic, ridged and flexible, etc., c) each amino acid encoded once.

5) RRS which encodes the set [N,S,K,R,D,E,$G^2$]: a) two acidics, two basics, b) two neutral hydrophilics, c) only glycine encoded twice.

6) N N T **which encodes the set** [F,S,Y,C,L,P,H,R,I,T,N,V,A,D,G]: a) sixteen DNA sequences provide fifteen different amino acids; only serine is repeated, all others are present in equal amounts (This allows very efficient sampling of the library.), b) there are equal numbers of acidic and basic amino acids (D and R, once each), c) all major classes of amino acids are present: acidic, basic, aliphatic hydrophobic, aromatic hydrophobic, and neutral hydrophilic.

7) NNG, **which encodes the set** [$L^2$,$R^2$,S,W,P,Q,M,T,K,V,A,E,G, stop] : a) fair preponderance of residues that favor formation of α-helices [L,M,A,Q,K,E; and, to a lesser extent, S,R,T]; b) encodes 13 different amino acids. (VHG encodes a subset of the set encoded by NNG which encodes 9 amino acids in nine different DNA sequences, with equal acids and bases, and 5/9 being α helix-favoring.)

For the initial variegation, NNT is preferred, in most cases. However, when the codon is encoding an amino acid to be incorporated into an α helix, NNG is preferred.

Below, we analyze several simple variegations as to the efficiency with which the libraries can be sampled.

Libraries of random hexapeptides encoded by $(NNK)^6$ have been reported (SCOT90, CWIR90). Table 130 shows the expected behavior of such libraries. NNK produces single codons for PHE, TYR, CYS, TRP, HIS, GLN, ILE, MET, ASN, LYS, ASP, and GLU (α set); two codons for each of VAL, ALA, PRO, THR, and GLY (Φ set) ; and three codons for each of LEU, ARG, and SER (Ω set). We have separated the 64,000,000 possible sequences into 28 classes, shown in Table 130A, based on the number of amino acids from each of these sets. The largest class is ΦΩαααα with ~14.6% of the possible sequences. Aside from any selection, all the sequences in one class have the same probability of being produced. Table 130B shows the probability that a given DNA sequence taken from the $(NNK)^6$ library will encode a hexapeptide belonging to one of the defined classes; note that only -6.3% of DNA sequences belong to the ΦΩαααα class.

Table 130C shows the expected numbers of sequences in each class for libraries containing various numbers of independent transformants (viz. $10^6$, $3\cdot10^6$, $10^7$, $3\ \text{-}10^7$, $10^8$, $3\cdot10^8$, $10^9$, and $3\cdot10^9$). At $10^6$ independent transformants (ITs), we expect to see 56% of the ΩΩΩΩΩΩ class, but only 0.1% of the αααααα class. The vast majority of sequences seen come from classes for which less than 10% of the class is sampled. Suppose a peptide from, for example, class ΦΦΩΩαα is isolated by fractionating the library for binding to a target. Consider how much we know about peptides that are related to the isolated sequence. Because only 4% of the ΦΦΩΩαα class was sampled, we can not conclude that the amino acids from the Ω set are in fact the best from the Ω set. We might have LEU at position 2, but ARG or SER could be better. Even if we isolate a peptide of the ΩΩΩΩΩΩ class, there is a noticeable chance that better members of the class were not present in the library.

With a library of $10^7$ ITs, we see that several classes have been completely sampled, but that the αααααα class is only 1.1% sampled. At $7.6\cdot10^7$ ITs, we expect display of 50% of all amino-acid sequences, but the classes containing three or more amino acids of the α set are still poorly sampled. To achieve complete sampling of the $(NNK)^6$ library requires about $3\cdot10^9$ ITs, 10-fold larger than the largest $(NNK)^6$ library so far reported.

Table 131 shows expectations for a library encoded by $(NNT)^4(NNG)^2$. The expectations of abundance are independent of the order of the codons or of interspersed unvaried codons. This library encodes 0.133 times as many amino-acid sequences, but there are only 0.0165 times as many DNA sequences. Thus $5.0\cdot10^7$ ITs (i.e. 60-fold fewer than required for $(NNK)^6$) gives almost complete sampling of the library. The results would be slightly better for $(NNT)^6$ and slightly, but not much, worse for $(NNG)^6$. The controlling factor is the ratio of DNA sequences to amino-acid sequences.

Table 132 shows the ratio of #DNA sequences/#AA sequences for codons NNK, NNT, and NNG. For NNK and NNG, we have assumed that the PBD is displayed as part of an essential gene, such as gene III in Ff phage, as is indicated by the phrase "assuming stops vanish". It is not in any way required that such an essential gene be used. If a non-essential gene is used, the analysis would be slightly different; sampling of NNK and NNG would be slightly less efficient. Note that $(NNT)^6$ gives 3.6-fold more amino-acid sequences than $(NNK)^5$ but requires 1.7-fold fewer DNA sequences. Note also that $(NNT)^7$ gives twice as many amino-acid sequences as $(NNK)^6$, but 3.3-fold fewer DNA sequences.

Thus, while it is possible to use a simple mixture (NNS, NNK or NNN) to obtain at a particular position all twenty amino acids, these simple mixtures lead to a highly biased set of encoded amino acids, This problem can be overcome

by use of complexly variegated codons.

We first will present the mixture calculated (see WO90/02809) to minimize the ratio of most favored amino acid to least favored amino acid when the nt distribution is subject to two constraints: equal abundances of acidic and basic amino acids and the least possible number of stop codons. We have simplified the search for an optimal nt distribution by limiting the third base to T or G (C or G is equivalent). However, it should be noted that the present invention embraces use of complexly variegated codons in which the third base is not limited to T or G (or to C or G).

The optimum distribution (the "fxS" codon) is shown in Table 10A and yields DNA molecules encoding each type amino acid with the abundances shown. Note that this chemistry encodes all twenty amino acids, with acidic and basic amino acids being equiprobable, and the most favored amino acid (serine) is encoded only 2.454 times as often as the least favored amino acid (tryptophan). The "fxS" vg codon improves sampling most for peptides containing several of the amino acids [F,Y,C,W,H-,Q,I,M,N,K,D,E] for which NNK or NNS provide only one codon. Its sampling advantages are most pronounced when the library is relatively small.

The results of searhing only for the complexly variegated codon which minimizes the ratio of most favored to least favored amino acid, without additional constraints, is shown in Table 10B. The changes are small, indicating that insisting on equality of acids and bases and minimizing stop codons costs us little. Also note that, without restraining the optimization, the prevalence of acidic and basic amino acids comes out fairly close. On the other hand, relaxing the restriction leaves a distribution in which the least favored amino acid is only .412 times as prevalent as SER.

The advantages of an NNT codon are discussed elsewhere in the present application. Unoptimized NNT provides 15 amino acids encoded by only 16 DNA sequences. It is possible to improve on NNT with the complexly variegated codon shown in Table 10C. This gives five amino acids (SER, LEU, HIS, VAL, ASP) in very nearly equal amounts. A further eight amino acids (PHE, TYR, ILE, ASN, PRO, ALA, ARG, GLY) are present at 78% the abundance of SER. THR and CYS remain at half the abundance of SER. When variegating DNA for disulfide-bonded mini-proteins, it is often desirable to reduce the prevalence of CYS. This distribution allows 13 amino acids to be seen at high level and gives no stops; the optimized fxS distribution allows only 11 amino acids at high prevalence.

The NNG codon can also be optimized. When equimolar T,C,A,G are used in NNG, one obtains double doses of LEU and ARG. Table 10D shows an approximately optimized NNG codon. There are, under this variegation, four equally most favored amino acids: LEU, ARG, ALA, and GLU. Note that there is one acidic and one basic amino acid in this set. There are two equally least favored amino acids: TRP and MET. The ratio of lfaa/mfaa is 0.5258. If this codon is repeated six times, peptides composed entirely of TRP and MET are 2% as common as peptides composed entirely of the most favored amino acids. We refer to this as "the prevalence of $(TRP/MET)^6$ in optimized $NNG^6$ $vgDNA^{11}$.

When synthesizing vgDNA by the "dirty bottle" method, it is sometimes desirable to use only a limited number of mixes. One very useful mixture is called the "optimized NNS mixture" in which we average the first two positions of the fxS mixture: $T_1$ = 0.24, $C_1$ = 0.17, $A_1$ = 0.33, $G_1$ = 0.26, the second position is identical to the first, $G_3$ = $G_3$ = 0.5. This distribution provides the amino acids ARG, SER, LEU, GLY, VAL, THR, ASN, and LYS at greater than 5% plus ALA, ASP, GLU, ILE, MET, and TYR at greater than 4%.

An additional complexly variegated codon is of interest. This codon is identical to the optimized NNT codon at the first two positions and has T:G::90:10 at the third position. This codon provides thirteen amino acids (ALA, ILE, ARG, SER, ASP, LEU, VAL, PHE, ASN, GLY, PRO, TYR, and HIS) at more than 5.5%. THR at 4.3% and CYS at 3.9% are more common than the LFAAs of NNK (3.125%). The remaining five amino acids are present at less than 1%. This codon has the feature that all amino acids are present; sequences having more than two of the low-abundance amino acids are rare. When we isolate an SBD using this codon, we can be reasonably sure that the first 13 amino acids were tested at each position. A similar codon, based on optimized NNG, could be used.

Several of the preferred simple or complex variegated codons encode a set of amino acids which includes cysteine. This means that some of the encoded binding domains will feature one or more cysteines in addition to the invariant disulfide-bonded cysteines. For example, at each NNT-encoded position, there is a one in sixteen chance of obtaining cysteine. If six codons are so varied, the fraction of domains containing additional cysteines is 0.33. Odd numbers of cysteines can lead to complications, see Perry and Wetzel (PERR84). On the other hand, many disulfide-containing proteins contain cysteines that do not form disulfides, e.g. trypsin. The possibility of unpaired cysteines can be dealt with in several ways:

First, the variegated phage population can be passed over an immobilized reagent that strongly binds free thiols, such as SulfoLink (catalogue number 44895 H from Pierce Chemical Company, Rockford, Illinois, 61105). Another product from Pierce is TNB-Thiol Agarose (Catalogue Code 20409 H). BioRad sells Affi-Gel 401 (catalogue 153-4599) for this purpose.

Second, one can use a variegation that excludes cysteines, such as:

NHT that gives [F,S,Y,L,P,H,I,T,N,V,A,D],
VNS that gives
[$L^2$,$P^2$,H,Q,$R^3$,I,M,$T^2$ ,N,K,S,$V^2$,$A^2$,E,D,$G^2$],

NNG that gives [$L^2$,S,W,P,Q,$R^2$,M,T,K,R,V,A,E,G,stop],
SNT that gives [L,P,H,R,V,A,D,G],
RNG that gives [M,T,R,R,V,A,E,G],
RMG that gives [T,K,A,E],
VNT that gives [L,P,H,R,I,T,N,S,V,A,D,G], or
RRS that gives [N,S,K,R,D,E,$G^2$].

However, each of these schemes has one or more of the disadvantages, relative to NNT: a) fewer amino acids are allowed, b) amino acids are not evenly provided, c) acidic and basic amino acids are not equally likely), or d) stop codons occur. Nonetheless, NNG, NHT, and VNT are almost as useful as NNT. NNG encodes 13 different amino acids and one stop signal. Only two amino acids appear twice in the 16-fold mix.

Thirdly, one can enrich the population for binding to the preselected target, and evaluate selected sequences post hoc for extra cysteines. Those that contain more cysteines than the cysteines provided for conformational constraint may be perfectly usable. It is possible that a disulfide linkage other than the designed one will occur. This does not mean that the binding domain defined by the isolated DNA sequence is in any way unsuitable. The suitability of the isolated domains is best determined by chemical and biochemical evaluation of chemically synthesized peptides.

Lastly, one can block free thiols with reagents, such as Ellman's reagent, iodoacetate, or methyl iodide, that specifically bind free thiols and that do not react with disulfides, and then leave the modified phage in the population. It is to be understood that the blocking agent may alter the binding properties of the mini-protein; thus, one might use a variety of blocking reagent in expectation that different binding domains will be found. The variegated population of thiol-blocked display phage are fractionated for binding. If the DNA sequence of the isolated binding mini-protein contains an odd number of cysteines, then synthetic means are used to prepare mini-proteins having each possible linkage and in which the odd thiol is appropriately blocked. Nishiuchi (NISH82, NISH86, and works cited therein) disclose methods of synthesizing peptides that contain a plurality of cysteines so that each thiol is protected with a different type of blocking group. These groups can be selectively removed so that the disulfide pairing can be controlled. We envision using such a scheme with the alteration that one thiol either remains blocked, or is unblocked and then reblocked with a different reagent.

Use of NNT or NNG variegated codons leads to very efficient sampling of variegated libraries because the ratio of (different amino-acid sequences)/(different DNA sequences) is much closer to unity than it is for NNK or even the optimized vg codon (fxS). Nevertheless, a few amino acids are omitted in each case. Both NNT and NNG allow members of all important classes of amino acids: hydrophobic, hydrophilic, acidic, basic, neutral hydrophilic, small, and large. After selecting a binding domain, a subsequent variegation and selection may be desirable to achieve a higher affinity or specificity. During this second variegation, amino acid possibilities overlooked by the preceding variegation may be investigated.

In the second round of variegation, a preferred strategy is to vary each position through a new set of residues which includes the amino acid(s) which were found at that position in the successful binding domains, and which include as many as possible of the residues which were excluded in the first round of variegation.

Thus, later rounds of variegation test both amino acid positions not previously mutated, and amino acid substitutions at a previously mutated position which were not within the previous substitution set.

If the first round of variegation is entirely unsuccessful, a different pattern of variegation should be used. For example, if more than one interaction set can be defined within a domain, the residues varied in the next round of variegation should be from a different set than that probed in the initial variegation. If repeated failures are encountered, one may switch to a different IPBD.

## AFFINITY SELECTION OF TARGET-BINDING MUTANTS

Affinity separation is used initially in the present invention to verify that the display system is working, i.e., that a chimeric outer surface protein has been expressed and transported to the surface of the phage and is oriented so that the inserted binding domain is accessible to target material. When used for this purpose, the binding domain is a known binding domain for a particular target and that target is the affinity molecule used in the affinity separation process. For example, a display system may be validated by using inserting DNA encoding BPTI into a gene encoding an outer surface protein of the phage of interest, and testing for binding to anhydrotrypsin, which is normally bound by BPTI.

If the phage bind to the target, then we have confirmation that the corresponding binding domain is indeed displayed by the phage. Phage which display the binding domain (and thereby bind the target) are separated from those which do not.

Once the display system is validated, it is possible to use a variegated population of phage which display a variety of different potential binding domains, and use affinity separation technology to determine how well they bind to one or more targets. This target need not be one bound by a known binding domain which is parental to the displayed

binding domains, i.e., one may select for binding to a new target.

For example, one may variegate a BPTI binding domain and test for binding, not to trypsin, but to another serine protease, such as human neutrophil elastase or cathepsin G, or even to a wholly unrelated target, such as horse heart myoglobin.

The term "affinity separation means" includes, but is not limited to: a) affinity column chromatography, b) batch elution from an affinity matrix material, c) batch elution from an affinity material attached to a plate, d) fluorescence activated cell sorting, and e) electrophoresis in the presence of target material. "Affinity material" is used to mean a material with affinity for the material to be purified, called the "analyte". In most cases, the association of the affinity material and the analyte is reversible so that the analyte can be freed from the affinity material once the impurities are washed away.

If affinity chromatography is to be used, then:

1) the molecules of the target material must be of sufficient size and chemical reactivity to be applied to a solid support suitable for affinity separation,
2) after application to a matrix, the target material preferably does not react with water,
3) after application to a matrix, the target material preferably does not bind or degrade proteins in a non-specific way, and
4) the molecules of the target material must be sufficiently large that attaching the material to a matrix allows enough unaltered surface area (generally at least 500 $A^2$. excluding the atom that is connected to the linker) for protein binding.

Affinity chromatography is the preferred separation means, but FACS, electrophoresis, or other means may also be used.

The present invention makes use of affinity separation of phage to enrich a population for those phage carrying genes that code for proteins with desirable binding properties.

The present invention may be used to select for binding domains which bind to one or more target materials, and/or fail to bind to one or more target materials. Specificity, of course, is the ability of a binding molecule to bind strongly to a limited set of target materials, while binding more weakly or not at all to another set of target materials from which the first set must be distinguished.

Almost any molecule that is suitable for affinity separation may be used as a target. Possible targets include, but are not limited to peptides, soluble and insoluble proteins, nucleic acids, lipids, carbohydrates, other organic molecules (monomeric or polymeric), inorganic compounds, and organometallic compounds. Serine proteases are an especially interesting class of potential target materials.

For chromatography, FACS, or electrophoresis there may be a need to covalently link the target material to a second chemical entity. For chromatography the second entity is a matrix, for FACS the second entity is a fluorescent dye, and for electrophoresis the second entity is a strongly charged molecule. In many cases, no coupling is required because the target material already has the desired property of: a) immobility, b) fluorescence, or c) charge. In other cases, chemical or physical coupling is required.

It is not necessary that the actual target material be used in preparing the immobilized or labeled analogue that is to be used in affinity separation; rather, suitable reactive analogues of the target material may be more convenient. Target materials that do not have reactive functional groups may be immobilized by first creating a reactive functional group through the use of some powerful reagent, such as a halogen. In some cases, the reactive groups of the actual target material may occupy a part on the target molecule that is to be left undisturbed. In that case, additional functional groups may be introduced by synthetic chemistry.

Two very general methods of immobilization are widely used. The first is to biotinylate the compound of interest and then bind the biotinylated derivative to immobilized avidin. The second method is to generate antibodies to the target material, immobilize the antibodies by any of numerous methods, and then bind the target material to the immobilized antibodies. Use of antibodies is more appropriate for larger target materials; small targets (those comprising, for example, ten or fewer non-hydrogen atoms) may be so completely engulfed by an antibody that very little of the target is exposed in the target-antibody complex.

Non-covalent immobilization of hydrophobic molecules without resort to antibodies may also be used. A compound, such as 2,3,3-trimethyldecane is blended with a matrix precursor, such as sodium alginate, and the mixture is extruded into a hardening solution. The resulting beads will have 2,3,3-trimethyldecane dispersed throughout and exposed on the surface.

Other immobilization methods depend on the presence of particular chemical functionalities. A polypeptide will present $-NH_2$ (N-terminal; Lysines), -COOH (C-terminal; Aspartic Acids; Glutamic Acids), -OH (Serines; Threonines; Tyrosines), and -SH (Cysteines). A polysaccharide has free -OH groups, as does DNA, which has a sugar backbone.

The following table is a nonexhaustive review of reactive functional groups and potential immobilization reagents:

| Group | Reagent |
|---|---|
| R-$NH_2$ | Derivatives of 2,4,6-trinitro benzene sulfonates (TNBS), (CREI84, p.11) |
| R-$NH_2$ | Carboxylic acid anhydrides, e.g. derivatives of succinic anhydride, maleic anhydride, citraconic anhydride (CREI84, p.11) |
| R-$NH_2$ | Aldehydes that form reducible Schiff bases (CREI84, p.12) guanido cyclohexanedione derivatives (CREI84, p.14) |
| R-$CO_2$H | Diazo cmpds (CRBI84, p.10) |
| R-$CO_2$- | Epoxides (CREI84, p.10) |
| R-OH | Carboxylic acid anhydrides |
| Aryl-OH | Carboxylic acid anhydrides |
| Indole ring | Benzyl halide and sulfenyl halides (CREI84, p.19) |
| R-SH | N-alkylmaleimides (CREI84, p.21) |
| R-SH | ethyleneimine derivatives (CREI84, p.21) |
| R-SH | Aryl mercury compounds, (CREI84, P.21) |
| R-SH | Disulfide reagents, (CREI84, p.23) |
| Thiol ethers | Alkyl iodides, (CREI84, p.20) Ketones Make Schiff's base and reduce with $NaBH_4$. (CREI84, p. 12-13) |
| Aldehydes | oxidize to COOH, vide supra. |
| R-$SO_3$H | Convert to R-$SO_2$Cl and react with immobilized alcohol or amine. |
| R-$PO_3$H | Convert to R-$PO_2$Cl and react with immobilized alcohol or amine. |
| CC double bonds | Add HBr and then make amine or thiol. |

The extensive literature on affinity chromatography and related techniques will provide further examples.

Matrices suitable for use as support materials include polystyrene, glass, agarose and other chromatographic supports, and may be fabricated into beads, sheets, columns, wells, and other forms as desired. Suppliers of support material for affinity chromatography include: Applied Protein Technologies Cambridge, MA; Bio-Rad Laboratories, Rockville Center, NY; Pierce Chemical Company, Rockford, IL. Target materials are attached to the matrix in accord with the directions of the manufacturer of each matrix preparation with consideration of aood presentation of the target.

Early in the selection process, relatively high concentrations of target materials may be applied to the matrix to facilitate binding; target concentrations may subsequently be reduced to select for higher affinity SBDs.

The population of display phage is applied to an affinity matrix under conditions compatible with the intended use of the binding protein and the population is fractionated by passage of a gradient of some solute over the column. The process enriches for PBDs having affinity for the target and for which the affinity for the target is least affected by the eluants used. The enriched fractions are those containing viable display phage that elute from the column at greater concentration of the eluant.

The eluants preferably are capable of weakening noncovalent interactions between the displayed PBDs and the immobilized target material. Preferably, the eluants do not kill the phage; the genetic message corresponding to successful mini-proteins is most conveniently amplified by reproducing the phage rather than by in vitro procedures such as PCR. The list of potential eluants includes salts (including Na+, $NH_4$+, Rb+, $SQ_4$--, $H_2PO_4$-, citrate, K+, Li+, Cs+, $HSO_4$-, $CO_3$--, Ca++, Sr++, Cl-, $PO_4$---, $HCO_3$-, Mg++, Ba++, Br-, $HPO_4$-- and acetate), acid, heat, compounds known to bind the target, and soluble target material (or analogues thereof).

Neutral solutes, such as ethanol, acetone, ether, or urea, are frequently used in protein purification and are known to weaken non-covalent interactions between proteins and other molecules. Many of these species are, however, very harmful to bacteria and bacteriophage. Urea is known not to harm M13 up to 8 M. Salt is a preferred solute for gradient formation in most cases. Decreasing pH is also a highly preferred eluant. In some cases, the preferred matrix is not stable to low pH so that salt and urea are the most preferred reagents. Other solutes that generally weaken non-covalent interaction between proteins and the target material of interest may also be used.

The uneluted display phage contain DNA encoding binding domains which have a sufficiently high affinity for the target material to resist the elution conditions. The DNA encoding such successful binding domains may be recovered in a variety of ways. Preferably, the bound display phage are simply eluted by means of a change in the elution conditions. Alternatively, one may culture the phage in situ, or extract the target-containing matrix with phenol (or other suitable solvent) and amplify the DNA by PCR or by recombinant DNA techniques. Or, if a site for a specific protease has been engineered into the display vector, the specific protease is used to cleave the binding domain from the GP.

variation in the support material (polystyrene, glass, agarose, cellulose, etc.) in analysis of clones carrying

SBDs is used to distinguish phage that bind to the support material rather than the target.

The harvested phage are now enriched for the binding-to-target phenotype by use of affinity separation involving the target material immobilized on an affinity matrix. Phage that fail to bind to the target material are washed away. It may be desirable to include a bacteriocidal agent, such as azide, in the buffer to prevent bacterial growth. The buffers used in chromatography include: a) many ions or other solutes needed to stabilize the target, and b) any ions or other solutes needed to stabilize the PBDs derived from the IPBD.

Recovery of phage that display binding to an affinity column is typically achieved by collecting fractions eluted from the column with a gradient of a chaotropic agent as described above, or of the target material in soluble form; fractions eluting later in the gradient are enriched for high-affinity phage. The eluted phage are then amplified in suitable host cells.

If some high-affinity phage cannot be eluted from the target in viable form, one may:

1) flood the matrix with a nutritive medium and grow the desired phage in situ,
2) remove parts of the matrix and use them to inoculate growth medium,
3) chemically or enzymatically degrade the linkage holding the target to the matrix so that GPs still bound to target are eluted, or
4) degrade the phage and recover DNA with phenol or other suitable solvent; the recovered DNA is used to transform cells that regenerate GPs.

It is possible to utilize combinations of these methods. It should be remembered that what we want to recover from the affinity matrix is not the phage per se, but the information in them as to the sequence of the successful epitope or binding domain.

As described in WO90/02809, one may modify the affinity separation of the method described to select a molecule that binds to material A but not to material B, or that binds to both A and B at competing or noncompeting sites, or that do not bind to selected targets.

**SUBSEQUENT PRODUCTION**

Using the method of the present invention, we can obtain a replicable phage that displays a novel protein domain having high affinity and specificity for a target material of interest. Such a phage carries both amino-acid embodiments of the binding protein domain and a DNA embodiment of the gene encoding the novel binding domain. The presence of the DNA facilitates expression of a protein comprising the novel binding protein domain within a high-level expression system, which need not be the same system used during the developmental process.

We can proceed to production of the novel binding protein in several ways, including: a) altering of the gene encoding the binding domain so that the binding domain is expressed as a soluble protein, not attached to a phage (either by deleting cordons 5' of those encoding the binding domain or by inserting stop codons 3' of those encoding the binding domain), b) moving the DNA encoding the binding domain into a known expression system, and c) utilizing the phage as a purification system. (If the domain is small enough, it may be feasible to prepare it by conventional peptide synthesis methods.)

As previously mentioned, an advantage inhering from the use of a mini-protein as an IPBD is that it is likely that the derived SBD will also behave like a mini-protein and will be obtainable by means of chemical synthesis. (The term "chemical synthesis", as used herein, includes the use of enzymatic agents in a cell-free environment.)

Peptides may be chemically synthesized either in solution or on supports. Various combinations of stepwise synthesis and fragment condensation may be employed.

During synthesis, the amino acid side chains are protected to prevent branching. Several different protective groups are useful for the protection of the thiol groups of cysteines:

1) 4-methoxybenzyl (MBzl; Mob)(NISH82; ZAFA88), removable with HF;
2) acetamidomethyl (Acm)(NISE82; NISH86; BECK89c), removable with iodine; mercury ions (e.g., mercuric acetate); silver nitrate; and
3) s-para-methoxybenzyl.

Other thiol protective groups may be found in standard reference works such as Greene, PROTECTIVE GROUPS IN ORGANIC SYNTHESIS (1981).

Once the polypeptide chain has been synthesized, disulfide bonds must be formed. Possible oxidizing agents include air (NISH86), ferricyanide (NISH82), iodine (NISH82), and performic acid. Temperature, pH, solvent, and chaotropic chemicals may affect the course of the oxidation.

A large number of mini-proteins with a plurality of disulfide bonds have been chemically synthesized in biologically active form.

The successful binding domains of the present invention may, alone or as part of a larger protein, be used for any purpose for which binding proteins are suited, including isolation or detection of target materials. In furtherance of this purpose, the noel binding proteins may be coupled directly or indirectly, covalently or noncovalently, to a label, carrier or support.

When used as a pharmaceutical, the novel binding proteins may be contained with suitable carriers or adjuvanants. *****

All references cited anywhere in this specification are incorporated by reference to the extent which they may be pertinent.

All cells used in the following examples are E. coli cells.

**EXAMPLE I**

**DISPLAY OF BPTI AS A FUSION TO M13 GENE VIII PROTEIN:**

Example I involves display of BPTI on M13 as a fusion to the mature gene VIII coat protein. Each DNA construction was confirmed by restriction digestion and DNA sequencing.

1. Construction of the viii-signal-sequence::bpti::mature-viii-coat-protein Display Vector.

The operative cloning vectors are M13 and phagemids derived from M13. The initial construction was in the f1-based phagemid pGEM-3Zf (-) (™) (Promega Corp., Madison, WI.).

We constructed a gene encoding, in order, : i) a modified lacUV5 promoter, ii) a Shine-Dalgarno sequence, iii) M13 gene VIII signal sequence, iv) mature BPTI, v) mature-M13-gene-VIII coat protein, vi) multiple stop codons, and vii) a transcription terminator. This gene is illustrated in Table 102. The operator of lacUV5 is the symmetrical lacO to allow tighter repression in the absence of IPTG. The longest segment that is identical to wild-type gene VIII is minimized so that genetic recombination with the co-existing gene VIII is unlikely.

**i) OCV based upon pGEM-3Zf.**

pGEM-3Zf(™) (Promega Corp., Madison, WI.) is a vector containing the amp gene, bacterial origin of replication, bacteriophage f1 origin of replication, a lacZ operon containing a multiple cloning site sequence, and the T7 and SP6 polymerase binding sequences.

BamHI and SalI sites were introduced at the boundaries of the lacZ operon (to facilitate removal of the lacZ operon and its replacement with the synthetic gene); this vector is named pGEM-MB3/4.

**ii) OCV based upon M13mp18.**

M13mp18 (YANI85) is a vector (New England Biolabs, Beverly, MA.) consisting of the whole of the phage genome plus a lacZ operon containing a multiple cloning site (MESS77). BamHI and SalI sites were introduced into M13mp18 at the 5' and 3' ends of the lacZ operon; this vector is named M13-MB1/2.

**B) Synthetic Gene.**

A synthetic gene (VIII-signal-seguence::mature-bpti::mature-VIII-coat-protein) was constructed from 16 synthetic oligonucleotides, synthesized by Genetic Designs Inc. of Houston, Texas, via a method similar to those in KIMH89 and ASHM89. Table 102 contains an annotated version of this sequence. The oligonucleotides were phosphorylated, with the exception of the 5' most molecules, using standard methods, annealed and ligated in stages. The overhangs were filled in with T4 DNA polymerase and the DNA was cloned into the HincII site of pGEM-3Zf(-); the initial construct is pGEM-MB1. Double-stranded DNA of pGEM-MB1 was cut with PstI. filled in with T4 DNA polymerase and ligated to a SalI linker (New England BioLabs) so that the synthetic gene is bounded by BamHI and SalI sites (Table 102). The synthetic gene was obtained on a BamHI-SalI cassette and cloned into pGEM-MB3/4 and M13-MB1/2 using the introduced BamHI and SalI sites, to generate pGEM-MB16 and M13-MB15, respectively. The synthetic insert was sequenced. The original Ribosome Binding Site (RBS) was in error (AGAGG instead of the designed AGGAGG) and we detected no expressed protein in vivo and in vitro.

**C) Alterations to the synthetic gene.**

**i) Ribosome binding site (RBS).**

In pGEM-MB16, a SacI-NheI DNA fragment (containing the RBS) was replaced with an oligonucleotide encoding a new RBS very similar to the RBS of E. coli phoA that is known to function.

```
Original putative RBS (5'-to-3')
GAGCTCagaggCTTACTATGAAGAAATCTCTGGTTCTTAAGGCTAGC
|SacI|                                 | Nhe I |

New RBS (5'-to-3')

GAGCTCTggaggaAATAAAATGAAGAAATCTCTGGTTCTTAAGGCTAGC
|SacI|                                     | Nhe I |
```

The putative RBSs are lower case and the initiating methionine codon is underscored and bold. The resulting construct is pGEM-MB20. In vitro expression of the gene carried by pGEM-MB20 produced a novel protein species of the expected size, about 14.5 kd.

**ii) tac promoter.**

To obtain higher expression of the fusion protein, the lacUV5 promoter was changed to a tac promoter. In pGEM-MB16, a BamHI-HpaII fragment (containing the lacUV5 promoter) was replaced with an oligonucleotide containing the -35 sequence of the trp promoter (Cf RUSS82) converting the lacUV5 promoter to tac. The vector is named pGEM-MB22.

```
MB16      5'- GATCC tctagagtcggc TTTACA ctttatgcttc(cg-
gctcg..-3'
      3'-         G agatctcagccg  aaatgt  gaaatacgaag
gc(cgagc..-5'
          |____|             |-35|           |____|
          BamHI                                HpaII

MB22     5'- GATCC actccccatccccctg TTGACA attaatcat  -3'
         3'-     G tgaggggtaggggggac AACTGT taattagtagc-5'
          |____|                       |-35|           |__
          BamHI
 (HpaII)
```

Promoter and RBS variants of the fusion protein gene were constructed as follows:

| | Promoter | RBS | Encoded Protein |
|---|---|---|---|
| pGEM-MB16 | lac | old | VIIIs.p.-BPTI-matureVIII |
| pGEM-MB20 | lac | new | ” |
| pGEM-MB22 | tac | old | ” |
| pGEM-MB26 | tac | new | ” |

The synthetic genes from pGEM-MB20 and pGEM-MB26 were recloned into the altered phage vector M13-MB1/2 to generate the phage M13-MB27 and M13-MB28 respectively.

**iii. Signal Peptide Sequence.**

In vitro expression of the synthetic gene regulated by tac and the "new" RBS produced a novel protein of the expected size for the unprocessed protein (~16 kd). In vivo expression also produced novel protein of full size; no processed protein could be seen on phage or in cell extracts by silver staining or by Western analysis with anti-BPTI antibody.

Thus we analyzed the signal sequence of the fusion. Table 106 shows a number of typical signal sequences. Charged residues are generally thought to be of great importance and are shown bold and underscored. Each signal sequence contains a long stretch of uncharged residues that are mostly hydrophobic; these are shown in lower case. At the right, in parentheses, is the length of the stretch of uncharged residues. We note that the fusions of gene VIII signal to BPTI and gene III signal to BPTI have rather short uncharged segments. These short uncharged segments may reduce or prevent processing of the fusion peptides. We know that the gene III signal sequence is capable of directing: a) insertion of the peptide comprising (mature-BPTI)::(mature-gene-III-protein) into the lipid bilayer, and b) translocation of BPTI and most of the mature gene III protein across the lipid bilayer (vide infra). That the gene III remains anchored in the lipid bilayer until the phage is assembled is directed by the uncharged anchor region near the carboxy terminus of the mature gene III protein (see Table 116) and not by the secretion signal sequence. The phoA signal sequence can direct secretion of mature BPTI into the periplasm of E. coli (MARK86). Furthermore, there is controversy over the mechanism by which mature authentic gene VIII protein comes to be in the lipid bilayer prior to phage assembly.

Thus we replaced the DNA coding for the gene-VIII-putative-signal-sequence by each of DNA coding for: 1) the phoA signal sequence, 2) the bla signal sequence, and 3) the M13 gene III signal. Each of these replacements produces a tripartite gene encoding a fusion protein that comprises, in order: (a) a signal peptide that directs secretion into the periplasm of parts (b) and (c), derived from a first gene; (b) an initial potential binding domain (BPTI in this case), derived from a second gene (in this case, the second gene is an animal gene); and (c) a structural packaging signal (the mature gene VIII coat protein), derived from a third gene.

The process by which the IPBD::packaging-signal fusion arrives on the phage surface is illustrated in Figure 1. In Figure 1a, we see that authentic gene VIII protein appears (by whatever process) in the lipid bilayer so that both the amino and carboxy termini are in the cytoplasm. Signal peptidase-I cleaves the gene VIII protein liberating the signal peptide (that is absorbed by the cell) and mature gene VIII coat protein that spans the lipid bilayer. Many copies of mature gene VIII coat protein accumulate in the lipid bilayer awaiting phage assembly (Figure 1c). Some signal sequences are able to direct the translocation of quite large proteins across the lipid bilayer. If additional codons are inserted after the codons that encode the cleavage site of the signal peptidase-I of such a potent signal sequence, the encoded amino acids will be translocated across the lipid bilayer as shown in Figure 1b. After cleavage by signal peptidase-I, the amino acids encoded by the added codons will be in the periplasm but anchored to the lipid bilayer by the mature gene VIII coat protein, Figure 1d. The circular single-stranded phage DNA is extruded through a part of the lipid bilayer containing a high concentration of mature gene VIII coat protein; the carboxy terminus of each coat protein molecule packs near the DNA while the amino terminus packs on the outside. Because the fusion protein is identical to mature gene VIII coat protein within the trans-bilayer domain, the fusion protein will co-assemble with authentic mature gene VIII coat protein as shown in Figure 1e.

In each case, the mature VIII coat protein moiety is intended to co-assemble with authentic mature VIII coat protein to produce phage particle having BPTI domains displayed on the surface. The source and character of the secretion signal sequence is not important because the signal sequence is cut away and degraded. The structural packaging signal, however, is quite important because it must co-assemble with the authentic coat protein to make a working virus sheath.

**a) Bacterial Alkaline Phosphatase (phoA) Signal Peptide.**

Construct pGEM-MB26 contains a SacI-AccIII fragment containing the new RBS and sequences encoding the initiating methionine and the signal peptide of M13 gene VIII pro-protein. This fragment was replaced with a duplex (annealed from four oligo-nts) containing the RBS and DNA coding for the initiating methionine and signal peptide of PhoA (INOU82); phage is pGEM-MB42. M13MB48 is a derivative of GemMB42. A BamHI-SalI DNA fragment from GenMB42, containing the gene construct, was ligated into a similarly cleaved vector M13MB1/2 giving rise to M13MB48.

PhoA RBS and signal peptide sequence

```
5'-GAGCTCCATGGGAGAAAATAAA.ATG.AAA.CAA.AGC.ACG.-
   |SacI|                     met lys gln ser thr

 .ATC.GCA.CTC.TTA.CCG.TTA.CTG.TTT.ACC.CCT.GTG.ACA.-
  ile ala leu leu pro leu leu phe thr pro val thr

           .AAA.GCC.CGT.CCG.GAT.-3'
            lys ala arg pro asp......
                       |AccIII|
```

**b) β-lactamase signal peptide.**

To allow transfer of the β-lactamase (amp) promoter and DNA coding for the signal peptide into the (mature-BPTI)::(mature-VIII-coat-protein) gene, we first introduced an AccIII site the amp gene adjacent to the codons for the β-lactamase signal peptide cleavage site ($C_{2504}$→T and $A_{2501}$→G); vector is pGEM-MB40. We then ligated a BamHI linker into the AatII site at nt 2260, 5' to the promoter; vector is pGEM-MB45. The BamHI-AccIII fragment now contains the amp promoter, amp RBS, initiating methionine and β-lactamase signal peptide. This fragment was used to replace the corresponding fragment from pGEM-MB26 to generate construct pGEM-MB46.

amp gene promoter and signal peptide sequences

```
5'-GGATCCGGTGGCACTTTTCGGGGAAATGTGCGCGGAACCCCTATTTGTT-

  TATTTTTCTAAATACATTCAAATATGTATCCGCTCATGAGACAATAACC-

  CTGATAAATGCTTCAATAATATTGAAAAAGGAAGAGT-

ATG.AGT.ATT.CAA.CAT.TTC.CGT.GTC.GCC.CTT.ATT.CCC.TTT.TTT.-
  met ser ile gln his phe arg val ala leu ile pro phe
phe

  GCG.GCA.TTT.TGC.CTT.CCT.GTT.TTT.GCT.CAT.CCG.-3'
  ala ala phe cys leu pro val phe ala his pro....
```

**c) M13-gene-III-signal::bpti::mature-VIII-coat-protein**

We may also construct M13-MB51 which would carry a $Km^R$ gene and a M13-gene-III-signal-peptide gene fragment fused to the previously described BPTI::mature-VIII-coat-protein gene fragment. Because M13-MB51 contains no gene III, the phage can not form plaques, but can render cells $Km^R$. Infectious phage particles can be obtained via helper phage. The gene III signal sequence is capable of directing (BPTI)::(mature-gene-III-protein) to the surface of phage (vide infra).

Summary of signal peptide fusion protein variants.

| | Promoter | RBS | Signal sequence | Fusion protein |
|---|---|---|---|---|
| pGEM-MB26 | tac | new | VIII | BPTI/VIII-coat |
| pGEM-MB42 | tac | new | phoA | BPTI/VIII-coat |
| pGEM-MB46 | amp | amp | amp | BPTI/VIII-coat |
| pGEM-MB51 (hypoth.) | III | III | III | BPTI/VIII-coat |
| M13 MB48 | tac | new | phoA | BPTI/VIII-coat |

2. Analysis of the Protein Products Encoded by the Synthetic (signal-peptide::mature-bpti::viii-coat-protein) Genes

**i) In vitro analysis**

A coupled transcription/translation prokaryotic system (Amersham Corp., Arlington Heights, IL) was utilized for the in vitro analysis of the protein products encoded by the BPTI/VIII synthetic gene and the derivatives.

Table 107 lists the protein products encoded by the listed vectors which are visualized by standard fluorography following in vitro synthesis in the presence of $^{35}$S-methionine and separation of the products using SDS polyacrylamide gel electrophoresis. In each sample, a pre-β-lactamase product (~31 kd) can be seen. This is derived from the amp gene which is the common selection gene for the vectors. In addition, a (pre-BPTI/VIII) product encoded by the synthetic gene and variants can be seen as indicated. The migration of these species (~14.5 kd) is consistent with the expected size of the encoded proteins.

**ii) In vivo analysis.**

The vectors detailed in sections (B) and (C) were freshly transfected into the E. coli strain XL1-blue (™) (Stratagene, La Jolla, CA) and in strain SEF'. E. coli strain SE6004 (LISS85) carries the prlA4 mutation and is more permissive in secretion than strains that carry the wild-type prlA. SE6004 is $F^-$ and is deleted for lacI; thus the cells can not be infected by M13 and lacUV5 and tac promoters can not be regulated with IPTG. Strain SEF' is derived from strain SE6004 (LISS85) by crossing with XL1-Blue(™); the F' in XL1-Blue(™) carries $Tc^R$ and $lacI^q$. SE6-004 is streptomycin$^R$, $Tc^S$ while XL1-Blue(™) is streptomycin$^S$, $Tc^R$ so that both parental strains can be killed with the combination of Tc and streptomycin. SEF' retains the secretion-permissive phenotype of the parental strain, SE6004(prlA4).

The fresh transfectants were grown in NZYCM medium (SAMB89) for 1 hour. IPTG was added over the range 1.0 μM to 0.5 mM (to derepress lacUV5 and tac) and grown for an additional 1.5 hours.

Aliquots of cells expressing the synthetic-insert encoded proteins together with controls (no vector, mock vector, and no IPTG) were lysed in SDS gel loading buffer and electrophoresed in 20% polyacrylamide gels containing SDS and urea. Duplicate gels were either silver stained (Daiichi, Tokyo, Japan) or electrotransferred to a nylon matrix (Immobilon from Millipore, Bedford, MA) for western analysis using rabbit anti-BPTI polyclonal antibodies.

Table 108 lists the interesting proteins seen by silver staining and western analysis of identical gels. We can see clearly by western analysis that IPTG-inducible protein species containing BPTI epitopes exist in the test strains which are absent from the control strains. In XL1-Blue(™), the migration of this species is predominantly that of the unprocessed form of the pro-protein althouah a small proportion of the protein appears to migrate at a size consistent with that of a fully processed form. In SEF', the processed form predominates, there being only a faint band corresponding to the unprocessed species.

Thus, in strain SEF', we have produced a tripartite fusion protein that is specifically cleaved after the secretion signal sequence. We believe that the mature protein comprises BPTI followed by the gene VIII coat protein and that the coat protein moiety spans the membrane. One or more copies, perhaps hundreds of copies, of this protein will co-assemble into M13 derived phage or M13-like phagemids. This construction will allow us to a) mutagenize the BPTI domain, b) display each of the variants on the coat of one or more phage (one type per phage), and c) recover those phage that display variants having novel binding properties with respect to target materials of our choice.

Rasched and Oberer (RASC86) report that phage produced in cells that express two alleles of gene VIII, that have differences within the first 11 residues of the mature coat protein, contain some of each protein. Thus, because we have achieved in vivo processing of the phoA(signal)::bpti::matureVIII fusion gene, it is highly likely that co-expression of this gene with wild-type VIII will lead to production of phage bearing BPTI domains on their surface. Mutagenesis of the bpti domain of these genes will provide a population of phage, each phage carrying a gene that codes for the variant of BPTI displayed on the phage surface.

**VIII Display Phage: Production, Preparation and Analysis.**

**i. Phage Production.**

The OCV can be grown in XL1-Blue(™) in the absence of IPTG. Typically, a plaque plug is taken from a plate and grown in 2 ml of medium, containing freshly diluted cells, for 6 to 8 hours. Following centrifugation of this culture, the supernatant is titered. This is kept as a phage stock for further infection, phage production, and display of the gene product of interest.

A 100-fold dilution of a fresh overnight culture of SEF' cells in 500 ml of NZCYM medium is allowed to grow to a cell density of 0.4 (Ab 600nm) in a shaker at 37°C. To this culture is added a sufficient amount of the phage stock to give a MOI of 10 together with IPTG to give a final concentration of 0.5 mM. The culture is allowed to grow for a further 2 hrs.

**ii. Phage Preparation and Purification.**

The phage-producing bacterial culture is centrifuged to separate the phage in the supernatant from the bacterial pellet. To the supernatant is added one quarter, by volume, of phage precipitation solution (20% PEG, 3.75 M ammonium acetate) and PMSF to a final concentration of 1mM. It is left on ice for 2 hours after which the precipitated phage is retrieved by centrifugation. The phage pellet is redissolved in TrisEDTA containing 0.1% Sarkosyl and left at 4˚C for 1 hour after which any bacteria and bacterial debris is removed by centrifugation. The phage in the supernatant is reprecipitated with PEG overnight at 4˚C. The phage pellet is resuspended in LB medium and repreciptated another two times to remove the detergent. The phage is stored in LB medium at 4˚C, titered, and used for analysis and binding studies.

A more stringent phage purification scheme involves centrifugation in a CsCl gradient (3.86 g of CsCl dissolved in NET buffer (0.1 M NaCl, 1mM EDTA, 0.1M Tris pH 7.7) to make 10 ml). $10^{12}$ to $10^{13}$ phage in TE Sarkosyl buffer are mixed with 5 ml of CsCl NET buffer and centrifuged overnight at 34K rpm in, for example, a Sorvall OTD-65B Ultracentrifuge. Aliquots of 400 μl are carefully removed. 5 μl aliqouts are removed from the fractions and analysed by agarose gel electrophoresis after heating at 65˚C for 15 minutes together with the gel loading buffer containing 0.1% SDS. Fractions containing phage are pooled, the phage reprecipitated and finally redissolved in LB medium to a concentration of $10^{12}$ to $10^{13}$ phage per ml.

**iii. Phage Analysis.**

The display phage are analyzed using standard methods of polyacrylamide gel electrophoresis and either silver staining of the gel or electrotransfer to a nylon matrix followed by analysis with anti-BPTI antiserum (Western analysis). Display of heterologous proteins is quantitated by comparison to serial dilutions of the starting protein, for example BPTI, together with the display phage samples in the electrophoresis and Western analyses. An alternative method involves running a 2-fold serial dilution of a phage in which both the major coat protein and the fusion protein are silver stained. Comparison of the ratios of the two protein species allows one to estimate the number of fusion proteins per phage since the number of VIII gene encoded proteins per phage (~3000) is know.

<u>Incorporation of fusion protein into bacteriophage.</u>

In vivo expression of the processed BPTI: VIII fusion protein, encoded by vectors GemMB42 and M13MB48, indicated that the processed fusion product is probably located within the cell membrane. Thus, it could be incorporated into the phage and that the BPTI moiety would be displayed at the phage surface.

SEF' cells were infected with either M13MB48 or M13mp18, as control. The resulting phage were electrophoresed (~ $10^{11}$ phage per lane) in a 20% polyacrylamide gel containing urea followed by electrotransfer to a nylon matrix and western analysis using anti-BPTI rabbit serum. A single species of protein was observed in phage derived from infection with the M13MB48 stock phage which was not observed in the control infection. This protein migrated at an apparent size of ~12 kd, consistent with that of the fully processed fusion protein.

Western analysis of SEF' bacterial lysate with or without phage infection demonstrated another species of protein of about 20kd. This species was also present, to a lesser degree, in phage preparations which were simply PEG precipitated without further purification (for example, using nonionic detergent or by CsCl gradient centrifugation). A comparison of M13MB48 phage preparations made in the presence or absence of detergent aldemonstrated that sarkosyl treatment and CsCl gradient purification did remove the bacterial contaminant while having no effect on the presence of the BPTI:VIII fusion protein. This indicates that the fusion protein has been incorporated and is a constituent of the phage body.

The time course of phage production and BPTI:VIII incorporation was followed post-infection and after IPTG induction. Phage production and fusion protein incorporation appeared to be maximal after two hours. This time course was utilized in further phage productions and analyses.

Polyacrylamide electrophoresis of the phage preparations, followed by silver staining, demonstrated that the preparations were essentially free of contaminating protein species and that an extra protein band was present in M13MB48 derived phage which was not present in the control phage. The size of the new protein was consistent with that seen by western analysis. A similar analysis of a serially diluted BPTI:VIII incorporated phage demonstrated that the ratio of fusion protein to major coat protein was typically about 1:150. Since the phage contains about 3000 copies of the gene VIII product, the phage population contains, on average, 10's of copies of fusion protein per phage.

**Altering the initiating methionine of the natural gene VIII.**

The OCV M13MB48 contains the synthetic gene encoding the BPTI:VIII fusion protein in the intergenic region of the modified M13mp18 phage vector. The remainder of the vector consists of the M13 genome. To increase the phage

incorporation of the fusion protein, we decided to diminish the production of the natural gene VIII product by altering the initiating methionine codon of this gene to CTG. In such cases, methionine is incorporated, but the rate of initiation is reduced. The change was achieved by site-specific oligonucleotide mutagenesis.

```
                          M   K   K   S   -rest of VIII
             ACT.TCC.TC.ATG.AAA.AAG.TCT.
 rest of XI  -  T   S   S stop

Site-specific mutagenesis.

                              (L)  K   K   S   -rest of
VIII
              ACT.TCC.AG.CTG.AAA.AAG.TCT.
  rest of XI  - T   S   S  stop
```

Analyses of the phage derived from this modified vector indicated that there was a significant increase in the ratio of fusion protein to major coat protein. Quantitative estimates indicated that within a phage population as much as 100 copies of the BPTI:VIII fusion were incorporated per phage.

Display of BPTI:VIII fusion protein by bacteriophage.

The BPTI:VIII fusion protein had been shown to be incorporated into the body of the phage. This phage was analyzed further to demonstrate that the BPTI moiety was accessible to specific antibodies and hence displayed at the phage surface.

We added purified polyclonal rabbit anti-BPTI IgG to a known titer of phage. Following incubation, protein A-agarose beads are added to bind IgG and left to incubate overnight. The IgG-protein A beads and any bound phage are removed by centrifugation followed by a retitering of the supernatant to determine loss of phage. The phage bound to the beads can also be acid eluted and titered. The assay includes controls, such as a WT phage stock (M13mp18) and IgG purified from normal rabbit pre-immune serum.

Table 140 shows that while the titer of the WT phage is unaltered by anti-BPTI IgG, BPTI-IIIM (positive control, vide infra), demonstrated a significant drop in titer with or without the extra addition of protein A beads. (Note, since the, BPTI moiety is part of gIIIp that binds phage to bacterial pili, this is expected.) Two batches of M13HB48 phage (containing the BPTI:VIII fusion protein) demonstrated a significant reduction in titer, as judged by pfus, when anti-BPTI antibodies and protein A beads were added. The initial drop in titer with the antibody alone, differs somewhat between the two batches of phage. Retrieval of the immunoprecipitated phage, while not quantitative, was significant when compared to the WT phage control.

Further controls are shown in Table 141 and Table 142. The data demonstrated that the loss in titer observed for the BPTI: VIII containing phage is a result of the display of BPTI epitopes by these phage and the specific interaction with anti-BPTI antibodies. No significant interaction with either protein A agarose beads or IgG purified from normal rabbit serum could be demonstrated. The larger drop in titer for M13MB48 batch five reflects the higher level incorporation of the fusion protein in this preparation.

Functionality of the BPTI moiety in the BPTI-VIII display phage.

The previous two sections demonstrated that the BPTI:VIII fusion protein has been incorporated into the phage body and that the BPTI moiety is displayed at the phage surface. To demonstrate that the displayed molecule is functional, binding experiments were performed in a manner almost identical to that described in the previous section except that proteases were used in place of antibodies. The display phage and controls are allowed to interact with immobilized proteases or immobilized inactivated proteases. Binding is assessed by the loss in titer of the display phage or by determining the phage bound to the beads.

Table 143 shows the results of an experiment in which BPTI.VIII display phage, M13MB48, were allowed to bind to anhydrotrypsin-agarose beads. There was a significant drop in titer compared to WT phage (no displayed BPTI): A pool of phage (5AA Pool), each contain a variegated 5 amino acid extension at the BPTI:major coat protein interface, demonstrated a similar decline in titer. In control (table 143), very little non-specific binding of the display phage was observed with agarose beads carrying an unrelated protein (streptavidin).

Actual binding of the display phage is demonstrated by the data shown for two experiments in Table 144. The

negative control is M13mp18 and the positive control is BPTI-IIIMK, a phage in which the BPTI moiety, attached to the gene III protein, has been shown to be displayed and functional. M13MB48 and M13MB56 both bind to anhydrotrypsin beads in a manner comparable to that of the positive control, being 40 to 60 times better than the negative control (non-display phage). Hence, functionality of the BPTI moiety, in the major coat fusion protein, was established.

Furthermore, Table 145 compares binding to active and inactivated trypsin by page. The control phage, M13mp18 and BPTI-III MK, demonstrated binding similar to that detailed elsewhere in the present application. Note that the relative binding is enhanced with trypsin due to the apparent marked reduction in the non-specific binding of the WT phage to the active protease. M13.3R7 and M13.3X11, which each contain 'EGGGS' linker extensions at the domain interface, bound to anhydrotrypsin and trypsin in a manner similar to BPTI-IIIMK phage. The binding, relative to non-display phage, was ~100-fold higher in the anhydrotrypsin binding assay and at least 1000-fold higher in the trypsin binding, assay. The binding of another 'EGGGS' linker variant (M13.3Xd) was similar to that of M13.3X7.

To demonstrate the specificity of binding the assays were repeated with human neutrophil elastase (HNE) beads and compared to that seen with trypsin beads Table 146. BPTI has a very high affinity for trypsin and a low affinity for HNE, hence the BPTI display phage should reflect these affinities when used in binding assays with these beads. The negative and positive controls for trypsin binding were as already described above while an additional positive control for the HNE beads, BPTI (K15L,MGNG)-III MA was included. The results, shown in Table 146, confirmed this prediction. M13MB48, M13.3X7 and M13.3X11 phage demonstrated good binding to trypsin, relative to WT phage and the HNE control (BPTI (K15L,MBNG) -III MA), being comparable to BPTI-IIIMK. page. Conversely poor binding occurred when HNE beads were used, with the exception of the HNE positive control phage.

Taken together the accumulated data demonstrated that when BPTI is part of a fusion protein with the major coat protein of M13 phage, the molecule is both displayed at the surface of the phage and a significant proportion of it is functional in a specific protease binding manner.

## EXAMPLE II

## CONSTRUCTION OF BPTI/GENE-III DISPLAY VECTOR

DNA manipulations were conducted according to standard procedures as described in Maniatis et al. (MANI82) or Sambrook et al. (SAMB89). First the lacZ gene of M13-MB1/2 was removed. M13-MB1/2 RF was cut with BamHI and Salt and the large fragment was isolated. The recovered 6819 bp fragment was filled in with Klenow enzyme, ligated to a HindIII 8mer linker, and used to transfect XL1-Blue(™) (Stratagene, La Jolla, CA) cells which were subsequently plated for plaque formation. RF DNA was prepared from chosen plaques and a clone, M13-MB1/2-delta, containing regenerated BamHI and SalI sites and a new HindIII site, all 500 bp upstream of the BglII site (6935), was picked.

A unique NarI site was introduced into codons 17 and 18 of gene III (changing the amino acids from H-S to G-A, Cf. Table 110) in M13-MB1/2-delta:

```
              13  14  15  16  17  18  19  20  21
              P   F   Y   S   H   S   A   E   T
          5'-ct ttc tat tct cac tcc gct gaa ac-3' wild-type
          3'-ga  aag  ata  aga  ccg  cgg  cga  ctt  tg-5'
mutagenizing oligo
          5'-ct ttc tat tct ggc gcc gct gaa ac-3' mutant
              P   F   Y   S   G   A   A   E   T
```

The presence of a unique NarI site at nucleotide 1630 was confirmed by restriction enzyme analysis; the new vector is M13-MB1/2-delta-NarI. Phage MK was made by cloning the 1.3 Kb BamHI $Km^R$ fragment from plasmid pUC4K (Pharmacia, Piscataway, NJ) into M13-MB1/2-delta-NarI. Phage MK grows as well as wild-type M13, indicating that the changes at the cleavage site of gene III protein are not detectably deleterious to the phage.

## INSERTION OF SYNTHETIC BPTI GENE

The BPTI-III , expression vector was constructed by standard means. The synthetic bpti-VIII fusion contains a NarI site that comprises the last two codons of the BPTI-encoding region. A second NarI site was introduced upstream of the BPTI-encoding region by ligating the adaptor shown to AccIII-cut M13-MB26:

```
5'-TATTCTGGCGCCCGT     -3'
3'-ATAAGACCGCGGGCAGGCC-5'
       |NarI|  |AccIII
```

The ligation sample was then restricted with NarI and a 180 bp DNA fragment encoding BPTI was isolated. RFDNA of phage MK was digested with NarI, dephosphorylated, and ligated to the 180 bp fragment. Ligation samples were used to transfect XL1-Blue(™) which were plated for $Km^R$ plaques. DNA, isolated from phage derived from plaques was test for hybridization to a $^{32}P$-phosphorylated double stranded DNA probe corresponding to the BPTI gene. Large scale RF preparations were made for clones exhibiting a strong hybridization signal. Restriction enzyme digestion analysis confirmed the insertion of a single copy of the synthetic BPTI gene into gene III of MK to generate phage MK-BPTI. Subsequent DNA sequencing confirmed that the sequence of the boti-III fusion gene is correct and that the correct reading frame is maintained. Table 116 shows the entire coding region, the translation into protein sequence, and the functional parts of the polypeptides chain.

**EXPRESSION OF THE BPTI-III FUSION GENE IN VITRO**

MK-BPTI RF DNA was added to a coupled prokaryotic transcription-translation extract (Amersham). Newly synthesized radiolabelled proteins were produced and subsequently separated by electrophoresis on a 15% SDS-polyacrylamide gel. The MK-BPTI DNA directs the synthesis of an unprocessed gene III fusion protein which is 7 Kd larger than the WT gene III, consistent with insertion of 58 amino acids of BPTI into gene III protein. We immunoprecipitated radiolabelled proteins from the cell-free prokaryotic extract. Neither rabbit anti (M13 -grene-VIII-protein) IgG nor normal rabbit IgG were able to immunoprecipitate the gene III protein encoded by either MK or MK-BPTI. However, rabbit anti-BPTI IgG is able to precipitate the gene III protein encoded by MK-BPTI but not by MK. This confirms that the increase in size of the III protein encoded by MK-BPTI is attributable to the insertion of the BPTI protein.

**WESTERN ANALYSIS**

Phage were recovered from cultures by PEG precipitation. To remove residual cells, recovered phage were resuspended in a high salt buffer and centrifuged, as per instructions for the MUTA-GENE(R) M13 in vitro Mutagenesis Kit (Catalogue Number 170-3571, Bio-Rad, Richmond, CA). Aliquots of phage (containing up to 40 μg of protein) were electrophoresed on a 12.5% SDS-urea-polyacrylamide gel and proteins were electro-transferred to a sheet of Immobilon. Western blots were developed using rabbit anti-BPTI serum, which had previously been incubated with an E. coli extract, followed by goat ant-rabbit antibody conjugated to alkaline phosphatase. An immunoreactive protein of 67 Kd is detected in preparations of the MK-BPTI but not the MK phage. The size of the immunoreactive protein is consistent with the predicted size of a processed BPTI-III fusion protein (6.4 Kd plus 60 Kd). These data indicate that BPTI-specific epitopes are presented on the surface of the MK-BPTI phage but not the MK phage.

**NEUTRALIZATION OF PHAGE TITER WITH AGAROSE-IMMOBILIZED ANHMRO-TRYPSIN**

Anhydro-trypsin is a derivative of trypsin having the active site serine converted to dehydroalanine. Anhydrotrypsin retains the specific binding of trypsin but not the protease activity. Unlike polyclonalantibodies, anhydro-trypsin is not expected to bind unfolded BPTI or incomplete fragment.

Phage MK-BPTI and MK were diluted to a concentration $1.4 \cdot 10^{12}$ particles per ml. in TBS buffer (PARM88) containing 1.0 mg/ml BSA. 30 μl of diluted phage were added to 2, 5, or 10 μl of a 50% slurry of agarose-immobilized anhydro-trypsin (Pierce Chemical Co., Rockford, IL) in TBS/BSA buffer. Following incubation at 25°C, aliquots were removed, diluted in ice cold LB broth and titered for plaque-forming units on a lawn of XL1-Blue(™) cells. Table 114 shows that incubation of the MK-BPTI phage with immobilized anhydro-trypsin results in a very significant loss in titer over a four hour period while no such effect is observed with the MK (control) phage. The reduction in phage titer is also proportional to the amount of immobilized anhydro-trypsin added to the MK-BPTI phage. Incubation with 5 μl of a 50% slurry of agarose-immobilized streptavidin (Sigma, St. Louis, MO) in TBS/BSA buffer does not reduce the titer of either the MK-BPTI or MK phage. These data are consistent with the presentation of a correctly-folded, functional BPTI protein on the surface of the MK-BPTI phage but not on the MK phage. Unfolded or incomplete BPTI domains are not expected to bind anhydro-trypsin. Furthermore, unfolded BPTI domains are expected to be non-specifically sticky.

## NEUTRALIZATION OF PERGE TITER WITE ANTI-BPTI ANTIBODY

MK-BPTI and MK phage were diluted to a concentration of 4-$10^3$ plaque-forming units per ml in LB broth. 15 μl of diluted phage were added to an equivalent volume of either rabbit anti-BPTI serum or normal rabbit serum (both diluted 10-fold in LB broth). Following incubation at 37°C, aliquots were removed, diluted by $10^4$ in ice-cold LB broth and titered for pfus on a lawn of XL1-Blue(™) Incubation of the MK BPTI phage with anti-BPTI serum results in a steady loss in titer over a two hour period while no such effect is observed with the MK phage. As expected, normal rabbit serum does not reduce the titer of either the MK-BPTI or the MK phage. Prior incubation of the anti-BPTI serum with authentic BPTI protein but not with an equivalent amount of E. coli protein, blocks the ability of the serum to reduce the titer of the MK-BPTI phase. These data are consistent with the presentation of BPTI-specific epitopes on the surface of the MK-BPTI phage but not the MK phage. More specifically, the data indicates that these BPTI epitopes are associated with the gene III protein and that association of this fusion protein with an anti-BPTI antibody blocks its ability to mediate the infection of cells.

## NEUTRALIZATION OF PHAGE TITER WITH TRYPSIN

MK-BPTI and MK phage were diluted to a concentration of $4 \cdot 10^3$ plaque-forming units per ml in LB broth. Diluted phage were added to an equivalent volume of trypsin diluted to various concentrations in LB broth. Following incubation at 37°C, aliquots were removed, diluted by $10^4$ in ice cold LB broth and titered for plaque-forming units on a lawn of XL1-Blue(™). Incubation of the MK-BPTI phage with 0.15 μg of trypsin results in a 70% loss in titer after two hours while only a 15% loss in titer is observed for MK phage. A reduction in the amount of trypsin added to phage results in a reduction in the loss of titer. However, at all trypsin concentrations investigated , the MK-BPTI phage are more sensitive to incubation with trypsin than the MK phage. Thus, association of the BPTI-III fusion protein displayed on the surface of the MK-BPTI phage with trypsin blocks its ability to mediate the infection of cells.

The reduction in titer of phage MK by trypsin is an example of a phenomenon that is probably general: proteasesy if present in sufficient quantity, will degrade proteins on the phage and reduce infectivity. The present application lists several means that can be used to overcome the problem.

## AFFINITY SELECTION SYSTEK

### Affinity Selection with Immobilized Anhydro-Trypain

MR-HPTI and MK phage were diluted to a concentration of $1.4 \cdot 10^{12}$ particles per ml in TBS buffer (PARM88) containing 1.0 mg/ml BSA. We added $4.0 \cdot 10^{10}$ phage to 5 μl of a 50% slurry of either agarose-immobilized anhydro-trypsin beads (Pierce Chemical Co.) or agarose-immobilized streptavidin beads (Sigma) in TBS/BSA. Following a 3 hour incubation at room temperature, the beads were pelleted by centrifugation for 30 seconds at 5000 rpm in a microfuge and the supernatant fraction was collected. The beads were washed 5 times with TBS/Tween buffer (PARM88) and after each wash the beads were pelleted by centrifugation and the supernatant was removed. Finally, beads were resuspended in elution buffer (0.1 N HCl containing 1.0 mg/ml BSA adjusted to pH 2.2 with glycine) and following a 5 minute incubation at room temperature, the beads were pelleted by centrifugation. The supernatant was removed and neutralized by the addition of 1.0 M Tris-HCl buffer, pH 8.0.

Aliquots of phage samples were applied to a Nytran membrane using a Schleicher and Schuell (Keene, NH) filtration minifold and phage DNA was immobilized onto the Nytran by baking at 80°C for 2 hours. The baked filter was incubated at 42°C for 1 hour in pre-wash solution (MANI82) and pre-hybridization solution (5Prime-3Prime, West Chester, PA). The 1.0 Kb NarI (base 1630)/XmnI (base 2646) DNA fragment from MK RF was radioactively labelled with $^{32}$P-dCTP using an oligolabelling kit (Pharmacia, Piscataway, NJ). The radioactive probe was added to the Nytran filter in hybridization solution (5Prime-3Prime) and, following overnight incubation at 42°C, the filter was washed and autoradiographed.

The efficiency of this affinity selection system can be semi-quantitatively determined using a dot-blot procedure. Exposure of MK-BPTI-phage-treated anhydro-trypsin beads to elution buffer releases bound NK-BPTI phage. Streptavidin, beads do not retain phage MK-BPTI. Anhydro-trypsin beads do not retain phage MK. In the experiment depicted in Table 115, we estimate that 20% of the total MK-BPTI phage were bound to 5 μl of the innobilized anhydro-trypsin and were subsequently recovered by washing the beads with elution buffer (pH 2.2 HCl/glycine). Under the same conditions, no detectable MK-BPTI phage were bound, and subsequently recovered from the streptavidin beads. The amount of MK-BPTI. phage recovered in the elution fraction is proportional to the amount of immobilized anhydro-trypsin added to the phage. No detectable MK phage were bound to either the immobilized anhydro-trypsin or streptavidin beads and no phage were recovered with elution buffer. These data indicate that the affinity selection system described above can be utilized to select for phage displaying a specific folded protein (in this case, BPTI). Unfolded or incomplete

BPTI domains are not expected to bind anhydro-trypsin.

**Affinity Selection with Anti-BPTI antibodies**

MK-BPTI and MK phage were diluted to a concentration of $1 \cdot 10^{10}$ particles per ml in Tris buffered saline solution (PARM88) containing 1.0 mg/ml BSA. Two$\cdot 10^2$ phage were added to 2.5 μg of either biotinylated rabbit anti-BPTI IgG in TBS/BSA or biotinylated rabbit anti-mouse antibody IgG (Sigma) in THS/BSA, and incubated overnight at 4°C. A 50% slurry of streptavidin-agarose (Sigma), washed three times with TBS buffer prior to incubation with 30 mg/ml BSA in TBS. buffer for 60 minutes at room temperature, was washed three times with TBS/Tween buffer (PARM88) and resuspended to a final concentration of 50% in this buffer. Samples containing phage and biotinylated IgG were diluted with TBS/Tween prior to the addition of streptavidin-agarose in TBS/Tween buffer. Following a 60 minute incubation at room temperature, streptavidin-agarose beads were pelleted by centrifugation for 30 seconds and the supernatant fraction was collected. The beads were washed 5 times with TBS/Tween buffer and after each wash, the beads were pelleted by centrifugation and the supernatant was removed. Finally, the streptavidin-agarose beads were resuspended in elution buffer (0.1 N HC1 containing 1.0 mg/ml BSA adjusted to pH 2.2 with glycine), incubated 5 minute at room temperature, and pelleted by centrifugation. The supernatant was removed and neutralized by the addition of 1.0 M Tris-HCl buffer, pH 8.0.

Aliquots of phage samples were applied to a Nytran membrane using a Schleicker and Schuell minifold apparatus. Phage DNA was immobilized onto the Nytran by baking at 80°C for 2 hours. Filters were washed for 60 minutes in pre-wash solution (MANI82) at 42°C then incubated at 42°C for 60 minutes in Southern pre-hybridization solution (5Prime-3Prime). The 1.0 Kb <u>Nar</u>I (1630bp)/<u>Xmn</u>I (2646 bp) DNA fragment from MK RF was radioactively labelled with $^{32}P$-αdCTP using an oligo-labelling kit (Pharmacia, Piscataway, NJ). Nytran membranes were transferred from pre-hybridization solution to Southern hybridization solution (SPrime-3Prime) at 42°C. The radioactive probe was added to the hybridization solution and following overnight incubation at 42°C, the filter was washed 3 times with 2 x SSC, 0.1% SDS at room temperature and once at 65°C in 2 x SSC, 0.1% SDS. Nytran membranes were subjected to autoradiography. The efficiency of the affinity selection system can be semi-quantitatively determined using the above dot blot procedure. Comparison of dots A1 and B1 or C1 and D1 indicates that the majority of phage did not stick to the streptavidin-agarose beads. Washing with TBS/Tween buffer removes the majority of phage which are non--specifically associated with streptavidin beads. Exposure of the streptavidin beads to elution buffer releases bound phage only in the case of MK-BPTI phage which have previously been incubated with biotinylated rabbit anti-BPTI IgG. This data indicates that the affinity selection system described above can be utilized to select for phage displaying a specific antigen (in this case BPTI). We estimate an enrichment factor of at least 40 fold based on the calculation

$$\text{Enrichment Factor} = \frac{\text{Percent MK-BPTI phage recovered}}{\text{Percent MK phage recovered}}$$

**EXAMPLE III**

**BPTI:VIII BOUNDARY EXTENSIONS.**

To increase the flexibility between BPTI and mature gVIIIp, we introduced codons for peptide extensions between these domains.

<u>Adding block extensions to the fusion protein interface.</u>

The M13 gene III product contains 'stalk-like' regions as implied by electron micrographic visualization of the bacteriophage (LOPE85). The predicted amino acid sequence of this protein contains repeating motifs, which include:

glu.gly.gly.gly.ser (EGGGS) seven times
gly·gly·gly·ser (GGGS) three times
glu.gly.gly.gly.thr (EGGGT) once.

The aim of this section was to insert, at the domain interface, multiple unit extensions which would mirror the repeating motifs observed in the III gene product.

Two synthetic oligonucleotides were synthesized. We picked the third base of these codons so that translation of the oligonucleotide in the opposite direction would yield SER. When annealed the synthetic oligonucleotides give the following unit duplex sequence (an EGGGS linker):

```
        E   G   G   G   S
5' C.GAG.GGA.GGA.GGA.TC     3'
3'     TC.CCT.CCT.CCT.AGG.C 5'
       (L) (S) (S) (S) (G)
```

The duplex has a common two base pair 5' overhang (GC) at either end of the linker which allows for both the ligation of multiple units and the ability to clone into the unique NarI recognition sequence present in OCV's M13MB48 and Gem MB42. This site is positioned within 1 codon of the DNA encoding the interface. The cloning of an EGGGS linker (or multiple linker) into the vector NarI site destroys this recognition sequence. Insertion of the EGGGS linker in reverse orientation leads .to insertion of GSSSL into the fusion protein.

Addition of a single EGGGS linker at the NarI site of the gene shown in Table 113 leads to the following gene:

```
 79  80  80a 80b 80c 80d 80e 81  82  83  84
 G   G   E   G   G   G   S   A   A   E   G
        -----------------
GGT.GGC.GAG.GGA.GGA.GGA.TCC.GCC.GCT.GAA.GGT
        -----------------
```

Note that there is no preselection for the orientation of the linker (s) inserted into the OCV and that multiple linkers of either orientation (with the predicted EGGGS or GSSSL amino acid sequence) or a mixture of orientations (inverted repeats of DNA) could occur.

A ladder of increasingly large multiple linkers was established by annealing and ligating the two starting oligonucleotides containing different proportions of 5' phosphorylated and non-phosphorylated ends. The logic behind this is that ligation proceeds from the 3' unphosphorylated end of an oligonucleotide to the 5' phosphorylated end of another. The use of a mixture of phosphorylated and non-phosphorylated oligonucleotides allows for an element of control over the extent of multiple linker formation. A ladder showing a range of insert sizes was readily detected by agarose gel electrophoresis spanning 15 bp (1 unit duplex-5 amino acids) to greater than 600 base pairs (40 ligated linkers-200 amino acids).

Large inverted repeats can lead to genetic instability. Thus we chose to remove them, prior to ligation into the OCV, by digesting the population of multiple linkers with the restriction enzymes AccIII or XhoI, since the linkers, when ligated 'head-to-head' or 'tail-to-tail', generate these recognition sequences. Such a digestion significantly reduces the range in sizes of the multiple linkers to between 1 and 8 linker units (i.e. between 5 and 40 amino acids in steps of 5), as assessed by agarose gel electrophoresis.

The linkers were ligated (as a pool of different insert sizes or as gel-purified discrete fragments) into NarI cleaved OCVs M13MB48 or GemMB42 using standard methods. Following ligation the restriction enzyme NarI was added to remove the self-ligating starting OCV (since linker insertion destroys the NarI recognition sequence). This mixture was used to transform XL-1 blue cells and appropriately plated for plaques (OCV M13MB48) or ampicillin resistant colonies (OCV GemMB42).

The transformants were screened using dot blot DNA analysis with one of two $^{32}P$ labeled oligonucleotide probes. One probe consisted of a sequence complementary to the DNA encoding the P1 loop of BPTI while the second had a sequence complementary to the DNA encoding the domain interface region. Suitable linker candidates would probe positively with the firs probe and negatively or poorly with the second. Plaque purified clones were used to generate phage stocks for binding analyses and BPTI display while the Rf DNA derived from phage inflected cells was used for restriction enzyme analysis and sequencing. Representative insert sequences of selected clones analyzed are as follows:

M13.3X4

```
(GG)C.GGA.TCC.TCC.TCC.CT(C.GCC)
      gly ser ser ser leu
```

(continued)

M13.3X7

(G C.GAG.GGA.GGA.GGA.TC(C.GCC)
glu gly gly gly ser

M13.3X11

(GG)C.GAG.GGA.GGA.GGA.TCC.GGA.TCC.TCC.
glu gly gly gly ser gly ser ser

TCC.CTC.GGA.TCC.TCC.TCC.CT(C.GCCC)
ser leu gly ser ser ser leu

These highly flexible oligomeric linkers are believed to be useful in joining a binding domain to the major coat (gene VIII) protein of filamentous phage to facilitate the display of the binding domain on the phage surface. They may also be useful in the construction of chimeric OSPs for other genetic packages as well.

**Incorporation of interdomain extension fusion proteins, into, page.**

A phage pool containing a variegated pentapeptide extension at the BPTI:coat protein interface was used to infect SEF' cells. Using the criteria of the previous section, we determined that extended fusion proteins were incorporated into phage. Gel electrophoresis of the generated phage, followed by silver staining or western analysis with anti-BPTI rabbit serum, demonstrated fusion proteins that migrated similarly to, but discernably slower than, the starting fusion protein.

With regard to the 'EGGGS linker extensions of the domain interface, individual phage stocks predicted to contain one or more 5-amino-acid unit extensions were analyzed in a similar fashion. The migration of the extended fusion proteins were readily distinguishable from the parent fusion protein when viewed by western analysis or silver staining. Those clones analyzed in more detail included M13.3X4 (which contains a single inverted EGGGS linker with a predicted amino acid sequence of GSSSL), M13.3X7 (which contains a correctly orientated linker with a predicted amino acid sequence of EGGGS), M13. 3X11 (which contains 3 linkers with an inversion and a predicted amino acid sequence for the extension of EGGGSGSSSLGSSSL) and M13.3Xd which contains an extension consisting of at least 5 linkers or 25 amino acids.

The extended fusion proteins were all incorporated into phage at high levels (on average 10's of copies per phage were present and when analyzed by gel electrophoresis migrated rates consistent with the predicted size of the extension. Clones M13.3X4 and M13.3X7 migrated at a position very similar to but discernably different from the parent fusion protein, while M13.3X11 and M13.3Xd **were markedly larger.**

**EXAMPLE IV**

**Peptide phage**

The following materials and methods were used in the examples which follow.

**1. Peptide Phage**

HPQ6, a putative disulfide-bonded mini-protein, was displayed on M13 phage as an insert in the gene III protein (gIIIp). M13 has about five copies of gIIIp per virion. The phage were constructed by standard methods. HPQ6 includes the sequence CHPQFPRC characteristic of Devlin's streptavidin-binding E peptide (DEVL90), as well as a $F.X_a$ recognition site (see Table 820). HPQ6 phage were shown to bind to streptavidin.

An unrelated display phage with no affinity for streptavidin, MRTN, was used as a control.

**2. Streptavidin.**

Commercially available immobilized to agarose beads (Pierce). Streptavidin (StrAv) immobilized to 6% beaded agarose at a concentration of 1 to 2 mg per ml gel, provided as a 50% slurry. Also available as free protein (Pierce) with a specific activity of 14.6 units per mg (1 unit will bind 1 μg of biotin). A stock solution of 1 mg per ml in PBS containing 0.01% azide is made.

**3. D-Biotin.**

Commercially available (Boehringer Mannheim) in crystallized form. A stock solution of 4 mM is made.

**4. Streptavidin coating of microtiter well plates.**

ïmmulon (#2 or #4) strips or plates are used. 100μL of StrAv stock is added to each 250 μL capacity well and incubated overnight at 4˚C. The stock is removed and replaced with 250 μL of PBS containing BSA at a concentration of 1 mg per mL and left at 4˚C for a further 1 hour. Prior to use in a phage binding assay the wells are washed rapidly 5 times with 250 μL of PBS containing 0.1% Tween.

**5. Binding Assays.**

<u>Bead Assay.</u>

Between 10 and 20 μL of the StrAv bead slurry (5 to 10 μL bead volume) is washed 3 times with binding buffer (TBS containing BSA at a concentration of 1 mg per mL) just prior to the binding assay. 50 to 100 μL of binding buffer containing control or peptide-display phage ($10^2$ to $10^{11}$ total plaque forming units - pfu's) is added to each microtube. Binding is allowed to proceed for 1 hour at room temperature using an end over end rotator. The beads are briefly centrifuged and the supernatant removed. The beads are washed a further 5 times with 1 mL of TBS containing 0.1% Tween, each wash consisting of as 5 min incubation and a brief centrifugation. Finally the bound phage are eluted from the StrAv beads by a 10 min incubation with pH 2 citrate buffer containing 1 mg per mL BSA which is subsequently neutralized with 260 μL of 1M tris pH 8. The number of phage present in each step is determined as plaque forming units (pfu's) following appropriate dilutions and plating in a lawn of F' containing *E. coli.*

<u>Plate Assay.</u>

To each StrAv-coated well is added 100 μL of binding buffer (PBS with 1 mg per mL BSA) containing a known quantity of phage (between $10^8$ and $10^{11}$ pfu's). Incubation proceed for 1 hr at room temperature, followed by removal of the non-bound phage and 10 rapid washes with PBS 0.1% Tween. The bound phage are eluted with 250 μL of pH2 citrate buffer containing 1 mg per mL BSA and neutralization with 60 μL of 1M tris pH 8. The number of phage present in each step is determined as plaque forming units (pfu's) following appropriate dilutions and plating in a lawn of F' containing *E. coli.*

**EXAMPLE V**

**Effect of Dithiothreitol (DTT) on display phage binding to streptavidia-agarose.**

<u>Preliminary control experiments.</u>

a. Use of HRP-conjugated biotin and streptavidin beads.

Binding capacity of StrAv agarose beads for HRP-conjugated biotin determined to be ~ 1 μg (equivalent to ~ 150 pmol biotin) per 5 μL beads (the amount used in these experiments).

b. Effect of DTT on HRP-conjugated biotin binding to StrAv beads.

5 μL of StrAv beads were incubated with 10 ng of HRP-biotin in binding buffer (TBS-BSA) in the presence of varying amounts of DTT (at least 99% reduced) . Following a 15 minute incubation at room temperature, the beads were washed two times in binding buffer and an HRP substrate added. Color development was allowed to proceed and noted in a semi-quantitative manner. Table 827 shows that the binding of biotinylated horseradish peroxidase (HRP) is not greatly affected by concentration of DTT below 20 mM. *N.B.* DTT concentrations of 20 and 50 mM also inhibited the interaction of HRP and substrate in the absence of StrAv beads hence having a general negative effect in this system.

<u>Effect of DTT on HPO6 display phage infectivity.</u>

$10^3$ pfus of HPQ6 were added to binding buffer (TBS-BSA) in the presence of different concentrations of DTT. Incubated at room temperature for 1 hour then diluted and plated to determine titer as pfus. Table 828 show the effect

of DTT on the infectivity of phage HPQ6. Hence, either DTT has no effect on phage infectivities over this range of concentrations or the effects are reversed on dilution of the phage. From these control experiments it is apparent that DTT can be used at concentrations below 10 mM in studies on the effect of reducing agents on peptide display phage binding to StrAv:

Stable 829 shows the effect of DTT on the binding of phage HPQ6 and MKTN to StrAv beads. The most significant effect-of DTT on HPQ6 binding to StrAv occurred between 0.1 and 1.0 mM DTT, a concentration at which no negative effects were observed in the preliminary control experiments. These results strongly indicate that, in the case of HPQ6 display phage, DTT has a marked effect on binding to StrAv and that the presence of a disulfide bridge within the displayed peptide is a requirement for good binding.

## EXAMPLE VI

## RELEASE OF STREPTAVIDIN-BOUND DISPLAY PHAGE BY FACTOR Xa CLEAVAGE

Phage HPQ6 contain a bovine $F.X_a$ recognition site (YIEGR/IV). In many instances, IEGR is sufficient recognition site for $F.X_a$, but we have extended the site in each direction to facilitate efficient cleavage. The effect of preincubating HPQ6 phage with $F.X_a$ on binding to StrAv beads is shown in Table 832. Thus while this concentration of $F.X_a$ (2.5 units) had no measurable effect on the titer of the treated, display phage it had a very marked effect on the ability of the treated display phage to bind to StrAv. This is consistent with the StrAv recognition sequence being removed by the action of $FX_a$ recognizing and cleaving the YIEGR/IV sequence.

Table 833 shows the effect of $FX_a$ treatment of HPQ6 following binding to StrAv. Is it possible to remove display phage bound to their target by the use of $FX_a$ in place of pH or chaotropic agent elution? HPQ6 display page were allowed to bind to StrAv then incubated either in $FX_a$ buffer or the same buffer together with 2.5 units of $FX_a$ for 3 hrs. The amount eluted was compared to the total number of phage bound as judged by a pH2 elution. Therefore, while the display phage are slowly removed in the buffer alone, the presence of $FX_a$ significantly increases this rate.

The removal of HPQ6 display phage from StrAv, by $FX_a$ was also studied as a function of the amount of enzyme added and the time of incubation, as shown in Table 834. *N.B.* at greater concentrations of the enzyme (1.2 U for 1 hour or 2.5 U for 2 hours), a loss in infectivity of the treated phage was noted as measured by pfus.

Table 10: Abundances obtained from various vgCodons.

A. Optimized fxS Codon, Restrained by [D] + [E] = [K]+[R]

| | T | C | A | G | |
|---|---|---|---|---|---|
| 1 \| | .26 | .18 | .26 | .30 | f |
| 2 \| | .22 | .16 | .40 | .22 | x |
| 3 \| | .5 | .0 | .0 | .5 | S |

| Amino acid | Abundance | | Amino acid | Abundance |
|---|---|---|---|---|
| A | 4.80% | | C | 2.86% |
| D | 6.00% | | E | 6.00% |
| F | 2.86% | | G | 6.60% |
| H | 3.60% | | I | 2.86% |
| K | 5.20%. | | L | 6.82% |
| M | 2.86% | | N | 5.20% |
| P | 2.88% | | Q | 3.60% |
| R | 6.82% | | S | 7.02% mfaa |
| T | 4.16% | | V | 6.60% |
| W | 2.86% | lfaa | Y | 5.20% |
| stop | 5.20% | | | |

[D] + [E] = [K] + [R] = .12

ratio = Abun (W) /Abun (S) = 0.4074

| i | $(1/ratio)^j$ | $(ratio)^j$ | stop-free |
|---|---|---|---|
| 1 | 2.454 | .4074 | .9480 |

(continued)

| i | $(1/ratio)^j$ | $(ratio)^j$ | | stop-free |
|---|---|---|---|---|
| 2 | 6.025 | .1660 | | .8987 |
| 3 | 14.788 | .0676 | | .8520 |
| 4 | 36.298 | .0275 | | .8077 |
| 5 | 89.095 | .0112 | | .7657 |
| 6 | 218.7 | $4.57 \cdot 10^{-3}$ | | .7258 |
| 7 | 536.8 | $1.86 \cdot 10^{4}$ | | .6881 |

B. Unrestrained, optimized

| | T | C | A | G |
|---|---|---|---|---|
| 1 \| | .27 | .19 | .27 | .27 |
| 2 \| | .21 | .15 | .43 | .21 |
| 3 \| | .5 | .0 | .0 | .5 |

| Amino acid | Abundance | | Amino acid | Abundance |
|---|---|---|---|---|
| A | 4.05% | | C | 2.84% |
| D | 5.81% | | E | 5.81% |
| F | 2.84% | | G. | 5.67% |
| H | 4.08% | | I | 2.84% |
| K | 5.81% | | L | 6.83% |
| M | 2.84% | | N | 5.81% |
| P | 2.85% | | Q | 4.08% |
| R | 6.83% | | S | 6.89% mfaa |
| T | 4.05% | | V | 5.67% |
| W | 2.84% | lfaa | Y | 5.81% |
| stop | 5.81% | | | |

[D] + [E] = 0.1162 [K] + [R] - 0.1264

ratio = Abun (W) /Abun (S) = 0.41176

| i | $(1/ratio)^j$ | $(ratio)^j$ | stop-free |
|---|---|---|---|
| 1 | 2.4286 | .41176 | .9419 |
| 2 | 5.8981 | .16955 | .8872 |
| 3 | 14.3241 | .06981 | .8356 |
| 4 | 34.7875 | 34.7875 | .7871 |
| 5 | 84.4849 | .011836 | .74135 |
| 6 | 205.180 | .004874 | .69828 |
| 7 | 498.3 | $2.007 \cdot 10^{-3}$ | .6577 |

C. Optimized NNT

| | T | C | A | G |
|---|---|---|---|---|
| 1 \| | .2071 | .2929 | .2071 | .2929 |
| 2 \| | .2929 | .2071 | .2929 | .2071 |
| 3 \| | 1. | .0 | .0 | .0 |

| Amino acid | Abundance | Amino acid | Abundance |
|---|---|---|---|
| A | 6.06% | C | 4.29% lfaa |
| D | 8.58% | E | none |
| F | 6.06% | G | 6.06% |

(continued)

| Amino acid | Abundance | | Amino acid | Abundance |
|---|---|---|---|---|
| H | 8.58% | | I | 6.06% |
| K | none | | L | 8.58% |
| M | none | | N | 6.06% |
| P | 6.06% | | Q | none |
| R | 6.06% | | S | 8.58% mfaa |
| T | 4.29% | lfaa | V | 8.58% |
| W | none | | Y | 6.06% |
| stop | none | | | |

| i | (1/ratio)$^j$ | (ratio)$^j$ | stop-free |
|---|---|---|---|
| 1 | 2.0 | .5 | 1. |
| 2 | 4.0 | .25 | 1. |
| 3 | 8.0 | .125 | 1. |
| 4 | 16.0 | .0625 | 1. |
| 5 | 32.0 | .03125 | 1. |
| 6 | 64.0 | .015625 | 1. |
| 7 | 128.0 | .0078125 | 1. |

D. Optimized NNG

| | T | C | A | G |
|---|---|---|---|---|
| 1 \| | .23 | .21 | .23 | .33 |
| 2 \| | .215 | .285 | .285 | .215 |
| 3 \| | .0 | .0 | .0 | 1.0 |

| Amino acid | Abundance | | Amino acid | Abundance |
|---|---|---|---|---|
| A | 9.40% | | C | none |
| D | none | | E | 9.40% |
| F | none | | G | 7.10% |
| H | none | | I | none |
| K | 6.60% | | L | 9.50% mfaa |
| M | 4.90% | | N | none |
| P | 6.00% | | Q | 6.00% |
| R | 9.50% | | S | 6.60% |
| T | 6.6 % | | V | 7.10% |
| W | 4.90% | lfaa | Y | none |
| stop | 6.60% | | | |

| i | (i/ratio)$^j$ | (ratio)$^j$ | stop-free |
|---|---|---|---|
| 1 | 1.9388 | .51579 | 0.934 |
| 2 | 3.7588 | .26604 | 0.8723 |
| 3 | 7.2876 | .13722 | 0.8148 |
| 4 | 14.1289 | .07078 | 0.7610 |
| 5 | 27.3929 | $3.65 \cdot 10^{-2}$ | 0.7108 |
| 6 | 53.109 | $1.88 \cdot 10^{-2}$ | 0.6639 |
| 7 | 102.96 | $9.72 \cdot 10^{-3}$ | 0.6200 |

(continued)

E. Unoptimized NNS : (NNK gives identical distribution)

| | T | C | A | G |
|---|---|---|---|---|
| 1 \| | .25 | .25 | .25 | .25 |
| 2 \| | .25 | .25 | .25 | .25 |
| 3 \| | .0 | .5 | .0 | 0.5 |

| Amino acid | Abundance | Amino acid | Abundance |
|---|---|---|---|
| A | 6.25% | C | 3.125% |
| D | 3.125% | E | 3.125% |
| F | 3.125% | G | 6.25% |
| H | 3.125% | I | 3.125% |
| K | 3.125% | L | 9.375% |
| M | 3.125% | N | 3.125% |
| P | 6.25% | Q | 3.125% |
| R | 9.375% | S | 9.375% |
| T | 6.25% | V | 6.25% |
| W | 3.125% | Y | 3.125% |
| stop | 3.125% | | |

| i | $(1/\text{ratio})^j$ | $(\text{ratio})^j$ | stop-free |
|---|---|---|---|
| 1 | 3.0 | .33333 | .96875 |
| 2 | 9.0 | .11111 | .9385 |
| 3 | 27.0 | .03704 | .90915 |
| 4 | 81.0 | .01234567 | .8807 |
| 5 | 243.0 | .0041152 | .8532 |
| 6 | 729.0 | $1.37 \cdot 10^{-3}$ | .82655 |
| 7 | .2187.0 | $4.57 \cdot 10^{-4}$ | .8007 |

Table 102b : Annotated Sequence of gene after insertion of SalI linker

nucleotide

number

5'-(GGATCC TCTAGA GTC) GGC- 3

from pGEM polylinker

tttaca CTTTATGCTTCCGGCTCG tataat GTGTGG- 39

-35 lacUV5 -10

aATTGTGAGCGcTcACAATT- 59

lacO-symm operator

gagctc AGAGG CttaCT- 77

SacI Shine-Dalgarno seq.

|fM | K | K | S | L | V | L | K | A | S |

| 1 | 2 | 3 | 4 | 5 | 6 | 7 | 8 | 9 | 10|

|ATG|AAG|AAA|TCT|CTG|GTT|CTT|AAG|GCT|AGC|- 107

```
                          | Afl II| Nhe I |

| V | A | V | A | T | L | V | P | M | L |
| 11| 12| 13| 14| 15| 16| 17| 18| 19| 20|
|GTT|GCT|GTC|GCG|ACC|CTG|GTA|CCT|ATG|TTG|-      137
        | Nru I|      | Kpn I|


| S | F | A | R | P | D | F | C | L | E |
| 21| 22| 23| 24| 25| 26| 27| 28| 29| 30|
|TCC|TTC|GCT|CGT|CCG|GAT|TTC|TGT|CTC|GAG|-      167
          ↑   |AccIII|            | Ava I |
M13/BPTI Jnct                     | Xho I |


| P | P | Y | T | G | P | C | K | A | R |
| 31| 32| 33| 34| 35| 36| 37| 38| 39| 40|
|CCA|CCA|TAC|ACT|GGG|CCC|TGC|AAA|GCG|CGC|_      197
   | PflM I        |     ||     |BssH II|
              | Apa I ||
              | Dra II |
              | Pss I  |
```

## Table 102b : Annotated Sequence of gene after insertion of SalI linker (continued)

| | | | | | | | | | | | |
|---|---|---|---|---|---|---|---|---|---|---|---|
| | I | I | R | Y | F | Y | N | A | K | A | |
| | 41 | 42 | 43 | 44 | 45 | 46 | 47 | 48 | 49 | 50 | |
| | ATC | ATC | CGC | TAT | TTC | TAC | AAT | GCT | AAA | GC | - 226 |

| | | | | | | | | | | | |
|---|---|---|---|---|---|---|---|---|---|---|---|
| | G | L | C | Q | T | F | V | Y | G | G | |
| | 51 | 52 | 53 | 54 | 55 | 56 | 57 | 58 | 59 | 60 | |
| A | GGC | CTG | TGC | CAG | ACC | TTT | GTA | TAC | GGT | GGT | - 257 |
| Stu I | Stu I | | | | | | Acc I | Acc I | | | |
| | | | | | | | Xca I | Xca I | | | |

| | | | | | | | | | | |
|---|---|---|---|---|---|---|---|---|---|---|
| | C | R | A | K | R | N | N | F | K | |
| | 61 | 62 | 63 | 64 | 65 | 66 | 67 | 68 | 69 | |
| | TGC | CGT | GCT | AAG | CGT | AAC | AAC | TTT | AAA | - 284 |
| | | | Esp I | Esp I | | | | | | |

| | | | | | | | | | | | |
|---|---|---|---|---|---|---|---|---|---|---|---|
| | S | A | E | D | C | M | R | T | C | G | |
| | 70 | 71 | 72 | 73 | 74 | 75 | 76 | 77 | 78 | 79 | |
| | TCG | GCC | GAA | GAT | TGC | ATG | CGT | ACC | TGC | GGT | - 314 |
| | XmaIII | XmaIII | | | Sph I | Sph I | | | | | |

```
BPTI/M13 boundary
   v|
| G | A | A | E | G | D | D | P | A | K | A | A |
| 80| 81| 82| 83| 84| 85| 86| 87| 88| 89| 90| 91|
|GGC|GCC|GCT|GAA|GGT|GAT|GAT|CCG|GCC|AAG|GCG|GCC|- 350
| Bbe I |                           |   Sfi I         |
| Nar I |


| F | N | S | L | Q | A | S | A | T |
| 92| 93| 94| 95| 96| 97| 98| 99|100|
|TTC|AAT|TCT|CTG|CAA|GCT|TCT|GCT|ACC|-           377
                |Hind 3|


| E | Y | I | G | Y | A | W |
|101|102|103|104|105|106|107|
|GAG|TAT|ATT|GGT|TAC|GCG|TGG|-                   398

  | A | M | V | V | V | I | V | G | A |
  |108|109|110|111|112|113|114|115|116|
  |GCC|ATG|GTG|GTG|GTT|ATC|GTT|GGT|GCT|-     425
    | BstX I       |
    | Nco I|
```

**Table 102b: Annotated Sequence after insertion of SalI linker (continued)**

```
|T|I|G|I|
|117|118|119|120|
|ACC|ATC|GGG|ATC|-                                437

|K|L|F|K|K|F|T|S|K|A|
|121|122|123|124|125|126|127|128|129|130|
|AAA|CTG|TTC|AAG|AAG|TTT|ACT|TCG|AAG|GCG|-       467
                              |Asu II|

|S|.|.|.|
|131|132|133|134|
|TCT|TAA|TGA|TAG|    GGTTACC-                     486
                     BstE II

AGTCTA AGCCCGC CTAATGA GCGGGCT TTTTTTTT-          521
    terminator

aTCGA    GACctgca GGTCGACC ggcatgc-3'
                  |SalI|
```

Note the following enzyme equivalences,

| | | | |
|---|---|---|---|
| Xma III | = Eag I | Acc III | = BspM II |
| Dra II | = EcoO109 I | Asu II | = BstB I |

Table 106: Signal Peptides

| | |
|---|---|
| PhoA | M **K** q s t i a l a l l p l l f t p v t **K** A /**R** T... (17) |
| MalE | M **K I K** T G A **R** i l a l s a l t t m m f s a a a l a /**K** I... (18) |
| OmpF | M M **K R** n i l a v i v p a l l v a g t a n a /a **E**... (19) |
| Bla | M S I Q H F **R** v a l i p f f a a f c l p v f a /h p... (>18) |
| LamB | M M I T L **R K** l p l a v a v a a g v m s a q a m a /v **D**... (19) |
| Lpp | M **K** A T **K** l v l g a v i l g s t l l a g /c s... (>17) |
| gpIII | M **K K** l l f a i p l v v p f y s h s /a **E** T V **E**... (16) |
| gpIII-BPTI | M **K K** l l f a i p l v v p f y s g a /**R** P **D**... (15) |
| gpVIII | M **K K** S L V L **K** a s v a v a t l v p m l s f a /a **E** G **D D**... (16) |
| gpVIII-BPTI | M **K K** S L V L **K** a s v a v a t l v p m l s f a /**R** P **D**.... (15) |
| gpVIII' | M **K K** s l v l l a s v a v a t l v p m l s f a /a E **G D D**... (21) |

Table 107: In vitro transcription/translation analysis of vector-encoded signal::BPTI::mature VIII protein species

| | 31 kd species[a] | 14.5 kd species[b] |
|---|---|---|
| No DNA (control) | -[c] | - |
| pGSN-3Zf (-) | + | - |
| pGBM-MB16 | + | - |
| pGEM-MB20 | + | + |
| pGBM-MB26 | + | + |
| pGEM-MB42 | + | + |
| pGEM-MB46 | ND | ND |

Notes:
a.) pre-beta-lactamase, encoded by the amp (bla) gene.
b.) pre-BPTI/VIII peptides encoded by the synthetic gene and derived constructs.
c.) - for absence of product; + for presence of product; ND for Not Determined.

Table 108: Western analysis[a] of in vivo expressed signal::BPTI::mature VIII protein species

A) expression in strain XL1-Blue

| | signal | 14.5 kd species[b] | 12 kd species[c] |
|---|---|---|---|
| pGEM-3Zf(-) | - | -[d] | - |
| pGEM-MB16 | VIII | - | - |
| pGEM-MB20 | VIII | ++ | - |
| pGEM-MB26 | VIII | +++ | +/- |
| pGEM-MB42 | phoA | ++ | + |

B) expression in strain SEF'

(continued)

| | signal | 14.5 kd species[b] | 12 kd species[c] |
|---|---|---|---|
| pGEM-MB42 | phoA | +/- | +++ |

Notes:
a) Analysis using rabbit anti-BPTI polyclonal antibodies and horse-radish-peroxidase-conjugated goat anti-rabbit IgG antibody.
b) pro-BPTI/VIII peptides encoded by the synthetic gene and derived constructs.
c) processed BPTI/VIII peptide encoded by the synthetic gene.
d) not present ..... - weakly present ..... +/- present ..... + strong presence .... ++ very strong presence +++

Table 109: M13 gene III

1579 5'-GT GAAAAAATTA TTATTCGCAA TTCCTTTAGT
1611 TGTTCCTTTC TATTCTCACT CCGCTGAAAC TGTTGAAAGT
1651 TGTTTAGCAA AACCCCATAC AGAAAATTCA TTTACTAACG
1691 TCTGGAAAGA CGACAAAACT TTAGATCGTT ACGCTAACTA
1731 TGAGGGTTGT CTGTGGAATG CTACAGGCGT TGTAGTTTGT
1771 ACTGGTGACG AAACTCAGTG TTACGGTACA TGGGTTCCTA
1811 TTGGGCTTGC TATCCCTGAA AATGAGGGTG GTGGCTCTGA
1851 GGGTGGCGGT TCTGAGGGTG GCGGTTCTGA GGGTGGCGGT
1891 ACTAAACCTC CTGAGTACGG TGATACACCT ATTCCGGGCT
1931 ATACTTATAT CAACCCTCTC GACGGCACTT ATCCGCCTGG
1971 TACTGAGCAA AACCCCGCTA ATCCTAATCC TTCTCTTGAG
2011 GAGTCTCAGC CTCTTAATAC TTTCATGTTT CAGAATAATA
2051 GGTTCCGAAA TAGGCAGGGG GCATTAACTG TTTATACGGG
2091 CACTGTTACT CAAGGCACTG ACCCCGTTAA AACTTATTAC
2131 CAGTACACTC CTGTATCATC AAAAGCCATG TATGACGCTT
2171 ACTGGAACGG TAAATTCAGA GACTGCGCTT TCCATTCTGG
2211 CTTTAATGAG GATCCATTCG TTTGTGAATA TCAAGGCCAA
2251 TCGTCTGACC TGCCTCAACC TCCTGTCAAT GCTGGCGGCG
2291 GCTCTGGTGG TGGTTCTGGT GGCGGCTCTG AGGGTGGTGG
2331 CTCTGAGGGT GGCGGTTCTG AGGGTGGCGG CTCTGAGGGA
2371 GGCGGTTCCG GTGGTGGCTC TGGTTCCGGT GATTTTGATT
2411 ATGAAAAGAT GGCAAACGCT AATAAGGGGG CTATGACCGA
2451 AAATGCCGAT GAAAACGCGC TACAGTCTGA CGCTAAAGGC
2491 AAACTTGATT CTGTCGCTAC TGATTACGGT GCTGCTATCG
2531 ATGGTTTCAT TGGTGACGTT TCCGGCCTTG CTAATGGTAA
2571 TGGTGCTACT GGTGATTTTG CTGGCTCTAA TTCCCAAATG
2611 GCTCAAGTCG GTGACGGTGA TAATTCACCT TTAATGAATA
2651 ATTTCCGTCA ATATTTACCT TCCCTCCCTC AATCGGTTGA
2691 ATGTCGCCCT TTTGTCTTTA GCGCTGGTAA ACCATATGAA
2731 TTTTCTATTG ATTGTGACAA AATAAACTTA TTCCGTGGTG
2771 TCTTTGCGTT TCTTTTATAT GTTGCCACCT TTATGTATGT
2811 ATTTTCTACG TTTGCTAACA TACTGCGTAA TAAGGAGTCT
2851 TAATCATGCC AGTTCTTTTG GGTATTCCGT

**Table 110: Introduction of NarI into gene III**

**A) Wild-type III, portion encoding the signal peptide**

| | | | | | | | | | | |
|---|---|---|---|---|---|---|---|---|---|---|
| | M | K | K | L | L | F | A | I | P | L |
| | 1 | 2 | 3 | 4 | 5 | 6 | 7 | 8 | 9 | 10 |
| 1579 | 5'-GTG | AAA | AAA | TTA | TTA | TTC | GCA | ATT | CCT | TTA |

/ Cleavage site (between 18 and 19)

| | | | | | | | | | | | | |
|---|---|---|---|---|---|---|---|---|---|---|---|---|
| | V | V | P | F | Y | S | H | S | A | E | T | V |
| | 11 | 12 | 13 | 14 | 15 | 16 | 17 | 18 | 19 | 20 | 21 | 22 |
| 1609 | GTT | GTT | CCT | TTC | TAT | TCT | CAC | TCC | GCT | GAA | ACT | GTT-3' |

**B) III, portion encoding the signal peptide with NarI site**

| | | | | | | | | | | |
|---|---|---|---|---|---|---|---|---|---|---|
| | m | k | k | l | l | f | a | I | p | l |
| | 1 | 2 | 3 | 4 | 5 | 6 | 7 | 8 | 9 | 10 |
| 1579 | 5'-gtg | aaa | aaa | tta | tta | ttc | gca | att | cct | tta |

/ cleavage site (between 18 and 19)

| | | | | | | | | | | | | |
|---|---|---|---|---|---|---|---|---|---|---|---|---|
| | v | v | p | f | y | s | G | A | a | e | t | v |
| | 11 | 12 | 13 | 14 | 15 | 16 | 17 | 18 | 19 | 20 | 21 | 22 |
| 1609 | gtt | gtt | cct | ttc | tat | tct | GGc | Gcc | gct | gaa | act | gtt-3' |

### Table 113 : Annotated Sequence of pGEM-MB42 comprising Ptac::RBS(GGAGGAAATAAA):: phoA-signal::mature-bpti::mature-VIII-coat-protein

```
5'-GGATCC actccccatcccc
   |    |
   BamHI


ctg TTGACA attaatcatcgGCTCG tataat GTGTGG-
     -35            tac        -10

   aATTGTGAGCGcTCACAATT-
    lacO-symm operator

                                   | M | K | Q | S | T |
                                   | 1 | 2 | 3 | 4 | 5 |
   GAGCTCCATGGGAGAAAATAAA|ATG|AAA|CAA|AGC|ACG|-
   |SacI|                |<----- phoA signal peptide

 | I | A | L | L | P | L | L | F | T | P | V | T |
 | 6 | 7 | 8 | 9 | 10| 11| 12| 13| 14| 15| 16| 17|
 |ATC|GCA|CTC|TTA|CCG|TTA|CTG|TTT|ACC|CCT|GTG|ACA|-
 ---------------- phoA signal continues ---------

     (There are no residues 20-23.)

     | K | A | R | P | D | F | C | L | E |
     | 18| 19| 24| 25| 26| 27| 28| 29| 30|
     |AAA|GCC|CGT|CCG|GAT|TTC|TGT|CTC|GAG|-
 phoA signal->^|  |AccIII|         | Ava I |
 phoA/BPTI Jnct                    | Xho I |
               |<----- BPTI insert ---------

 | P | P | Y | T | G | P | C | K | A | R |
 | 31| 32| 33| 34| 35| 36| 37| 38| 39| 40|
 |CCA|CCA|TAC|ACT|GGG|CCC|TGC|AAA|GCG|CGC|-
    | PflM I      |  ||        |BssH II|
                  | Apa I |
                  | Dra II |
                  | Pss I  |
```

**Table 113 : Annotated Sequence of Ptac::RBS(GGAGGAAATAAA):: phoA-signal::mature-bpti::mature-VIII-coat-protein gene (continued)**

```
 |  I|  I|  R|  Y|  F|  Y|  N|  A|  K|  A|
 | 41| 42| 43| 44| 45| 46| 47| 48| 49| 50|
 |ATC|ATC|CGC|TAT|TTC|TAC|AAT|GCT|AAA|GC |-

 |  G|  L|  C|  Q|  T|  F|  V|  Y|  G|  G|
 | 51| 52| 53| 54| 55| 56| 57| 58| 59| 60|
A|GGC|CTG|TGC|CAG|ACC|TTT|GTA|TAC|GGT|GGT|-
| Stu I|                  | Acc I |
                          | Xca I |

 |  C|  R|  A|  K|  R|  N|  N|  F|  K|
 | 61| 62| 63| 64| 65| 66| 67| 68| 69|
 |TGC|CGT|GCT|AAG|CGT|AAC|AAC|TTT|AAA|-
         | Esp I  |

 |  S|  A|  E|  D|  C|  M|  R|  T|  C|  G|
 | 70| 71| 72| 73| 74| 75| 76| 77| 78| 79|
 |TCG|GCC|GAA|GAT|TGC|ATG|CGT|ACC|TGC|GGT|-
  |XmaIII|        | Sph I|
-------------- BPTI insert----------------

         BPTI/M13 boundary
 |  G|  A|  A|  E|  G|  D|  D|  P|  A|  K|  A|  A|
 | 80| 81| 82| 83| 84| 85| 86| 87| 88| 89| 90| 91|
 |GGC|GCC|GCT|GAA|GGT|GAT|GAT|CCG|GCC|AAG|GCG|GCC|-
 | Bbe I  |                    |    Sfi I       |
 | Nar I  |
-- BPTI-->|<----- mature gene VIII coat protein ----

 |  F|  N|  S|  L|  Q|  A|  S|  A|  T|
 | 92| 93| 94| 95| 96| 97| 98| 99|100|
 |TTC|AAT|TCT|CTG|CAA|GCT|TCT|GCT|ACC|-
                  |Hind 3|

 |  E|  Y|  I|  G|  Y|  A|  W|
 |101|102|103|104|105|106|107|
 |GAG|TAT|ATT|GGT|TAC|GCG|TGG|-
```

**Table 113 : Annotated Sequence of Ptac::RBS(GGAGGAAATAAA):: phoA-signal::mature-bpti::mature-VIII-coat-protein gene (continued)**

| A | M | V | V | V | I | V | G | A | |
|---|---|---|---|---|---|---|---|---|---|
| 108 | 109 | 110 | 111 | 112 | 113 | 114 | 115 | 116 | |
| GCC | ATG | GTG | GTG | GTT | ATC | GTT | GGT | GCT | - |

|BstX I|

|Nco I|

| T | I | G | I | |
|---|---|---|---|---|
| 117 | 118 | 119 | 120 | |
| ACC | ATC | GGG | ATC | - |

| K | L | F | K | K | F | T | S | K | A | |
|---|---|---|---|---|---|---|---|---|---|---|
| 121 | 122 | 123 | 124 | 125 | 126 | 127 | 128 | 129 | 130 | |
| AAA | CTG | TTC | AAG | AAG | TTT | ACT | TCG | AAG | GCG | - |

|Asu II|

| S | . | . | : |
|---|---|---|---|
| 131 | 132 | 133 | 134 |
| TCT | TAA | TGA | TAG |

GGTTACC-
BstE II

AGTCTA AGCCCGC CTAATGA GCGGGCT TTTTTTTT-
terminator

aTCGA GACctgca GGTCGAC-3'
|SalI|

Table 114: Neutralization of Phage Titer Using Agarose-immobilized Anhydro-Trypsin

| | | Percent Residual Titer As a Function of Time (hours) | | |
|---|---|---|---|---|
| Phage Type | Addition | 1 | 2 | 4 |
| MK-BPTI | 5 μl IS | 99 | 104 | 105 |
| | 2 μl IAT | 82 | 71 | 51 |
| | 5 μl IAT | 57 | 40 | 27 |
| | 10 μl IAT | 40 | 30 | 24 |
| MK | 5 μl IS | 106 | 96 | 98 |
| | 2 μl IAT | 97 | 103 | 95 |
| | 5 μl IAT | 110 | 111 | 96 |
| | 10 μl IAT | 99 | 93 | 106 |

Legend: IS = Immobilized streptavidin
IAT = Immobilized anhydro-trypsin

Table 115: Affinity Selection of MK-BPTI Phage on Immobilized Anhydro-Trypsin

| Phage Type | Addition | Percent of Total Phage Recovered in Elution Buffer |
|---|---|---|
| MK-BPTI | 5 μl IS | <<1[a] |
| | 2 μl IAT | 5 |
| 5 μl | IAT | 20 |
| | 10 μl | IAT 50 |
| MK | 5 μl IS | <<1[a] |
| | 2 μl IAT | <<1 |
| 5 | μl TAT | <<1 |
| | 10 μl IAT | <<1 |

Legend: IS = Immobilized streptavidin

IAT = Immobilized anhydro-trypsin

[a] not detectable.

**Table 116: translation of Signal-III::bpti::mature-III**

```
  1   2   3   4   5   6   7   8   9  10  11  12  13  14  15
 fM   K   K   L   L   F   A   I   P   L   V   V   P   F   Y
GTG AAA AAA TTA TTA TTC GCA ATT CCT TTA GTT GTT CCT TTC TAT
|<------- gene III signal peptide -------------------------

 16  17  18  19  20  21  22  23  24  25  26  27  28  29  30
  S   G   A   R   P   D   F   C   L   E   P   P   Y   T   G
TCT GGC GCC cgt ccg gat ttc tgt ctc gag cca cca tac act ggg
----------->|<----- BPTI insertion ------------------------

 31  32  33  34  35  36  37  38  39  40  41  42  43  44  45
  P   C   K   A   R   I   I   R   Y   F   Y   N   A   K   A
ccc tgc aaa gcg cgc atc atc cgc tat ttc tac aat gct aaa gca

 46  47  48  49  50  51  52  53  54  55  56  57  58  59  60
  G   L   C   Q   T   F   V   Y   G   G   C   R   A   K   R
ggc ctg tgc cag acc ttt gta tac ggt ggt tgc cgt gct aag cgt

 61  62  63  64  65  66  67  68  69  70  71  72  73  74  75
  N   N   F   K   S   A   E   D   C   M   R   T   C   G   G
aac aac ttt aaa tcg gcc gaa gat tgc atg cgt acc tgc ggt ggc

 76  77  78  79  80  81  82  83  84  85  86  87  88  89  90
  A   G   A   A   E   T   V   E   S   C   L   A   K   P   H
gcc GGC GCC GCT GAA ACT GTT GAA AGT TGT TTA GCA AAA CCC CAT
    |<------- mature gene III protein --------------------

 91  92  93  94  95  96  97  98  99 100 101 102 103 104 105
  T   E   N   S   F   T   N   V   W   K   D   D   K   T   L
ACA GAA AAT TCA TTT ACT AAC GTC TGG AAA GAC GAC AAA ACT TTA
```

Table 116: translation of Signal-III::bpti::mature-III (continued)

| | | | | | | | | | | | | | | |
|---|---|---|---|---|---|---|---|---|---|---|---|---|---|---|
| 106 | 107 | 108 | 109 | 110 | 111 | 112 | 113 | 114 | 115 | 116 | 117 | 118 | 119 | 120 |
| D | R | Y | A | N | Y | E | G | C | L | W | N | A | T | G |
| GAT | CGT | TAC | GCT | AAC | TAT | GAG | GGT | TGT | CTG | TGG | AAT | GCT | ACA | GGC |
| 121 | 122 | 123 | 124 | 125 | 126 | 127 | 128 | 129 | 130 | 131 | 132 | 133 | 134 | 135 |
| V | V | V | C | T | G | D | E | T | Q | C | Y | G | T | W |
| GTT | GTA | GTT | TGT | ACT | GGT | GAC | GAA | ACT | CAG | TGT | TAC | GGT | ACA | TGG |
| 136 | 137 | 138 | 139 | 140 | 141 | 142 | 143 | 144 | 145 | 146 | 147 | 148 | 149 | 150 |
| V | P | I | G | L | A | I | P | E | N | E | G | G | G | S |
| GTT | CCT | ATT | GGG | CTT | GCT | ATC | CCT | GAA | AAT | GAG | GGT | GGT | GGC | TCT |
| 151 | 152 | 153 | 154 | 155 | 156 | 157 | 158 | 159 | 160 | 161 | 162 | 163 | 164 | 165 |
| E | G | G | G | S | E | G | G | G | S | E | G | G | G | T |
| GAG | GGT | GGC | GGT | TCT | GAG | GGT | GGC | GGT | TCT | GAG | GGT | GGC | GGT | ACT |
| 166 | 167 | 168 | 169 | 170 | 171 | 172 | 173 | 174 | 175 | 176 | 177 | 178 | 179 | 180 |
| K | P | P | E | Y | G | D | T | P | I | P | G | Y | T | Y |
| AAA | CCT | CCT | GAG | TAC | GGT | GAT | ACA | CCT | ATT | CCG | GGC | TAT | ACT | TAT |
| 181 | 182 | 183 | 184 | 185 | 186 | 187 | 188 | 189 | 190 | 191 | 192 | 193 | 194 | 195 |
| I | N | P | L | D | G | T | Y | P | P | G | T | E | Q | N |
| ATC | AAC | CCT | CTC | GAC | GGC | ACT | TAT | CCG | CCT | GGT | ACT | GAG | CAA | AAC |
| 196 | 197 | 198 | 199 | 200 | 201 | 202 | 203 | 204 | 205 | 206 | 207 | 208 | 209 | 210 |
| P | A | N | P | N | P | S | L | E | E | S | Q | P | L | N |
| CCC | GCT | AAT | CCT | AAT | CCT | TCT | CTT | GAG | GAG | TCT | CAG | CCT | CTT | AAT |
| 211 | 212 | 213 | 214 | 215 | 216 | 217 | 218 | 219 | 220 | 221 | 222 | 223 | 224 | 225 |
| T | F | M | F | Q | N | N | R | F | R | N | R | Q | G | A |
| ACT | TTC | ATG | TTT | CAG | AAT | AAT | AGG | TTC | CGA | AAT | AGG | CAG | GGG | GCA |

Table 116: translation of Signal-III::bpti::mature-III (continued)

| | | | | | | | | | | | | | | |
|---|---|---|---|---|---|---|---|---|---|---|---|---|---|---|
| 226 | 227 | 228 | 229 | 230 | 231 | 232 | 233 | 234 | 235 | 236 | 237 | 238 | 239 | 240 |
| L | T | V | Y | T | G | T | V | T | Q | G | T | D | P | V |
| TTA | ACT | GTT | TAT | ACG | GGC | ACT | GTT | ACT | CAA | GGC | ACT | GAC | CCC | GTT |
| 241 | 242 | 243 | 244 | 245 | 246 | 247 | 248 | 249 | 250 | 251 | 252 | 253 | 254 | 255 |
| K | T | Y | Y | Q | Y | T | P | V | S | S | K | A | M | Y |
| AAA | ACT | TAT | TAC | CAG | TAC | ACT | CCT | GTA | TCA | TCA | AAA | GCC | ATG | TAT |
| 256 | 257 | 258 | 259 | 260 | 261 | 262 | 263 | 264 | 265 | 266 | 267 | 268 | 269 | 270 |
| D | A | Y | W | N | G | K | F | R | D | C | A | F | H | S |
| GAC | GCT | TAC | TGG | AAC | GGT | AAA | TTC | AGA | GAC | TGC | GCT | TTC | CAT | TCT |
| 271 | 272 | 273 | 274 | 275 | 276 | 277 | 278 | 279 | 280 | 281 | 282 | 283 | 284 | 285 |
| G | F | N | E | D | P | F | V | C | E | Y | Q | G | Q | S |
| GGC | TTT | AAT | GAG | GAT | CCA | TTC | GTT | TGT | GAA | TAT | CAA | GGC | CAA | TCG |
| 286 | 287 | 288 | 289 | 290 | 291 | 292 | 293 | 294 | 295 | 296 | 297 | 298 | 299 | 300 |
| S | D | L | P | Q | P | P | V | N | A | G | G | G | S | G |
| TCT | GAC | CTG | CCT | CAA | CCT | CCT | GTC | AAT | GCT | GGC | GGC | GGC | TCT | GGT |
| 301 | 302 | 303 | 304 | 305 | 306 | 307 | 308 | 309 | 310 | 311 | 312 | 313 | 314 | 315 |
| G | G | S | G | G | G | S | E | G | G | G | S | E | G | G |
| GGT | GGT | TCT | GGT | GGC | GGC | TCT | GAG | GGT | GGT | GGC | TCT | GAG | GGT | GGC |
| 316 | 317 | 318 | 319 | 320 | 321 | 322 | 323 | 324 | 325 | 326 | 327 | 328 | 329 | 330 |
| G | S | E | G | G | G | S | E | G | G | G | S | G | G | G |
| GGT | TCT | GAG | GGT | GGC | GGC | TCT | GAG | GGA | GGC | GGT | TCC | GGT | GGT | GGC |
| 331 | 332 | 333 | 334 | 335 | 336 | 337 | 338 | 339 | 340 | 341 | 342 | 343 | 344 | 345 |
| S | G | S | G | D | F | D | Y | E | K | M | A | N | A | N |
| TCT | GGT | TCC | GGT | GAT | TTT | GAT | TAT | GAA | AAG | ATG | GCA | AAC | GCT | AAT |

Table 116: translation of Signal-III::bpti::mature-III (continued)

| | | | | | | | | | | | | | | |
|---|---|---|---|---|---|---|---|---|---|---|---|---|---|---|
| 346 | 347 | 348 | 349 | 350 | 351 | 352 | 353 | 354 | 355 | 356 | 357 | 358 | 359 | 360 |
| K | G | A | M | T | E | N | A | D | E | N | A | L | Q | S |
| AAG | GGG | GCT | ATG | ACC | GAA | AAT | GCC | GAT | GAA | AAC | GCG | CTA | CAG | TCT |
| 361 | 362 | 363 | 364 | 365 | 366 | 367 | 368 | 369 | 370 | 371 | 372 | 373 | 374 | 375 |
| D | A | K | G | K | L | D | S | V | A | T | D | Y | G | A |
| GAC | GCT | AAA | GGC | AAA | CTT | GAT | TCT | GTC | GCT | ACT | GAT | TAC | GGT | GCT |
| 376 | 377 | 378 | 379 | 380 | 381 | 382 | 383 | 384 | 385 | 386 | 387 | 388 | 389 | 390 |
| A | I | D | G | F | I | G | D | V | S | G | L | A | N | G |
| GCT | ATC | GAT | GGT | TTC | ATT | GGT | GAC | GTT | TCC | GGC | CTT | GCT | AAT | GGT |
| 391 | 392 | 393 | 394 | 395 | 396 | 397 | 398 | 399 | 400 | 401 | 402 | 403 | 404 | 405 |
| N | G | A | T | G | D | F | A | G | S | N | S | Q | M | A |
| AAT | GGT | GCT | ACT | GGT | GAT | TTT | GCT | GGC | TCT | AAT | TCC | CAA | ATG | GCT |
| 406 | 407 | 408 | 409 | 410 | 411 | 412 | 413 | 414 | 415 | 416 | 417 | 418 | 419 | 420 |
| Q | V | G | D | G | D | N | S | P | L | M | N | N | F | R |
| CAA | GTC | GGT | GAC | GGT | GAT | AAT | TCA | CCT | TTA | ATG | AAT | AAT | TTC | CGT |
| 421 | 422 | 423 | 424 | 425 | 426 | 427 | 428 | 429 | 430 | 431 | 432 | 433 | 434 | 435 |
| Q | Y | L | P | S | L | P | Q | S | V | E | C | R | P | F |
| CAA | TAT | TTA | CCT | TCC | CTC | CCT | CAA | TCG | GTT | GAA | TGT | CGC | CCT | TTT |
| 436 | 437 | 438 | 439 | 440 | 441 | 442 | 443 | 444 | 445 | 446 | 447 | 448 | 449 | 450 |
| V | F | S | A | G | K | P | Y | E | F | S | I | D | C | D |
| GTC | TTT | AGC | GCT | GGT | AAA | CCA | TAT | GAA | TTT | TCT | ATT | GAT | TGT | GAC |

Table 116: translation of Signal-III::bpti::mature-III (continued)

```
451 452 453 454 455 456 457 458 459 460 461 462 463 464 465
 K   I   N   L   F   R   G   V   F   A   F   L   L   Y   V
AAA ATA AAC TTA TTC CGT GGT GTC TTT GCG TTT CTT TTA TAT GTT
                        |<----- uncharged anchor region -----

466 467 468 469 470 471 472 473 474 475 476 477 478 479 480
 A   T   F   M   Y   V   F   S   T   F   A   N   I   L   R
GCC ACC TTT ATG TAT GTA TTT TCT ACG TTT GCT AAC ATA CTG CGT
--------- uncharged anchor region continues --------->|

481 482 483 484 485
 N   K   E   S   .
AAT AAG GAG TCT TAA
```

| | | |
|---|---|---|
| Molecular weight of peptide | = | 58884 |
| Charge on peptide | = | -20 |
| [A+G+P] | = | 143 |
| [C+F+H+I+L+M+V+W+Y] | = | 140 |
| [D+E+K+R+N+Q+S+T+.] | = | 202 |

Table 116: translation of Signal-III::bpti::mature-III (continued)

| | Second Base t | c | a | g | |
|---|---|---|---|---|---|
| t | 15 | 21 | 15 | 8 | t |
| | 12 | 5 | 10 | 6 | c |
| | 10 | 4 | 0 | 0 | a |
| | 0 | 3 | 0 | 4 | g |
| c | 6 | 20 | 2 | 8 | t |
| | 3 | 4 | 0 | 3 | c |
| | 1 | 4 | 9 | 1 | a |
| | 4 | 3 | 7 | 0 | g |
| a | 5 | 19 | 21 | 1 | t |
| | 5 | 4 | 11 | 1 | c |
| | 2 | 4 | 16 | 1 | a |
| | 8 | 2 | 4 | 2 | g |
| g | 13 | 22 | 14 | 41 | t |
| | 6 | 7 | 12 | 29 | c |
| | 4 | 5 | 12 | 1 | a |
| | 1 | 3 | 16 | 4 | g |

| AA | # | AA | # | AA | # | AA | # |
|---|---|---|---|---|---|---|---|
| A | 37 | C | 14 | D | 26 | E | 28 |
| F | 27 | G | 75 | H | 2 | I | 12 |
| K | 20 | L | 24 | M | 9 | N | 32 |
| P | 31 | Q | 16 | R | 15 | S | 35 |
| T | 29 | V | 23 | W | 4 | Y | 25 |
| . | 1 | | | | | | |

Table 130: Sampling of a Library encoded by $(NNK)^6$

**A. Numbers of hexapeptides in each class**

total = 64,000,000 stop-free sequences.

α can be one of [WMFYCIKDENHQ]
Φ can be one of [PTAVG]
Ω can be one of [SLR]

| | | | | | |
|---|---|---|---|---|---|
| αααααα | = | 2985984. | Φααααα | = | 7464960. |
| Ωααααα | = | 4478976. | ΦΦαααα | = | 7776000. |
| ΦΩαααα | = | 9331200. | ΩΩαααα | = | 2799360. |
| ΦΦΦααα | = | 4320000. | ΦΦΩααα | = | 7776000. |
| ΦΩΩααα | = | 4665600. | ΩΩΩααα | = | 933120. |
| ΦΦΦΦαα | = | 1350000. | ΦΦΦΩαα | = | 3240000. |
| ΦΦΩΩαα | = | 2916000. | ΦΩΩΩαα | = | 1166400. |
| ΩΩΩΩαα | = | 174960. | ΦΦΦΦΦα | = | 225000. |
| ΦΦΦΦΩα | = | 675000. | ΦΦΦΩΩα | = | 810000. |
| ΦΦΩΩΩα | = | 486000. | ΦΩΩΩΩα | = | 145800. |
| ΩΩΩΩΩα | = | 17496. | ΦΦΦΦΦΦ | = | 15625. |
| ΦΦΦΦΦΩ | = | 56250. | ΦΦΦΦΩΩ | = | 84375. |
| ΦΦΦΩΩΩ | = | 67500. | ΦΦΩΩΩΩ | = | 30375. |
| ΦΩΩΩΩΩ | = | 7290. | ΩΩΩΩΩΩ | = | 729. |

ΦΦΩΩαα, for example, stands for the set of peptides having two amino acids from the α class, two from Φ, and two from Ω arranged in any order. There are, for example, $729 = 3^6$ sequences composed entirely of S, L, and R.

## Table 130: Sampling of a Library encoded by $(NNK)^6$ (continued)

B. Probability that any given stop-free DNA sequence will encode a hexapeptide from a stated class.

| | P | % of class |
|---|---|---|
| αααααα... | 3.364E-03 | (1.13E-07) |
| Φααααα... | 1.682E-02 | (2.25E-07) |
| Ωααααα... | 1.514E-02 | (3.38E-07) |
| ΦΦαααα... | 3.505E-02 | (4.51E-07) |
| ΦΩαααα... | 6.308E-02 | (6.76E-07) |
| ΩΩαααα... | 2.839E-02 | (1.01E-06) |
| ΦΦΦααα... | 3.894E-02 | (9.01E-07) |
| ΦΦΩααα... | 1.051E-01 | (1.35E-06) |
| ΦΩΩααα... | 9.463E-02 | (2.03E-06) |
| ΩΩΩααα... | 2.839E-02 | (3.04E-06) |
| ΦΦΦΦαα... | 2.434E-02 | (1.80E-06) |
| ΦΦΦΩαα... | 8.762E-02 | (2.70E-06) |
| ΦΦΩΩαα... | 1.183E-01 | (4.06E-06) |
| ΦΩΩΩαα... | 7.097E-02 | (6.08E-06) |
| ΩΩΩΩαα... | 1.597E-02 | (9.13E-06) |
| ΦΦΦΦΦα... | 8.113E-03 | (3.61E-06) |
| ΦΦΦΦΩα... | 3.651E-02 | (5.41E-06) |
| ΦΦΦΩΩα... | 6.571E-02 | (8.11E-06) |
| ΦΦΩΩΩα... | 5.914E-02 | (1.22E-05) |
| ΦΩΩΩΩα... | 2.661E-02 | (1.83E-05) |
| ΩΩΩΩΩα... | 4.790E-03 | (2.74E-05) |
| ΦΦΦΦΦΦ... | 1.127E-03 | (7.21E-06) |
| ΦΦΦΦΦΩ... | 6.084E-03 | (1.08E-05) |
| ΦΦΦΦΩΩ... | 1.369E-02 | (1.62E-05) |
| ΦΦΦΩΩΩ... | 1.643E-02 | (2.43E-05) |
| ΦΦΩΩΩΩ... | 1.109E-02 | (3.65E-05) |
| ΦΩΩΩΩΩ... | 3.992E-03 | (5.48E-05) |
| ΩΩΩΩΩΩ... | 5.988E-04 | (8.21E-05) |

## Table 130: Sampling of a Library encoded by $(NNK)^6$ (continued)

C. Number of different stop-free amino-acid sequences in each class expected for various library sizes

Library size = 1.0000E+06

total = 9.7446E+05 % sampled = 1.52

| Class | Number | % | Class | Number | % |
|---|---|---|---|---|---|
| αααααα... | 3362.6 | .1 | Φααααα... | 16803.4 | .2 |
| Ωααααα... | 15114.6 | .3 | ΦΦαααα... | 34967.8 | .4 |
| ΦΩαααα... | 62871.1 | .7 | ΩΩαααα... | 28244.3 | 1.0 |
| ΦΦΦααα... | 38765.7 | .9 | ΦΦΩααα... | 104432.2 | 1.3 |
| ΦΩΩααα... | 93672.7 | 2.0 | ΩΩΩααα... | 27960.3 | 3.0 |
| ΦΦΦΦαα... | 24119.9 | 1.8 | ΦΦΦΩαα... | 86442.5 | 2.7 |
| ΦΦΩΩαα... | 115915.5 | 4.0 | ΦΩΩΩαα... | 68853.5 | 5.9 |
| ΩΩΩΩαα... | 15261.1 | 8.7 | ΦΦΦΦΦα... | 7968.1 | 3.5 |
| ΦΦΦΦΩα... | 35537.2 | 5.3 | ΦΦΦΩΩα... | 63117.5 | 7.8 |
| ΦΦΩΩΩα... | 55684.4 | 11.5 | ΦΩΩΩΩα... | 24325.9 | 16.7 |
| ΩΩΩΩΩα... | 4190.6 | 24.0 | ΦΦΦΦΦΦ... | 1087.1 | 7.0 |
| ΦΦΦΦΦΩ... | 5767.0 | 10.3 | ΦΦΦΦΩΩ... | 12637.2 | 15.0 |
| ΦΦΦΩΩΩ... | 14581.7 | 21.6 | ΦΦΩΩΩΩ... | 9290.2 | 30.6 |
| ΦΩΩΩΩΩ... | 3073.9 | 42.2 | ΩΩΩΩΩΩ... | 408.4 | 56.0 |

Library size = 3.0000E+06

total = 2.7885E+06 % sampled = 4.36

| | | | | | |
|---|---|---|---|---|---|
| αααααα... | 10076.4 | .3 | Φααααα... | 50296.9 | .7 |
| Ωααααα... | 45190.9 | 1.0 | ΦΦαααα... | 104432.2 | 1.3 |
| ΦΩαααα... | 187345.5 | 2.0 | ΩΩαααα... | 83880.9 | 3.0 |
| ΦΦΦααα... | 115256.6 | 2.7 | ΦΦΩααα... | 309107.9 | 4.0 |
| ΦΩΩααα... | 275413.9 | 5.9 | ΩΩΩααα... | 81392.5 | 8.7 |
| ΦΦΦΦαα... | 71074.5 | 5.3 | ΦΦΦΩαα... | 252470.2 | 7.8 |
| ΦΦΩΩαα... | 334106.2 | 11.5 | ΦΩΩΩαα... | 194606.9 | 16.7 |
| ΩΩΩΩαα... | 41905.9 | 24.0 | ΦΦΦΦΦα... | 23067.8 | 10.3 |
| ΦΦΦΦΩα... | 101097.3 | 15.0 | ΦΦΦΩΩα... | 174981.0 | 21.6 |
| ΦΦΩΩΩα... | 148643.7 | 30.6 | ΦΩΩΩΩα... | 61478.9 | 42.2 |
| ΩΩΩΩΩα... | 9801.0 | 56.0 | ΦΦΦΦΦΦ... | 3039.6 | 19.5 |
| ΦΦΦΦΦΩ... | 15587.7 | 27.7 | ΦΦΦΦΩΩ... | 32516.8 | 38.5 |
| ΦΦΦΩΩΩ... | 34975.6 | 51.8 | ΦΦΩΩΩΩ... | 20215.5 | 66.6 |
| ΦΩΩΩΩΩ... | 5879.9 | 80.7 | ΩΩΩΩΩΩ... | 667.0 | 91.5 |

## Table 130: Sampling of a Library encoded by (NNK)[6] (continued)

Library size = 1.0000E+07

total = 8.1204E+06 % sampled = 12.69

| | | | |
|---|---|---|---|
| αααααα... | 33455.9( 1.1) | Φααααα... | 166342.4( 2.2) |
| Ωααααα... | 148871.1( 3.3) | ΦΦαααα... | 342685.7( 4.4) |
| ΦΩαααα... | 609987.6( 6.5) | ΩΩαααα... | 269958.3( 9.6) |
| ΦΦΦααα... | 372371.8( 8.6) | ΦΦΩααα... | 983416.4( 12.6) |
| ΦΩΩααα... | 856471.6( 18.4) | ΩΩΩααα... | 244761.5( 26.2) |
| ΦΦΦΦαα... | 222702.0( 16.5) | ΦΦΦΩαα... | 767692.5( 23.7) |
| ΦΦΩΩαα... | 972324.6( 33.3) | ΦΩΩΩαα... | 531651.3( 45.6) |
| ΩΩΩΩαα... | 104722.3( 59.9) | ΦΦΦΦΦα... | 68111.0( 30.3) |
| ΦΦΦΦΩα... | 281976.3( 41.8) | ΦΦΦΩΩα... | 450120.2( 55.6) |
| ΦΦΩΩΩα... | 342072.1( 70.4) | ΦΩΩΩΩα... | 122302.6( 83.9) |
| ΩΩΩΩΩα... | 16364.0( 93.5) | ΦΦΦΦΦΦ... | 8028.0( 51.4) |
| ΦΦΦΦΦΩ... | 37179.9( 66.1) | ΦΦΦΦΩΩ... | 67719.5( 80.3) |
| ΦΦΦΩΩΩ... | 61580.0( 91.2) | ΦΦΩΩΩΩ... | 29586.1( 97.4) |
| ΦΩΩΩΩΩ... | 7259.5( 99.6) | ΩΩΩΩΩΩ... | 728.8(100.0) |

Library size = 3.0000E+07

total = 1.8633E+07 % sampled = 29.11

| | | | |
|---|---|---|---|
| αααααα... | 99247.4( 3.3) | Φααααα... | 487990.0( 6.5) |
| Ωααααα... | 431933.3( 9.6) | ΦΦαααα... | 983416.5( 12.6) |
| ΦΩαααα... | 1712943.0( 18.4) | ΩΩαααα... | 734284.6( 26.2) |
| ΦΦΦααα... | 1023590.0( 23.7) | ΦΦΩααα... | 2592866.0( 33.3) |
| ΦΩΩααα... | 2126605.0( 45.6) | ΩΩΩααα... | 558519.0( 59.9) |
| ΦΦΦΦαα... | 563952.6( 41.8) | ΦΦΦΩαα... | 1800481.0( 55.6) |
| ΦΦΩΩαα... | 2052433.0( 70.4) | ΦΩΩΩαα... | 978420.5( 83.9) |
| ΩΩΩΩαα... | 163640.3( 93.5) | ΦΦΦΦΦα... | 148719.7( 66.1) |
| ΦΦΦΦΩα... | 541755.7( 80.3) | ΦΦΦΩΩα... | 738960.1( 91.2) |
| ΦΦΩΩΩα... | 473377.0( 97.4) | ΦΩΩΩΩα... | 145189.7( 99.6) |
| ΩΩΩΩΩα... | 17491.3(100.0) | ΦΦΦΦΦΦ... | 13829.1( 88.5) |
| ΦΦΦΦΦΩ... | 54058.1( 96.1) | ΦΦΦΦΩΩ... | 83726.0( 99.2) |
| ΦΦΦΩΩΩ... | 67454.5( 99.9) | ΦΦΩΩΩΩ... | 30374.5(100.0) |
| ΦΩΩΩΩΩ... | 7290.0(100.0) | ΩΩΩΩΩΩ... | 729.0(100.0) |

Table 130: Sampling of a Library encoded by $(NNK)^6$ (continued)

Library size = 7.6000E+07

total = 3.2125E+07 % sampled = 50.19

| | | | |
|---|---|---|---|
| αααααα... | 245057.8( 8.2) | Φααααα... | 1175010.0( 15.7) |
| Ωααααα... | 1014733.0( 22.7) | ΦΦαααα... | 2255280.0( 29.0) |
| ΦΩαααα... | 3749112.0( 40.2) | ΩΩαααα... | 1504128.0( 53.7) |
| ΦΦΦααα... | 2142478.0( 49.6) | ΦΦΩααα... | 4993247.0( 64.2) |
| ΦΩΩααα... | 3666785.0( 78.6) | ΩΩΩααα... | 840691.9( 90.1) |
| ΦΦΦΦαα... | 1007002.0( 74.6) | ΦΦΦΩαα... | 2825063.0( 87.2) |
| ΦΦΩΩαα... | 2782358.0( 95.4) | ΦΩΩΩαα... | 1154956.0( 99.0) |
| ΩΩΩΩαα... | 174790.0( 99.9) | ΦΦΦΦΦα... | 210475.6( 93.5) |
| ΦΦΦΦΩα... | 663929.3( 98.4) | ΦΦΦΩΩα... | 808298.6( 99.8) |
| ΦΦΩΩΩα... | 485953.2(100.0) | ΦΩΩΩΩα... | 145799.9(100.0) |
| ΩΩΩΩΩα... | 17496.0(100.0) | ΦΦΦΦΦΦ... | 15559.9( 99.6) |
| ΦΦΦΦΦΩ... | 56234.9(100.0) | ΦΦΦΦΩΩ... | 84374.6(100.0) |
| ΦΦΦΩΩΩ... | 67500.0(100.0) | ΦΦΩΩΩΩ... | 30375.0(100.0) |
| ΦΩΩΩΩΩ... | 7290.0(100.0) | ΩΩΩΩΩΩ... | 729.0(100.0) |

Library size = 1.0000E+08

total = 3.6537E+07 % sampled = 57.09

| | | | |
|---|---|---|---|
| αααααα... | 318185.1( 10.7) | Φααααα... | 1506161.0( 20.2) |
| Ωααααα... | 1284677.0( 28.7) | ΦΦαααα... | 2821285.0( 36.3) |
| ΦΩαααα... | 4585163.0( 49.1) | ΩΩαααα... | 1783932.0( 63.7) |
| ΦΦΦααα... | 2566085.0( 59.4) | ΦΦΩααα... | 5764391.0( 74.1) |
| ΦΩΩααα... | 4051713.0( 86.8) | ΩΩΩααα... | 888584.3( 95.2) |
| ΦΦΦΦαα... | 1127473.0( 83.5) | ΦΦΦΩαα... | 3023170.0( 93.3) |
| ΦΦΩΩαα... | 2865517.0( 98.3) | ΦΩΩΩαα... | 1163743.0( 99.8) |
| ΩΩΩΩαα... | 174941.0(100.0) | ΦΦΦΦΦα... | 218886.6( 97.3) |
| ΦΦΦΦΩα... | 671976.9( 99.6) | ΦΦΦΩΩα... | 809757.3(100.0) |
| ΦΦΩΩΩα... | 485997.5(100.0) | ΦΩΩΩΩα... | 145800.0(100.0) |
| ΩΩΩΩΩα... | 17496.0(100.0) | ΦΦΦΦΦΦ... | 15613.5( 99.9) |
| ΦΦΦΦΦΩ... | 56248.9(100.0) | ΦΦΦΦΩΩ... | 84375.0(100.0) |
| ΦΦΦΩΩΩ... | 67500.0(100.0) | ΦΦΩΩΩΩ... | 30375.0(100.0) |
| ΦΩΩΩΩΩ... | 7290.0(100.0) | ΩΩΩΩΩΩ... | 729.0(100.0) |

Table 130: Sampling of a Library encoded by (NNK)[6] (continued)

Library size = 3.0000E+08

total = 5.2634E+07 % sampled = 82.24

| | | | |
|---|---|---|---|
| αααααα... | 856451.3( 28.7) | Φααααα... | 3668130.0( 49.1) |
| Ωααααα... | 2854291.0( 63.7) | ΦΦαααα... | 5764391.0( 74.1) |
| ΦΩαααα... | 8103426.0( 86.8) | ΩΩαααα... | 2665753.0( 95.2) |
| ΦΦΦααα... | 4030893.0( 93.3) | ΦΦΩααα... | 7641378.0( 98.3) |
| ΦΩΩααα... | 4654972.0( 99.8) | ΩΩΩααα... | 933018.6(100.0) |
| ΦΦΦΦαα... | 1343954.0( 99.6) | ΦΦΦΩαα... | 3239029.0(100.0) |
| ΦΦΩΩαα... | 2915985.0(100.0) | ΦΩΩΩαα... | 1166400.0(100.0) |
| ΩΩΩΩαα... | 174960.0(100.0) | ΦΦΦΦΦα... | 224995.5(100.0) |
| ΦΦΦΦΩα... | 674999.9(100.0) | ΦΦΦΩΩα... | 810000.0(100.0) |
| ΦΦΩΩΩα... | 486000.0(100.0) | ΦΩΩΩΩα... | 145800.0(100.0) |
| ΩΩΩΩΩα... | 17496.0(100.0) | ΦΦΦΦΦΦ... | 15625.0(100.0) |
| ΦΦΦΦΦΩ... | 56250.0(100.0) | ΦΦΦΦΩΩ... | 84375.0(100.0) |
| ΦΦΦΩΩΩ... | 67500.0(100.0) | ΦΦΩΩΩΩ... | 30375.0(100.0) |
| ΦΩΩΩΩΩ... | 7290.0(100.0) | ΩΩΩΩΩΩ... | 729.0(100.0) |

Library size = 1.0000E+09

total = 6.1999E+07 % sampled = 96.87

| | | | |
|---|---|---|---|
| αααααα... | 2018278.0( 67.6) | Φααααα... | 6680917.0( 89.5) |
| Ωααααα... | 4326519.0( 96.6) | ΦΦαααα... | 7690221.0( 98.9) |
| ΦΩαααα... | 9320389.0( 99.9) | ΩΩαααα... | 2799250.0(100.0) |
| ΦΦΦααα... | 4319475.0(100.0) | ΦΦΩααα... | 7775990.0(100.0) |
| ΦΩΩααα... | 4665600.0(100.0) | ΩΩΩααα... | 933120.0(100.0) |
| ΦΦΦΦαα... | 1350000.0(100.0) | ΦΦΦΩαα... | 3240000.0(100.0) |
| ΦΦΩΩαα... | 2916000.0(100.0) | ΦΩΩΩαα... | 1166400.0(100.0) |
| ΩΩΩΩαα... | 174960.0(100.0) | ΦΦΦΦΦα... | 225000.0(100.0) |
| ΦΦΦΦΩα... | 675000.0(100.0) | ΦΦΦΩΩα... | 810000.0(100.0) |
| ΦΦΩΩΩα... | 486000.0(100.0) | ΦΩΩΩΩα... | 145800.0(100.0) |
| ΩΩΩΩΩα... | 17496.0(100.0) | ΦΦΦΦΦΦ... | 15625.0(100.0) |
| ΦΦΦΦΦΩ... | 56250.0(100.0) | ΦΦΦΦΩΩ... | 84375.0(100.0) |
| ΦΦΦΩΩΩ... | 67500.0(100.0) | ΦΦΩΩΩΩ... | 30375.0(100.0) |
| ΦΩΩΩΩΩ... | 7290.0(100.0) | ΩΩΩΩΩΩ... | 729.0(100.0) |

**Table 130: Sampling of a Library encoded by (NNK)[6] (continued)**

Library size = 3.0000E+09

total = 6.3890E+07 % sampled = 99.83

| | | | |
|---|---|---|---|
| αααααα... | 2884346.0( 96.6) | Φααααα... | 7456311.0( 99.9) |
| Ωααααα... | 4478800.0(100.0) | ΦΦαααα... | 7775990.0(100.0) |
| ΦΩαααα... | 9331200.0(100.0) | ΩΩαααα... | 2799360.0(100.0) |
| ΦΦΦααα... | 4320000.0(100.0) | ΦΦΩααα... | 7776000.0(100.0) |
| ΦΩΩααα... | 4665600.0(100.0) | ΩΩΩααα... | 933120.0(100.0) |
| ΦΦΦΦαα... | 1350000.0(100.0) | ΦΦΦΩαα... | 3240000.0(100.0) |
| ΦΦΩΩαα... | 2916000.0(100.0) | ΦΩΩΩαα... | 1166400.0(100.0) |
| ΩΩΩΩαα... | 174960.0(100.0) | ΦΦΦΦΦα... | 225000.0(100.0) |
| ΦΦΦΦΩα... | 675000.0(100.0) | ΦΦΦΩΩα... | 810000.0(100.0) |
| ΦΦΩΩΩα... | 486000.0(100.0) | ΦΩΩΩΩα... | 145800.0(100.0) |
| ΩΩΩΩΩα... | 17496.0(100.0) | ΦΦΦΦΦΦ... | 15625.0(100.0) |
| ΦΦΦΦΦΩ... | 56250.0(100.0) | ΦΦΦΦΩΩ... | 84375.0(100.0) |
| ΦΦΦΩΩΩ... | 67500.0(100.0) | ΦΦΩΩΩΩ... | 30375.0(100.0) |
| ΦΩΩΩΩΩ... | 7290.0(100.0) | ΩΩΩΩΩΩ... | 729.0(100.0) |

## Table 130, continued

D. Formulae for tabulated quantities.

```
Lsize is the number of independent transformants.
31**6 is 31 to sixth power; 6*3 means 6 times 3.
A = Lsize/(31**6)
α can be one of [WMFYCIKDENHQ.]
Φ can be one of [PTAVG]
Ω can be one of [SLR]
F0 = (12)**6      F1 = (12)**5      F2 = (12)**4
F3 = (12)**3      F4 = (12)**2      F5 = (12)
F6 = 1

αααααα = F0 * (1-exp(-A))
Φααααα = 6 * 5 * F1 * (1-exp(-2*A))
Ωααααα = 6 * 3 * F1 * (1-exp(-3*A))
ΦΦαααα = (15) * 5**2 * F2 * (1-exp(-4*A))
ΦΩαααα = (6*5)*5*3 *F2  * (1-exp(-6*A))
ΩΩαααα = (15) * 3**2 * F2 * (1-exp(-9*A))
ΦΦΦααα = (20)*(5**3) * F3 * (1-exp(-8*A))
ΦΦΩααα = (60)*(5*5*3)*F3* (1-exp(-12*A))
ΦΩΩααα = (60)*(5*3*3)*F3*(1-exp(-18*A))
ΩΩΩααα = (20)*(3)**3*F3*(1-exp(-27*A))
ΦΦΦΦαα = (15)*(5)**4*F4*(1-exp(-16*A))
ΦΦΦΩαα = (60)*(5)**3*3*F4*(1-exp(-24*A))
ΦΦΩΩαα = (90)*(5*5*3*3)*F4*(1-exp(-36*A))
ΦΩΩΩαα = (60)*(5*3*3*3)*F4*(1-exp(-54*A))
ΩΩΩΩαα = (15)*(3)**4 * F4 *(1-exp(-81*A))
ΦΦΦΦΦα = (6)*(5)**5 * F5 * (1-exp(-32*A))
ΦΦΦΦΩα = 30*5*5*5*5*3*F5*(1-exp(-48*A))
ΦΦΦΩΩα = 60*5*5*5*3*3*F5*(1-exp(-72*A))
ΦΦΩΩΩα = 60*5*5*3*3*3*F5*(1-exp(-108*A))
ΦΩΩΩΩα = 30*5*3*3*3*3*F5*(1-exp(-162*A))
ΩΩΩΩΩα = 6*3*3*3*3*3*F5*(1-exp(-243*A))
ΦΦΦΦΦΦ = 5**6 * (1-exp(-64*A))
ΦΦΦΦΦΩ = 6*3*5**5*(1-exp(-96*A))
ΦΦΦΦΩΩ = 15*3*3*5**4*(1-exp(-144*A))
ΦΦΦΩΩΩ = 20*3**3*5**3*(1-exp(-216*A))
ΦΦΩΩΩΩ = 15*3**4*5**2*(1-exp(-324*A))
ΦΩΩΩΩΩ = 6*3**5*5*(1-exp(-486*A))
ΩΩΩΩΩΩ = 3**6*(1-exp(-729*A))
total  = αααααα + Φααααα + Ωααααα + ΦΦαααα + ΦΩαααα +
         ΩΩαααα + ΦΦΦααα + ΦΦΩααα + ΦΩΩααα + ΩΩΩααα +
         ΦΦΦΦαα + ΦΦΦΩαα + ΦΦΩΩαα + ΦΩΩΩαα + ΩΩΩΩαα +
         ΦΦΦΦΦα + ΦΦΦΦΩα + ΦΦΦΩΩα + ΦΦΩΩΩα + ΦΩΩΩΩα +
         ΩΩΩΩΩα + ΦΦΦΦΦΦ + ΦΦΦΦΦΩ + ΦΦΦΦΩΩ + ΦΦΦΩΩΩ +
         ΦΦΩΩΩΩ + ΦΩΩΩΩΩ + ΩΩΩΩΩΩ
```

Table 131: Sampling of a Library Encoded by (NNT) 4 (NNG) 2

X can be F, S, Y, C, L, P, H, R, I, T, N, V, A, D, G

Γ can be $L^2$ $R^2$, S,W, P, Q,N, T,K,V,A, E, G

Library comprises $8.55 \cdot 10^6$ amino-acid sequences; $1.47 \cdot 10^7$ DNA sequences.

Total number of possible aa sequences= 8,555,625

| | |
|---|---|
| X | LVPTRRGFYCHIND |
| S | S |
| θ | VPTAGWQMKES |
| Ω | LR |

The first, second, fifth, and sixth positions can hold x or S; the third and fourth position can hold θ or Ω. I have lumped sequences by the number of xs, Ss, θs, and Ωs.

For example xxθΩSS stands for:

[xxθΩss, xSθΩxS, xSθΩSx, SSθΩxx, SxθΩxs, SxθΩSx,
xxΩθSS, xSΩθxS, xSΩθSx, SSΩθxS, SxΩθSx]

The following table shows the likelihoood that any particular DNA sequence will fall into one of the defined classes.

| | | | |
|---|---|---|---|
| Library size = | 1.0 | Sampling | = .00001% |
| total..... | 1.0000E+00 | %-sampled..... | 1.1688E-07 |
| xxθθxx..... | 3.1524E-01 | xxθΩxx..... | 2.2926E-01 |
| xxΩΩxx..... | 4.1684E-02 | xxθθxS | 1.8013E-01 |
| xxθΩxS..... | 1.3101E-01 | xxΩΩxS...... | 2.3819E-02 |
| xxθθSS..... | 3.8600E-02 | xxθΩSS...... | 2.8073E-02 |
| xxΩΩSS..... | 5.1042E-03 | xSθθSS..... | 3.6762E-03 |
| xSθΩSS..... | 2. 6736E-03 | xSΩΩSS..... | 4.8611E-04 |
| SSθθSS..... | 1.3129E-04 | SSθΩSS..... | 9.5486E-05 |
| SSΩΩSS..... | 1.7361E-05 | | |

The following sections show how many sequences of each class are expected for libraries of different sizes.

| | | | |
|---|---|---|---|
| Library size = | 1.0000E+05 | | |
| total...... | 9.9137E+04 | fraction sampled = | 1.1587E-02 |
| Type | Number % | Type | Number % |
| xxθθxx..... | 31416.9 ( .7) | xxθΩxx...... | 22771.4( 1.3) |
| xxΩΩxx..... | 4112.4( 2.7) | xxθθxS ..... | 17891.8( 1.3) |
| xxθθxS..... | 12924.6( 2.7) | xxΩΩxS..... | 2318.5( 5.3) |
| xxθθSS...... | 3808.1 2.7) | xxθΩSS..... | 2732.5( 5.3) |
| xxΩΩSS....... | 483.7.( 10.3) | xSθθSS..... | 357.8( 5.3) |
| xSθΩSS..... | 253.4( 10.3) | xSΩΩSS..... | 43.7( 19.5) |
| SSθθSS ..... | 12.4( 10.3) | SSθΩSS...... | 8.6( 19.5) |
| SSΩΩSS..... | 1.4( 35.2) | | |
| Library size = | 1.0000E+06 | | |
| total...... | 9.2064E+05 | fraction sampled = | 1.0761E-01 |
| xxθθxx..... | 304783.9(6.6) | xxθΩxx..... | 214394.0( 12.7) |
| xxΩΩxx..... | 36508.6( 23.8) | xxθθxS...... | 168452.5( 12.7) |
| xxθΩxS..... | 114741.4( 2348) | xxΩΩxS..... | 18383.8( 41.9) |

(continued)

| Type | Number % | Type | Number % |
|---|---|---|---|
| xxθθSS..... | 33807.7( 23.8) | xxθΩSS..... | 21666.6( 41.9) |
| XXΩΩSS....... | 3114.6( 66.2) | xSθθSS..... | 2837.3( 41.9) |
| xSθΩSS..... | 1631.5( 66.2) | xSΩΩSS..... | 198.4( 88.6) |
| SSθθSS...... | 80.1( 66.2) | SSθΩSS..... | 39.0( 88.6) |
| SSΩΩSS..... | 3.9( 98.7) | | |
| Library size = | 3.0000E+06 | | |
| total..... | 2.38809+06 | fraction sampled = | 2.7912E-01 |
| xxθθxx..... | 855709.5( 18.4) | xxθΩxx..... | 565051.6( 33.4) |
| xxΩΩxx..... | 85564.7( 55.7) | xxθθxS..... | 443969.1( 33.4) |
| xxθΩxS..... | 268917.8 55.7) | xxΩΩxS..... | 35281.3( 80.4) |
| xxxθθSS..... | 79234.7( 55.7) | xxθΩSS..... | 41581.5( 80.4) |
| xxΩΩSS..... | 4522.6( 96.1) | xSθθSS..... | 5445.2( 80.4) |
| xSθΩSS..... | 2369.0( 96.1) | xSΩΩSS..... | 223.7( 99.9) |
| SSθθSS..... | 116.3( 96.1) | SSθΩSS..... | 43.9( 99.9) |
| SSΩΩSS..... | 4.0(100.0) | | |
| Library size = | 8.5556E+06 | | |
| total..... | 4.9303E+06 | fraction sampled = | 5.7626E-01 |
| xxθθxx..... | 2046301.0( 44.0) | XX0θΩX..... | 1160645.0 ( 68.7) |
| xxΩΩxx..... | 138575.9( 90.2) | xxθθxS..... | 911935.6 ( 68.7) |
| xxθΩxS..... | 435524.3 ( 90.2) | xxΩΩxS..... | 43480.7( 99.0) |
| xxθθSS..... | 128324.1 ( 90.2) | xxθΩSS..... | 51245.1 ( 99.0) |
| xxΩΩSS..... | 4703.6(100.0) | xSθθSS..... | 6710.7 ( 99.0) |
| xSθΩSS..... | 2463.8 (100.0) | xsΩΩSS..... | 224.0(100.0) |
| SSθθSS..... | 121.0 (100.0) | SSθΩSS..... | 44.0 (100.0) |
| SSΩΩSS..... | 4.0(100.0) | | |
| Library size = | 1.0000E+07 | | |
| total..... | 5.3667E+06 | fraction sampled = | 6.2727E-01 |
| xxθθxx..... | 2289093.0 ( 49.2) | xxθΩxx..... | 1254877.0 ( 74.2) |
| xxΩΩxx..... | 143467.0( 93.4) | xxθθxS..... | 985974.9 ( 74.2) |
| xxθΩxS..... | 450896.3 ( 93.4) | xxΩΩxS..... | 43710.7 ( 99.6) |
| xxθθSS..... 132853.4 | ( 93.4) | xxθΩSS..... | 51516.1 ( 99.6) |
| xxΩΩSS..... | 4703.9(100.0) | xSθθSS..... | 6746.2 ( 99.6) |
| xSθΩSS..... | 2464.0(100.0) | xSΩΩSS..... | 224.0(100.0) |
| SSθθSS..... | 121.0(100.0) | SSθΩSS.... | 44.0(100.0) |
| SSΩΩSS..... | 4.0(100.0) | | |
| Library size = | 3.0000E+07 | | |
| total..... | 7.8961E+06 | fraction sampled = | 9.2291E-01 |
| xxθθxx..... | 4040589.0( 86.9) | xxθΩxx..... | 1661409.0( 98.3) |
| xxΩΩXX..... | 153619.1(100.0) | xxθθxS..... | 1305393.0 ( 98.3) |
| xxθΩxS..... | 482802.9 (100.0) | xxΩΩxS..... | 43904.0 (100.0) |
| xxθθSS..... | 142254.4 (100.0) | xxθΩSS..... | 51744.0 (100.0) |
| xxΩΩSS..... | 4704.0(100.0) | xSθθSS..... | 6776.0 (100.0) |
| xSθΩSS..... | 2464.0 (100.0) | xSΩΩSS..... | 224.0 (100.0) |
| SSθθSS..... | 121.0 (100.0) | SSθΩSS..... | 44.0 (100.0) |
| SSΩΩSS..... | 4.0(100.0) | | |
| Library size = | 5.0000E+07 | | |
| total...... | 8.3956E+06 | fraction sampled = | 9.8130E-01 |
| xxθθxx..... | 4491779.0 (96.6) | xxθΩxx..... | 1688387.0( 99.9) |
| xxΩΩxx..... | 153663.8(100.0) | xxθθxS..... | 1326590.0( 99.9) |
| xxθΩxS..... | 482943.4(100.0) | xxΩΩxS..... | 43904.0(100.0) |

(continued)

| Type | Number % | Type | Number % |
|---|---|---|---|
| xxθθSS..... | 142295.8(100.0) | xxθΩSS ..... | 51744.0(100.0) |
| xxΩΩSS..... | 4704.0(100.0) | xSθθSS ..... | 6776.0(100.0) |
| xSθΩSS...... | 2464.0(100.0) | xSΩΩSS..... | 224.0(100.0) |
| SSθθSS..... | 121.0(100.0) | SSθΩSS..... | 44.0(100.0) |
| SSΩΩSS..... | 4.0(100.0) | | |
| Library seize = | 1.0000E+08 | | |
| total..... | 8.5503E+06 | fraction sampled = | 9.9938E-01 |
| xxθθxx ..... | 4643063.0 (99.9) | xxθΩxx ..... | 1690302.0(100.0) |
| xxΩΩxx..... | 153664.0(100.0) | xxθθxS..... | 1328094.0(100.0) |
| xxθΩxS..... | 482944.0(100.0) | xxΩΩxS..... | 43904.0(100.0) |
| xxθθSS..... | 142296.0(100.0) | xxθΩSS..... | 51744.0(100.0) |
| xxΩΩSS..... | 4704.0(100.0) | xSθθSS..... | 6776.0(100.0) |
| xSθΩSS..... | 2464.0(100.0) | xSΩΩSS..... | 224.0(100.0) |
| SSθθSS..... | 121.0(100.0) | SSθΩSS...... | 44.0(100.0). |
| SSΩΩSS..... | 4.0(100.0) | | |

Table 132: Relative efficiencies of various simple variegation codons

| | Number of codons | | |
|---|---|---|---|
| | 5 | 6 | 7 |
| vgCodon | #DNA/#AA [#DNA] (#AA) | #DNA/#A [#DNA] (#AA) | #DNA/#AA [#DNA] (#AA) |
| NNR | 8.95 | 13.86 | 21.49 |
| assuming | [2.86-$10^{7}$] | [8.87-$10^{3}$] | [2.75-$10^{10}$] |
| stops vanish | (3.2-$10^{6}$) | (6.4-$10^{7}$) | (1.28-$10^{9}$) |
| NNT | 1.38 | 1.47 | 1.57 |
| | [1.05-106] | [1.68-$10^{7}$] | [2.68-$10^{2}$] |
| | (7.59-$10^{5}$) | (1.14-$10^{7}$) | (1.71-$10^{2}$) |
| NNG | 2.04 | 2.36 | 2.72 |
| assuming | [7.59-$10^{5}$] | [1.14-$10^{6}$] | [1.71-$10^{5}$] |
| stops vanish | (3.7-$10^{5}$) | (4.83-$10^{6}$) | (6.27-$10^{7}$) |

Table 140. Effect of anti BPTI IgG on phage titer.

| Phage Strain | Input | +Anti-BPTI | +Anti-BPTI +Protein A(a) | Eluted Phage |
|---|---|---|---|---|
| M13MP18 | 100 (b) | 98 | 92 | 7-$10^{-4}$ |
| BPTI.3 | 100 | 26 | 21 | 6 |
| M13MB48 (c) | 100 | 90 | 36 | 0.8 |
| M13MB48 (d) | 100 | 60 | 40 | 2.6 |

(a) Protein A-agarose beads.
(b) Percentage of input phage measured as plaque forming units
(c) Batch number 3
(d) Batch number 4

Table 141. Effect of anti-BPTI or protein A on phage titer.

| Strain | Input | No Addition | +Anti-BPTI | +Protein A (a) | +Anti-BPTI +Protein A |
|---|---|---|---|---|---|
| M13MP18 | 100(b) | 107 | 105 | 72 | 65 |
| M13MB48 (b) 100 | | 92 | $7.10^{-3}$ | 58 | $<10^{-4}$ |

(a) Protein A-agarose beads
(b) Percentage of input phage measured as plaque forming units
(c) Batch number 5

Table 142 Effect of anti-BPTI and non-immune serum on phage titer

| Strain | Input | +Anti-BPTI | +NRS (a) | +Anti-BPTI +Protein A (b) | +NRS +Protein |
|---|---|---|---|---|---|
| M13MP18 | 100(C) | 65 | 104 | 71 | 68 |
| M13MB48(d) | 100 | 30 | 125 | 13 | 121 |
| M13MB48(e) | 100 | 2 | 105 | 0.7 | 110 |

(a) Purified IgG from normal rabbit serum.
(b) Protein A-agaroae beads.
(c) Percentage of input phage measured as plaque forming units
(d) Batch number 4
(e) Batch number 5

Table 143. Loss in titer of display phage with anhydrotrypsin.

| Strain | Anhydrotrypsin Beads | | Streptavidin Beads | |
|---|---|---|---|---|
| | Start | Post Incubation | Start | Post Incubation |
| M13MP18 | 100 (a) | 121 | ND | ND |
| M13MB48 | 100 | 58 | 100 | 98 |
| 5AA Pool | 100 | 44 | 100 | 93 |

(a) Plaque forming units expressed as a percentage of input.

Table 144. Binding of Display Phage to Anhydrotrypsin.

Experiment 1.

| Strain | Bluted Phage (a) | Relative to M13MP18 |
|---|---|---|
| M13MP18 | 0.2 (a) | 1.0 |
| BPTI-IIIMK | 7.9 | 39.5 |
| M13MB48 | 11.2 | 56.0 |

Experiment 2.

| Strain | Eluted Phage (a) | Relative to M13mp18 |
|---|---|---|
| M13mp18 | 0.3 | 1.0 |
| BPTI-IIIMK | 12.0 | 40.0 |

(continued)

| Experiment 2. Strain | Eluted Phage (a) | Relative to M13mp18 |
|---|---|---|
| M13MB56 | 17.0 | 56.7 |

(a) Plaque forming units acid eluted from beads, expressed as a percentage of the input.

Table 145. Binding of Display Phage to Anhydrotrypsin or Trypsin.

| Strain | Anhydrotrypsin Beads | | Trypsin Beads | |
|---|---|---|---|---|
| | Eluted Phage (a) | Relative Binding (b) | Eluted Phage | Relative Binding |
| M13MP18 | 0.1 | 1 | $2.3x10^{-4}$ | 1.0 |
| BPTI-IIIMK | 9.1 | 91 | 1.17 | 5x103 |
| M13.3X7 | 25.0 | 250 | 1.4 | $6x10^{3}$ |
| M13.3X11 | 9.2 | 92 | 0.27 | $1.2x10^{3}$ |

(a) Plaque forming units eluted from beads, expressed as a percentage of the input.
(b) Relative to the non-display phage, M13MP18.

Table 146. Binding of Display Phage to Trypsin or Human Neutrophil Elastase.

| Strain | Trypsin Beads | | HNE Beads | |
|---|---|---|---|---|
| | Eluted Phage (a) | Relative Binding(b) | Eluted | Relative Phage Binding |
| M13MP18 | $5x10^{-4}$ | 1 | $3x10^{-4}$ | 1.0 |
| BPTI-IIIMK | 1.0 | 2000 | $5x10^{-3}$ | 16.7 |
| M13MB48 | 0.13 | 260 | $9x10^{-3}$ | 30.0 |
| M13.3X7 | 1.15 | 2300 | $1x10^{-3}$ | 3.3 |
| M13.3X11 | 0.8 | 1600 | $2x10^{-3}$ | 6.7 |
| BPTI3.CL (c) | $1x10^{-3}$ | 2 | 4.1 | $1.4x10^{4}$ |

(a) Plaque forming units acid eluted from the beads, expressed as a percentage of input.
(b) Relative to the non-display phage, M13MP18.
(c) BPTI-IIIMK (K15L MGNG)

Table 820: Streptavidin-Binding Phage

| Name | Putative Streptavidin Binding Peptide Seq. |
|---|---|
| DEV(F) | A E - P C H P O Y R L C Q R P L K Q P P P P P P A E... |
| Dev(E) | A E - L C R P O F P R C N L F R K V P P P P P P A E... |
| HPQ6 | A E G P C H P O F P R C Y I E G R I V - - - - - E... |
| | 11111111112222222 |
| | 2 3 4 5 6 7 8 9 0 1 2 3 4 5 6 7 8 9 0 1 2 3 4 5 6 |
| | - - - - C - - - - - C - - - - - - - - - - - - - E |

Table 827: Effect of DTT on biotinylated HRP binding to streptavidin agarose.

| Conc. DTT (mM) | Biotin-HRP Color Development |
|---|---|
| 0 | ++++ |
| 2 | ++++ |
| 10 | +++ |
| 20 | + |
| 50 | - |

Table 828: Effect of DTT on HPQ6 display page infectivity.

| DTT (mM) | Relative Infectivity |
|---|---|
| 0 | 1.00 |
| 2 | 0.95 |
| 10 | 1.00 |
| 20 | 1.08 |
| 50 | 1.23 |

Table 829: Effect of the presence of DTT on the binding of display phage to streptavidin agarose beads. Inputs: MKTN 4.2 x $10^{11}$, HPQ6 3.3 x $10^{11}$.

| Name | Concn DTT (μM) | Fraction Bound | Relative Binding |
|---|---|---|---|
| MKTN | 0 | 4.8 x $10^{-6}$ | 1.00 |
| | 2,000 | 5.4 x $10^{-6}$ | 1.10 |
| | 10,000 | 5.2 x $10^{-6}$ | 1.10 |
| HPQ6 | 0 | 1.6 x $10^{-4}$ | 1.00 |
| | 1 | 1.6 x $1^{0}$-4 | 1.00 |
| | 10 | 1.5 x $10^{-4}$ | 0.90 |
| | 100 | 9.7 x $10^{-5}$ | 0.60 |
| | 1000 | 1.6 x $10^{-5}$ | 0.10 |
| | 2,000 | 1.0 x $10^{-5}$ | 0.06 |
| | 5.000 | 8.8 x $10^{-6}$ | 0.05 |

Table 832: Effect of preincubating HPQ6 display phage with Factor $X_a$ on binding to streptavidin beads.

| Factor $X_a$ | Titer after Treatment | Relative Titer | Fraction Bound | Relative Binding |
|---|---|---|---|---|
| - | 3.3 x $10^{11}$ | 1 | 1.4 x $10^{-4}$ | 1 |
| + | 3.3 x $10^{11}$ | 1 | 1.2 x $10^{-5}$ | 8.5 x $10^{-2}$ |

Table 833: $FX_a$ treatment of HPQ6 display phage following binding to straptavidin.

| Factor $X_a$ | Total Fraction Bound | Fraction Eluted by Treatment | % Removed by Treatment |
|---|---|---|---|
| - | 7.6 x $10^{-3}$ | 1.6 x $10^{-3}$ | 14 |

| + | $6.4 \times 10^{-3}$ | $2.6 \times 10^{-3}$ | 40 |
|---|---|---|---|

Table 834: Removal of HPQ6 display phage from streptavidin by $FX_a$

| Amount of $FX_a$ Added (units) | Time (hrs) | % Removed by Treatment. |
|---|---|---|
| 0 | 1 | 17 |
| 2.5 | 1 | 21 |
| 6.3 | 1 | 22 |
| 12.5 | 1 | 35 |
| 0 | 2 | 18 |
| 2.5 | 2 | 53 |
| 6.3 | 2 | 54 |
| 12.5 | 2 | 52 |

**CITATIONS**

ASHM89: Ashman, Matthews, and Frank (1989) Protein Engineering 2(5): 387-91.
BANN81: Banner, DW, C Nave, and DA Marvin, Nature (1981), 289:814-816.
BECK89c: Becker, S, E Atherton, and RD Gordon, Eur J Biochem, (Oct 20 1989), 185(1)79-84.
BERG88: Berg, JM, Proc Natl Acad Sci USA (1988), 85:99-102.
BETT88: Better, M, CP Chang, RR Robinson, and AH Horwitz, Science (1988), 240:1041-1043.
BOEK80: Boeke, JD, M Russel, and P Model, J Mol Biol (1980), 144:103-116.
CHAN79: Chang, CN, P Model, and G Blobel, Proc Natl Acad Sci USA (1979), 76:1251-1255.
CHOU74: Chou, PY, and GD Fasman, Biochemistry (1974), 13:(2)222-45.
COLM87: Hemostasis and Thrombosis, Second Edition, Editors Colman, Hirsh, Marder, and Salzman, Published by Pippincott, Philadelphia, PA, 1987, ISBN 0-397-50679-1.
CREI84: Creighton, TE, Proteins: Structures and Molecular Principles, W H Freeman & Co, New York, 1984.
CWIR90: Cwirla, SE, EA Peters, RW Barrett, and WJ Dower, Proc Natl Acad Sci USA, (August 1990), 87:6378-6382.
DELA88: de la Cruz, VF, AA Lal and TF McCutchan, J Biol Chem, (1988), 263(9)4318-22.
DEVL90: Devlin, JJ, LC Panganiban, and PE Devlin, Science, (27 July 1990), 249:404-406.
DICK83: Dickerson, RE, and I Geis, Hemoglobin: Structure, Function, Evolution, and Pathology, The Bejamin/ Cummings Publishing Co, Menlo Park, CA, 1983.
DULB86: Dulbecco, R, US Patent 4,593,002, June 3, 1986.
GAUS87: Gauss, P, KB Krassa, DS McPheeters, MA Nelson, and L Gold, Proc Natl Acad Sci USA (1987), 84: 8515-19.
GIBS88: Gibson, TJ, JPM Postma, RS Brown, and P Argos, Protein Engineering (1988), 2(3)209-218.
GUAN91: Guan, KL and Dixon JE, Anal. Biochem, (1991), 192: 262-67.
HARD90: Hard, T, E Kellenbach, R Boelens, BA Maler, K Dahlman, LP Freedman, J Carlstedt-Duke, KR Yamamoto, J-A Gustafsson, and R Kaptein, Science (13 July 1990), 249:157-60.
HORV89: Horvat, S, B Grgas, N Raos, and VI Simeon, Int J Peptide Protein Res (1989), 34:346-51.
INOU82: Inouye, H, W Barnes, and J Beckwith, J Bacteriol (1982), 149(2)434-439.
ITOK79: Ito, K, G Mandel, and W Wickner, Proc Natl Acad Sci USA (1979), 76:1199-1203.
JANA89: Janatova, J, KBM Reid, and AC Willis, Biochem (1989), 28:4754-61.
JANI85: Janin, J, and C Chothia, Methods in Enzymology (1985), 115(28)420-430.
KAPL78: Kaplan, DA, L Greenfield, and G Wilcox, in The Single-Stranded DNA Phages, Denhardt, DT, D Dressler, and DS Ray editors, Cold Spring Harbor Laboratory, 1978., p461-467.
KATZ90: Katz, B, and AA Kossiakoff, Proteins, Struct, Funct, and Genet (1990), 7:343-57.
KIMH89: Kim, et al., Protein Engineering (1989), 2(1): 379-86.
KISH85: Kishore, R, and P Balaram, Biopolymers (1985), 24:2041-43.
KUHN85a: Kuhn, A, and W Wickner, J Biol Chem (1985), 260:15914-15918.
KUHN85b: Kuhn, A, and W Wickner, J Biol Chem (1985), 260:15907-15913.
KUHN87: Kuhn, A, Science (1987), 238:1413-1415.

LISS85: Liss, LR, BL Johnson, and DB Oliver, J Bacteriol (1985), 164(2)925-8.
LOPE85a: Lopez, J, and RE Webster, J Bacteriol (1985), 163(3)1270-4.
LUIT85: Luiten, RGM, DG Putterman, JGG Schoenmakers, RNH Konings, and LA Day, J Virology, (1985), 56(1) 268-276.
LUIT87: Luiten, RGM, RIL Eggen, JGG Schoenmakers, and RNH Konings, DNA (1987), 6(2)129-37.
MAK080: Makowski, L, DLD Caspar, and DA Marvin, J Mol Biol (1980), 140:149-181.
MANI82: Maniatis, T, EF Fritsch, and J Sambrook, Molecular Cloning, Cold Spring Harbor Laboratory, 1982.
MARK86: Marks, CB, M Vasser, P Ng, W Henzel, and S Anderson, J Biol Chem (1986), 261:7115-7118.
MARV78: Marvin, DA, in The Single-Stranded DNA Phages, Denhardt, DT, D Dressler, and DS Ray editors, Cold Spring Harbor Laboratory, 1978., p583-603.
MATS89: Matsumura, M, WJ Becktel, M Levitt, and BW Matthews, Proc Natl Acad Sci USA (1989), 86:6562-6.
MCCA90: McCafferty, J, AD Griffiths, G Winter, and DJ Chiswell, Nature, (6 Dec 1990), 348:552-4.
MESS77: Messing, J, B Gronenborn, B Muller-Hill, and PH Hofschneider, Proc Natl Acad Sci USA (1977), 74:3642-6.
MESS78: Messing, J, and B Gronenborn, in The Single-Stranded DNA Phages, Denhardt, DT, D Dressler, and DS Ray editors, Cold Spring Harbor Laboratory, 1978.,p449-453.
NICH88: Nicholson, H, WJ Becktel, and BW MAtthews, Nature (1988), 336:651-56.
NISH82: Nishiuchi, Y, and S Sakakibara, FEBS Lett (1982), 148:260-2.
NISH86: Nishiuchi, Y, K Kumagaye, Y Noda, TX Watanabe, and S Sakakibara, Biopolymers, (1986), 25:S61-8.
OHKA81: Ohkawa, I, and RE Webster, J Biol Chem (1981), 256:9951-9958.
OLIV90a: Olivera, BM, J Rivier, C Clark, CA Ramilo, GP Corpuz, FC Abogadie, EE Mena, SR Woodward, DR Hillyard, LJ Cruz, Science, (20 July 1990), 249:257-263.
PAB079: Pabo, CO, RT Sauer, JM Sturtevant, and M Ptashne, Proc Natl Acad Sci USA (1979), 76:1608-1612.
PARM88: Parmley, SF, and GP Smith, Gene (1988), 73:305-318.
PERR84: Perry, LJ, and R Wetzel, Science (1984), 226:555-7.
PERR86: Perry, LJ, and R Wetzel, Biochem (1986), 25:733-39.
POTE83: Poteete, AR, J Mol Biol (1983), 171:401-418.
RASC86: Rasched, I, and E Oberer, Microbiol Rev (1986) 50:401-427.
RASH84: Rashin, A, Biocheostry (1984), 23:5518.
ROSE85: Rose, GD, Methods in Enzymololgy (1985) 115(29)430-440.
RUSS81: Russel, M, and P Model, Proc Natl Acad Sci USA (1981), 78:1717-1721. (1981), 78:1717-1721.
RUSS82: Russel and Model, Cell (1982), 28(1): 177-84.
SALI88: Sali, D, M Bycroft, and AR Fersht, Nature (1988), 335:740-3.
SAMB89: Sambrook, J, EF Fritsch, and T Maniatis, Molecular Cloning, A Laboratory Manual, Second Edition, Cold Spring Harbor Laboratory, 1989.
SAUE86: Sauer, RT, K Hehir, RS Stearrman, MA Weiss, A Jeitler-Nilsson, EG Suchanek, and CO Pabo, Biochem (1986), 25:5992-98.
SCHA78: Schaller, H, E Beck, and M Takanami, in The Single-Stranded DNA Phages, Denhardt, D.T., D. Dressler, and D.S. Ray editors, Cold Spring Harbor Laboratory, 1978., p139-163.
SCHN86: Schnabel, E, W Schroeder, and G Reinhardt, Biol Chem Hoppe-Seyler (1986), 367:1167-76.
SCOT87a: Scott, MJ, CS Huckaby, I Kato, WJ Kohr, M Laskowski Jr., M-J Tsai and BW O'Malley, J Biol Chem (1987), 262(12)5899-5907.
SCOT90: Scott, JK, and GP Smith, Science, (27 July 1990), 249:386-390.
SMIT85: Smith GP, Science (1985), 228:1315-1317.
SUMM91: Summers, J. Cell Biochem. (1991), 45(1): 41-8.
VITA84: Vita, C, D Dalzoppo, and A Fontana, Biochemistry (1984), 23:5512-5519.
WEBS78: Webster, RE, and JS Cashman, in The Single-Stranded DNA Phages, Denhardt, DT, D Dressler, and DS Ray editors, Cold Spring Harbor Laboratory, 1978., p557-569.
WELL86: Wells, JA, and DB Powers, J Biol Chem (1986), 261:6564-70.
YANI85: Yanisch-Perron, C, J Vieira, and J Messing, Gene, (1985), 33:103-119.
ZAFA88: Zafaralla, GC, C Ramilo, WR Gray, R Karlstrom, BM Olivera, and LJ Cruz, Biochemistry, (1988), 27(18) 7102-5.
ZIMM82: Zimmermann, R, C Watts, and W Wickner, J Biol Chem (1982), 257:6529-6536.

The following pages 156 - 162 relate to preferred embodiments of the invention, wherein the word "claim" means "embodiment".

1. In a process for developing novel epitopes or binding proteins with a desired binding activity against a particular target material which comprises providing a library of phage which each displays on its surface, as a result of

expression of a first phage gene, one or more copies of a particular chimeric coat protein, each chimeric coat protein comprising a potential epitope, or a potential binding domain which is a mutant of a known protein domain foreign to said phage, said library collectively displaying a plurality of potential epitopes or binding domains, contacting said library of phage with the target material, and separating the phage on the basis of their affinity for the target material, the improvement wherein said chimeric coat protein further comprises a linker peptide which is specifically cleavable by said site-specific protease.

2. The method of 1 wherein the site-specific protease is Factor Xa, Factor XIa, kallikrein, thrombin, Factor XIIa, collagenase or enterokinase.

3. The method of 1 wherein, after said library of phage is contacted with said target material, (1) low affinity phage are removed, (2) high affinity phage still bound to said target material are released by cleavage of said chimeric coat protein at said linker by means of a site-specific proteases, and the released high affinity phage are recovered.

4. In as process for developing novel binding peptides or proteins with a desired binding activity against a particular target material which comprises providing a library of phage which each displays on its surface, as a result of expression of a first phage gene, one or more copies of a particular chimeric coat protein, each chimeric coat protein comprising a mutant of a known protein domain foreign to said phage, said library collectively displaying a plurality of potential binding domains, contacting said library of phage with the target material, and separating the phage on the basis of their affinity for the target material, the improvement wherein said said potential binding domain has at least one intrachain covalent crosslink between a first amino acid position and a second amino acid position thereof, the amino acids at said first and second positions being invariant in all of the chimeric proteins displayed by said library, and where low affinity phage are removed from said target material first, and then high affinity phage are released or rendered more readily eluted from the target material by treating the phage with a reagent which cleaves the crosslink, preferably a reagent which does not kill the phage.

5. The method of any of 1-4 wherein the domain is a mini-protein of less than sixty amino acids, more preferably a micro-protein of less than forty amino acids.

6. The method of 4 wherein the crosslink is a disulfide bond and the amino acids at said first and second positions are cysteines.

7. The method of 6 wherein the reagent is dithiothreitol.

8. The method of any of 1-7 wherein the phage further comprises a second phage gene encoding the cognate wild-type coat protein of the phage.

9. In a process for developing novel epitopes with a desired binding activity against a particular target material which comprises providing a library of phage which each displays on its surface, as a result of expression of a first phage gene, one or more copies of a particular chimeric coat protein, each chimeric coat protein comprising a potential epitope, said library collectively displaying a plurality of potential epitopes, contacting said library of phage with the target material, and separating the phage on the basis of their affinity for the target material, the improvement wherein the phage further comprises a second phage gene encoding the cognate wild-type coat protein of the phage.

10. In a process for developing novel epitopes or binding proteins with a desired binding activity against a particular target material which comprises providing a library of phage which each displays on its surface, as a result of expression of a first phage gene, one or more copies of a particular chimeric coat protein, each chimeric coat protein comprising a potential epitope, or a potential binding domain which is a mutant of a known protein domain foreign to said phage, said library collectively displaying a plurality of potential epitopes or binding domains, contacting said library of phage with the target material, and separating the phage on the basis of their affinity for the target material, the improvement wherein the chimeric coat protein includes only an assemblable fragment of a coat protein of said phage, and not that portion of the coat protein which is responsible for pilus binding, and the phage also comprises a second phage gene encoding the cognate native coat protein of the phage.

11. In a process for developing novel epitopes with a desired binding activity against a particular target material which comprises providing a library of phage which each displays on its surface, as a result of expression of a first phage gene, one or more copies of a particular chimeric coat protein, each chimeric coat protein comprising a potential epitope, said library collectively displaying a plurality of potential epitopes, contacting said library of phage with the target material, and separating the phage on the basis of their affinity for the target material, the improvement wherein the cognate wild-type coat protein of the phage is the major coat protein of the phage.

12. The method of 8-11 wherein the initiation codon of the second phage gene is a leucine.

13. The method of any of 1-12 wherein the first phage gene further comprises a cytoplasmic secretion signal sequence which codes for a signal peptide which directs the immediate expression product to the inner membrane of the bacterial host cell infected by said phage, where it is processed to remove said signal peptide, yielding a mature chimeric coat protein comprising the potential binding domain and at least a portion of a geneVIII-like protein of the phage, said chimeric protein being assembled with wild-type coat protein into the phage coat, wherein the secretion signal is encoded by a signal sequence selected from the group consisting of the signal sequences of the phoA, bla and geneIII genes.

14. In a process for developing novel epitopes or binding proteins with a desired binding activity against a particular target material which comprises providing a library of phage which each displays on its surface, as a result of expression of a first phage gene, one or more copies of a particular chimeric coat protein, each chimeric coat protein comprising a potential epitope, or a potential binding domain which is a mutant of a known protein domain foreign to said phage, said library collectively displaying a plurality of potential epitopes or binding domains, contacting said library of phage with the target material, and separating the phage on the basis of their affinity for the target material, the improvement wherein the phage also comprises a second phage gene encoding the cognate native coat protein of the phage, and the initiation codon of the second phage gene is a Leucine codon.

15. The method of any of 1-14 wherein the differentiation among said plurality of different potential binding domains occurs through the at least partially random variation of one or more predetermined amino acid positions of said known domain to randomly obtain at each said position an amino acid belonging to a predetermined set of two or more amino acids, the amino acids of said set occurring at said position in predetermined expected proportions.

16. The method of 15 wherein the differentiation among said potential binding domains of said library is limited to no more than about 20 predetermined amino acid residues of said sequences.

17. The method of 15 wherein, for each set, the ratio of the probability of occurrence of the most favored amino acid to that for the least favored amino acid is less than about 2.6.

18. The method of any of 1-17 wherein, for any potentially encoded potential binding domain, the probability that it will be displayed by at least one package in said population is at least 50%, more preferably at least 90%.

19. The method of any of 1-18 wherein said population is characterized by the display of at least $10^5$ different potential binding binding domains.

20. The method of any of 1-19 wherein the initially chosen parental potential binding domain is selected from the group consisting of (a) binding domains of bovine pancreatic trypsin inhibitor, crambin, Cucurbita maxima trypsin inhibitor III, a heat-stable enterotoxin of Escherichia coli, an alpha-, mu- or omega-conotoxin, apamin, charybdotoxin, secretory leukocyte protease inhibitor, cystatin, eglin, barley protease inhibitor, ovomucoid, T4 lysozyme, hen egg white lysozyme, ribonuclease, azurin, tumor necrosis factor, and CD4, and (b) domains at least substantially homologous with any of the foregoing domains which have a melting point of at least 50˚C.

21. In a process for developing novel epitopes with a desired affinity for a particular binding protein target material which comprises providing a library of phage which each displays its surface, as a result of expression of a first phage gene, one or more copies of a particular chimeric coat protein, each chimer coat protein comprising a potential epitope, or a potential bindi: domain which is a mutant of a known protein domain foreign to sai phage, said library collectively displaying a plurality of potential epitopes or binding domains, contacting said library of phage with the target material, and separating the phage on the basis of their affinity for the binding protein target material, the improvement wherein the differentiation among said plurality of different potential binding domains occurs through the at leas partially random variation of one or more predetermined amino acid positions of said known domain to randomly obtain at each said position an amino acid belonging to a predetermined set of two or more amino acids, the amino acids of said set occurring at said position in predetermined expected proportions, and in substantially all sets the ratio of the frequency of occurrence o the most favored amino acid to that for the least favored amino acid is less than 2.6.

22. The method of 21 in which at least one variable amino acid position is encoded by a simply variegated codon selected from the group consisting of NNT, NNG, RNG, RMG, VNT, RRS, and SNT.

23. The method of 21 wherein none of the variabl amino acid positions is encoded by a simply variegated codon selected from the group consisting of NNN, NNK and NNS.

24. The method of 21 in which at least one variable amino acid position is encoded by a complexly variegated codon.

25. A library of display phage which each displays on its surface, as a result of expression of a first phage gene, one or more copies of a particular chimeric coat protein, each chimeric coat protein comprising a potential epitope, or a potential binding domain which is a mutant of a known protein domain foreign to said phage, said library collectively displaying a plurality of potential epitopes or binding domains, contacting said library of phage with the target material, and separating the phage on the basis of their affinity for the binding protein target material, wherein the differentiation among said plurality of different potential binding domains occurs through the at least partially random variation of one or more predetermined amino acid positions of said known domain to randomly obtain at each said position an amino acid belonging to a predetermined set of two or more amino acids, the amino acids of said set occurring at said position in predetermined expected proportions, and in substantially all sets the ratio of the frequency of occurrence of the most favored amino acid to that for the least favored amino acid is less than 2.6.

26. A library of display phage which each displays on its surface; as a result of expression of a first phage gene, one or more copies of a particular chimeric coat protein, each chimeric coat protein comprising a potential epitope, or a potential binding domain which is a mutant of a known protein domain foreign to said phage, said library collectively displaying a plurality of potential epitopes or binding domains, contacting said library of phage with the target material, and separating the phage on the basis of their affinity for the target material, wherein said chimeric

coat protein further comprises a linker peptide which is specifically cleavable by said site-specific protease.

## Claims

1. A method for identifying binding proteins which bind a target, the method comprising the steps of:

    (a) providing a library of phage, wherein each phage in the library displays at least one copy of a chimeric coat protein encoded by a first phage gene, wherein the chimeric coat protein comprises a potential binding domain which is foreign to said phage; and wherein each phage further comprises a second phage gene encoding the native phage coat protein; and said population collectively displaying a plurality of different potential binding domains, wherein the differentiation among said plurality occurs through at least partially random variation of one or more predetermined amino acid positions of said known domain;
    (b) contacting the packages with the target material; and
    (c) separating the packages according to their affinity for said target material.

2. The method of claim 1, wherein the phage is a DNA phage.

3. The method of claim 1 or 2, wherein the phage is a filamentous phage.

4. The method of any one of claims 1-3, wherein the potential binding domain comprises a potential epitope.

5. The method of any one of claims 1-4, wherein the first phage gene further comprises a cytoplasmic secretion signal sequence which codes for a signal peptide, wherein the secretion signal sequence is selected from the group consisting of the signal sequences of the phoA, bla and geneIII genes.

6. The method of claim 5, wherein the signal peptide directs a protein encoded by the first phage gene to the inner membrane of the bacterial host cell infected by said phage, wherein the signal peptide is removed, resulting in a mature chimeric coat protein.

7. The method of any one of claims 1-6, wherein the chimeric coat protein comprises at least a portion of a gene VIII protein of the phage.

8. The method of any one of claims 1-7, wherein the chimeric protein is assembled with a wild-type phage coat protein into the phage coat.

9. The method of any one of claims 1-8, wherein the differentiation among the potential binding domains of the library is limited to no more than about 20 predetermined amino acid residues of an amino acid sequence encoding the domain.

10. The method of any one of claims 1-9, wherein for any potential binding domain, the probability that it will be displayed by at least one phage in said population is at least 50%.

11. The method of any one of claims 1-10, wherein for any potential binding domain, the probability that it will be displayed by at least one phage in said population is at least 90%.

12. The method of any one of claims 1-11, wherein said library of phage is **characterized by** the display of at least $10^5$ different potential binding domains.

13. The method of any one of claims 1-12, wherein the potential binding domain is selected from the group consisting of (a) binding domains of bovine pancreatic trypsin inhibitor, crambin, Cucurbita maxima trypsin inhibitor III, a heat-stable enterotoxin of Escherichia coli, an alpha-, mu- or omega-conotoxin, apamin, charybdotoxin, secretory leukocyte protease inhibitor, cystatin, eglin, barley protease inhibitor, ovomucoid, T4 lysozyme, hen egg white lysozyme, ribonuclease, azurin, tumor necrosis factor, and CD4, and (b) domains at least substantially homologous with any of the foregoing domains and which have a melting point of at least 50°C.

14. The method of one of claims 1-13, wherein the chimeric coat protein comprises an assemblable fragment of a coat protein of said phage without the pilus portion of the coat protein.

**15.** A library of display phage, wherein each phage in the library displays at least one copy of a chimeric coat protein encoded by a first phage gene, wherein the chimeric coat protein comprises a potential binding domain which is foreign to said phage; and wherein each phage further comprises a second phage gene encoding the native phage coat protein; and said population collectively displaying a plurality of different potential binding domains, wherein the differentiation among said plurality occurs through at least partially random variation of one or more predetermined amino acid positions of said known domain.

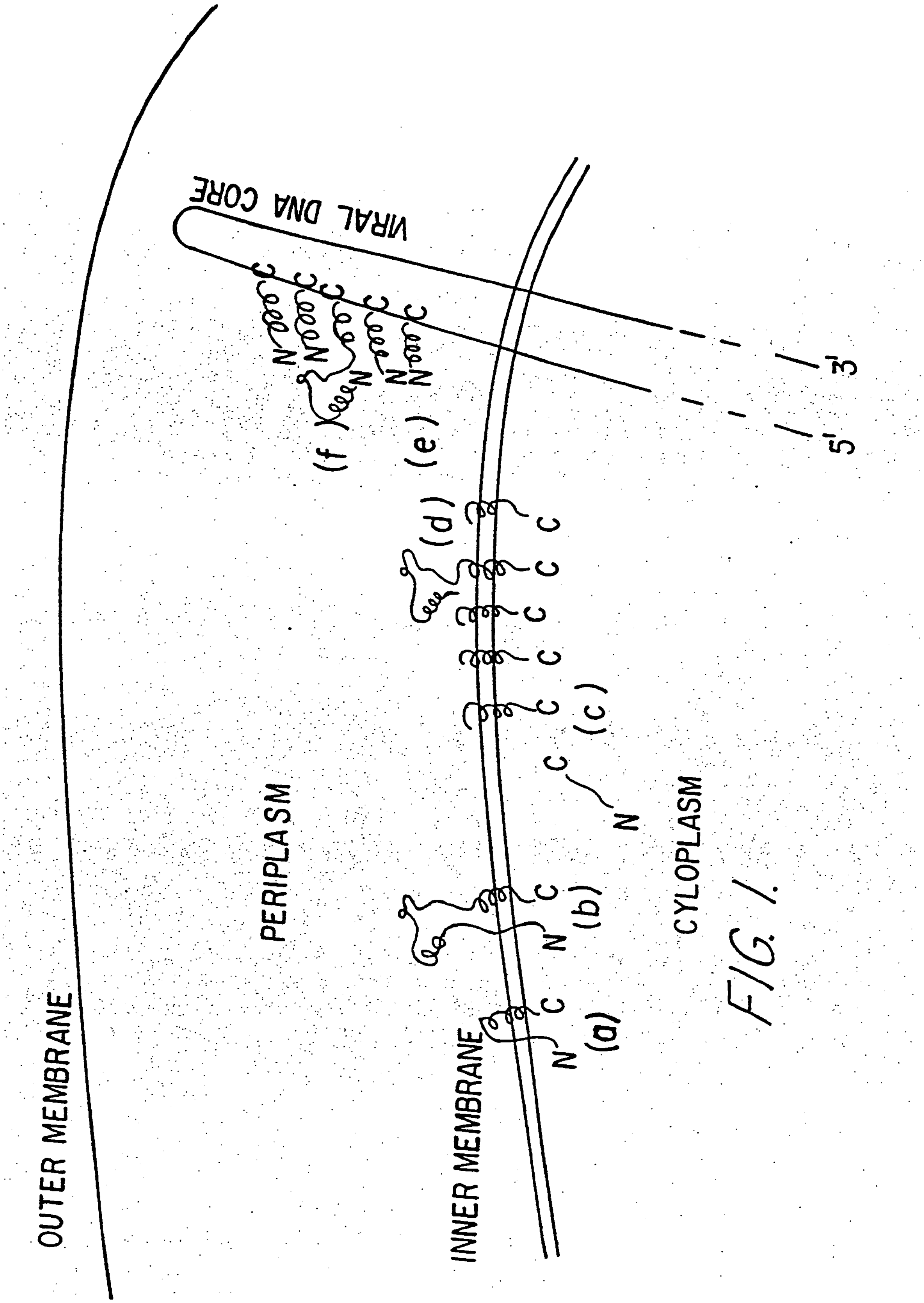
OUTER MEMBRANE
INNER MEMBRANE
PERIPLASM
CYLOPLASM
VIRAL DNA CORE
(a)
(b)
(c)
(d)
(e)
(f)
5'
3'

FIG. 1.

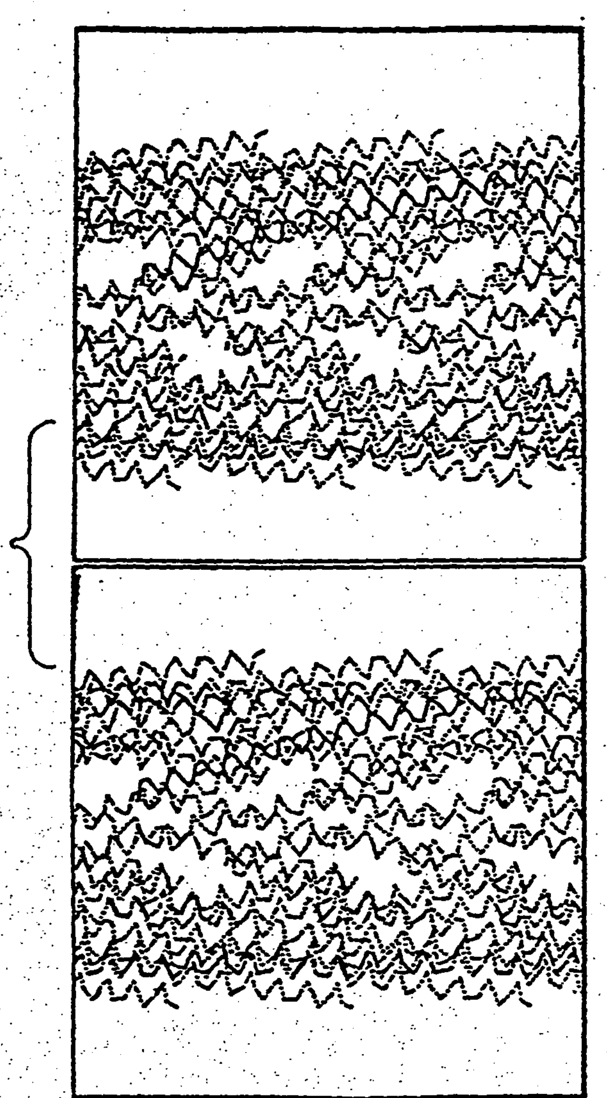

FIG. 2.

Europäisches Patentamt
European Patent Office
Office européen des brevets

# EUROPEAN SEARCH REPORT

Application Number
EP 10 00 4494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 90/02809 A (PROTEIN ENG CORP) 22 March 1990 (1990-03-22)<br>* page 45, paragraph 3 *<br>* abstract *<br>* page 126, line 25 - page 134 *<br>* page 146, line 1 - line 5 *<br>* claims *<br>----- | 1-15 | INV.<br>C12N15/10<br>C12N15/62<br>G01N33/50<br>G01N33/68 |
| A | SCOTT J K ET AL: "SEARCHING FOR PEPTIDE LIGANDS WITH AN EPITOPE LIBRARY"<br>SCIENCE,<br>vol. 249, 27 July 1990 (1990-07-27), pages 386-390, XP000571645<br>ISSN: 0036-8075<br>* the whole document *<br>----- | 1-15 | |
| A,P | MARKLAND W ET AL: "DESIGN, CONSTRUCTION AND FUNCTION OF A MULTICOPY DISPLAY VECTOR USING FUSIONS TO THE MAJOR COAT PROTEIN OF BACTERIOPHAGE M13"<br>GENE,<br>vol. 109, 1991, pages 13-19, XP000575848<br>ISSN: 0378-1119<br>* the whole document *<br>----- | 1-15 | |

TECHNICAL FIELDS SEARCHED (IPC)
C12N
G01N

The present search report has been drawn up for all claims

| Place of search | Date of completion of the search | Examiner |
|---|---|---|
| Munich | 1 July 2010 | Page, Michael |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 00 4494

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2010

| Patent document cited in search report | Publication date | Patent family member(s) | Publication date |
| --- | --- | --- | --- |
| WO 9002809 A | 22-03-1990 | AT 151110 T | 15-04-1997 |
| | | AU 4308689 A | 02-04-1990 |
| | | CA 1340288 C | 29-12-1998 |
| | | DE 68927933 D1 | 07-05-1997 |
| | | DE 768377 T1 | 02-01-1998 |
| | | EP 1997891 A1 | 03-12-2008 |
| | | EP 0436597 A1 | 17-07-1991 |
| | | IL 91501 A | 10-03-1998 |
| | | JP 4502700 T | 21-05-1992 |
| | | JP 3771253 B2 | 26-04-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8806630 A, Ladner, Glick, and Bird
- US 07021046 B
- WO 9002809 A, Ladner
- US 4769326 A, Rutter
- US 4593002 A, Dulbecco, R


### Non-patent literature cited in the description


- **Greene.** *PROTECTIVE GROUPS IN ORGANIC SYNTHESIS,* 1981
- **Ashman ; Matthews ; Frank.** *Protein Engineering,* 1989, vol. 2 (5), 387-91
- **Banner, DW ; C Nave ; DA Marvin.** *Nature,* 1981, vol. 289, 814-816
- **Becker, S ; E Atherton ; RD Gordon.** *Eur J Biochem,* 20 October 1989, vol. 185 (1), 79-84
- **Berg, JM.** *Proc Natl Acad Sci USA,* 1988, vol. 85, 99-102
- **Better, M ; CP Chang ; RR Robinson ; AH Horwitz.** *Science,* 1988, vol. 240, 1041-1043
- **Boeke, JD ; M Russel ; P Model.** *J Mol Biol,* 1980, vol. 144, 103-116
- **Chang, CN ; P Model ; G Blobel.** *Proc Natl Acad Sci USA,* 1979, vol. 76, 1251-1255
- **Chou, PY ; GD Fasman.** *Biochemistry,* 1974, vol. 13 (2), 222-45
- Hemostasis and Thrombosis. Pippincott, 1987
- **Creighton, TE.** Proteins: Structures and Molecular Principles. W H Freeman & Co, 1984
- **Cwirla, SE ; EA Peters ; RW Barrett ; WJ Dower.** *Proc Natl Acad Sci USA,* August 1990, vol. 87, 6378-6382
- **de la Cruz, VF ; AA Lal ; TF McCutchan.** *J Biol Chem,* 1988, vol. 263 (9), 4318-22
- **Devlin, JJ ; LC Panganiban ; PE Devlin.** *Science,* 27 July 1990, vol. 249, 404-406
- **Dickerson, RE ; I Geis.** Hemoglobin: Structure, Function, Evolution, and Pathology. The Bejamin/Cummings Publishing Co, 1983
- **Gauss, P ; KB Krassa ; DS McPheeters ; MA Nelson ; L Gold.** *Proc Natl Acad Sci USA,* 1987, vol. 84, 8515-19
- **Gibson, TJ ; JPM Postma ; RS Brown ; P Argos.** *Protein Engineering,* 1988, vol. 2 (3), 209-218
- **Guan, KL ; Dixon JE.** *Anal. Biochem,* 1991, vol. 192, 262-67
- **Hard, T ; E Kellenbach ; R Boelens ; BA Maler ; K Dahlman ; LP Freedman ; J Carlstedt-Duke ; KR Yamamoto ; J-A Gustafsson ; R Kaptein.** *Science,* 13 July 1990, vol. 249, 157-60
- **Horvat, S ; B Grgas ; N Raos ; VI Simeon.** *Int J Peptide Protein Res,* 1989, vol. 34, 346-51
- **Inouye, H ; W Barnes ; J Beckwith.** *J Bacteriol,* 1982, vol. 149 (2), 434-439
- **Ito, K ; G Mandel ; W Wickner.** *Proc Natl Acad Sci USA,* 1979, vol. 76, 1199-1203
- **Janatova, J ; KBM Reid ; AC Willis.** *Biochem,* 1989, vol. 28, 4754-61
- **Janin, J ; C Chothia.** *Methods in Enzymology,* 1985, vol. 115 (28), 420-430
- **Kaplan, DA ; L Greenfield ; G Wilcox.** The Single-Stranded DNA Phages. Cold Spring Harbor Laboratory, 1978, 461-467
- **Katz, B ; AA Kossiakoff.** *Proteins, Struct, Funct, and Genet,* 1990, vol. 7, 343-57
- **Kim et al.** *Protein Engineering,* 1989, vol. 2 (1), 379-86
- **Kishore, R ; P Balaram.** *Biopolymers,* 1985, vol. 24, 2041-43
- **Kuhn, A ; W Wickner.** *J Biol Chem,* 1985, vol. 260, 15914-15918
- **Kuhn, A ; W Wickner.** *J Biol Chem,* 1985, vol. 260, 15907-15913
- **Kuhn, A.** *Science,* 1987, vol. 238, 1413-1415
- **Liss, LR ; BL Johnson ; DB Oliver.** *J Bacteriol,* 1985, vol. 164 (2), 925-8
- **Lopez, J ; RE Webster.** *J Bacteriol,* 1985, vol. 163 (3), 1270-4
- **Luiten, RGM ; DG Putterman ; JGG Schoenmakers ; RNH Konings ; LA Day.** *J Virology,* 1985, vol. 56 (1), 268-276
- **Luiten, RGM ; RIL Eggen ; JGG Schoenmakers ; RNH Konings.** *DNA,* 1987, vol. 6 (2), 129-37
- **Makowski, L ; DLD Caspar ; DA Marvin.** *J Mol Biol,* 1980, vol. 140, 149-181
- **Maniatis, T ; EF Fritsch ; J Sambrook.** Molecular Cloning. Cold Spring Harbor Laboratory, 1982

- **Marks, CB ; M Vasser ; P Ng ; W Henzel ; S Anderson.** *J Biol Chem,* 1986, vol. 261, 7115-7118
- **Marvin, DA.** The Single-Stranded DNA Phages. Cold Spring Harbor Laboratory, 1978, 583-603
- **Matsumura, M ; WJ Becktel ; M Levitt ; BW Matthews.** *Proc Natl Acad Sci USA,* 1989, vol. 86, 6562-6
- **McCafferty, J ; AD Griffiths ; G Winter ; DJ Chiswell.** *Nature,* 06 December 1990, vol. 348, 552-4
- **Messing, J ; B Gronenborn ; B Muller-Hill ; PH Hofschneider.** *Proc Natl Acad Sci USA,* 1977, vol. 74, 3642-6
- **Messing, J ; B Gronenborn.** The Single-Stranded DNA Phages. Cold Spring Harbor Laboratory, 1978, 449-453
- **Nicholson, H ; WJ Becktel ; BW MAtthews.** *Nature,* 1988, vol. 336, 651-56
- **Nishiuchi, Y ; S Sakakibara.** *FEBS Lett,* 1982, vol. 148, 260-2
- **Nishiuchi, Y ; K Kumagaye ; Y Noda ; TX Watanabe ; S Sakakibara.** *Biopolymers,* 1986, vol. 25, S61-8
- **Ohkawa, I ; RE Webster.** *J Biol Chem,* 1981, vol. 256, 9951-9958
- **Olivera, BM ; J Rivier ; C Clark ; CA Ramilo ; GP Corpuz ; FC Abogadie ; EE Mena ; SR Woodward ; DR Hillyard ; LJ Cruz.** *Science,* 20 July 1990, vol. 249, 257-263
- **Pabo, CO ; RT Sauer ; JM Sturtevant ; M Ptashne.** *Proc Natl Acad Sci USA,* 1979, vol. 76, 1608-1612
- **Parmley, SF ; GP Smith.** *Gene,* 1988, vol. 73, 305-318
- **Perry, LJ ; R Wetzel.** *Science,* 1984, vol. 226, 555-7
- **Perry, LJ ; R Wetzel.** *Biochem,* 1986, vol. 25, 733-39
- **Poteete, AR.** *J Mol Biol,* 1983, vol. 171, 401-418
- **Rasched, I ; E Oberer.** *Microbiol Rev,* 1986, vol. 50, 401-427
- **Rashin, A.** *Biocheostry,* 1984, vol. 23, 5518
- **Rose, GD.** *Methods in Enzymololgy,* 1985, vol. 115 (29), 430-440
- **Russel, M ; P Model.** *Proc Natl Acad Sci USA,* 1981, vol. 78, 1717-1721
- *PROC NATL ACAD SCI USA,* vol. 78, 1717-1721
- **Russel ; Model.** *Cell,* 1982, vol. 28 (1), 177-84
- **Sali, D ; M Bycroft ; AR Fersht.** *Nature,* 1988, vol. 335, 740-3
- **Sambrook, J ; EF Fritsch ; T Maniatis.** Molecular Cloning, A Laboratory Manual. Cold Spring Harbor Laboratory, 1989
- **Sauer, RT ; K Hehir ; RS Stearrman ; MA Weiss ; A Jeitler-Nilsson ; EG Suchanek ; CO Pabo.** *Biochem,* 1986, vol. 25, 5992-98
- **Schaller, H ; E Beck ; M Takanami.** The Single-Stranded DNA Phages. Cold Spring Harbor Laboratory, 1978, 139-163
- **Schnabel, E ; W Schroeder ; G Reinhardt.** *Biol Chem Hoppe-Seyler,* 1986, vol. 367, 1167-76
- **Scott, MJ ; CS Huckaby ; I Kato ; WJ Kohr ; M Laskowski Jr. ; M-J Tsai ; BW O'Malley.** *J Biol Chem,* 1987, vol. 262 (12), 5899-5907
- **Scott, JK ; GP Smith.** *Science,* 27 July 1990, vol. 249, 386-390
- **Smith GP.** *Science,* 1985, vol. 228, 1315-1317
- **Summers.** *J. Cell Biochem.,* 1991, vol. 45 (1), 41-8
- **Vita, C ; D Dalzoppo ; A Fontana.** *Biochemistry,* 1984, vol. 23, 5512-5519
- **Webster, RE ; JS Cashman.** The Single-Stranded DNA Phages. Cold Spring Harbor Laboratory, 1978, 557-569
- **Wells, JA ; DB Powers.** *J Biol Chem,* 1986, vol. 261, 6564-70
- **Yanisch-Perron, C ; J Vieira ; J Messing.** *Gene,* 1985, vol. 33, 103-119
- **Zafaralla, GC ; C Ramilo ; WR Gray ; R Karlstrom ; BM Olivera ; LJ Cruz.** *Biochemistry,* 1988, vol. 27 (18), 7102-5
- **Zimmermann, R ; C Watts ; W Wickner.** *J Biol Chem,* 1982, vol. 257, 6529-6536